# EUROPEAN PATENT APPLICATION

(11) **EP 3 650 469 A1**
(43) Date of publication of application: **13.05.2020**
(21) Application number: 18820564.5
(22) Date of filing: 21.03.2018
(51) Int. Cl.: C07K 16/30, C12N 15/13, C12N 5/095, A61K 39/395, A61P 35/00, A61P 35/04, G01N 33/574

(54) **MONOCLONAL ANTIBODY OF TARGETED HUMAN TUMOR STEM CELLS AND APPLICATION OF MONOCLONAL ANTIBODY**

(30) Priority: 23.06.2017 CN 201710485302; 23.06.2017 CN 201710485170; 23.06.2017 CN 201710484849
(71) Applicant: Suzhou Bojuhua Biomedical Technology Co. Ltd, Jiangsu 215000 (CN)
(72) Inventor: SHEN, Min, Suzhou Jiangsu 215000 (CN)
(74) Representative: Nederlandsch Octrooibureau
(86) International application number: PCT/CN2018/079785
(87) International publication number: WO 2018/233333

(57) **Abstract**

The invention relates to the field of biomedicine. In particular, the invention relates to an isolated monoclonal antibody or antigen-binding fragment thereof against human tumor stem cells, and the use of said antibody or fragment in the treatment and diagnosis of tumors.

## Description

### Technical Field

The invention relates to the field of biomedicine. In particular, the invention relates to an isolated monoclonal antibody or antigen-binding fragment thereof against human tumor stem cells, and to the use of said antibody or fragment in the treatment and diagnosis of tumors.

### Background

Malignant tumors (cancers) have become the "number one killer" threatening the lives and health of people around the world. There are more than 14 million new cancer cases per year in the world, while there are more than 3 million new cancer patients each year in China.

The main causes for high mortality of cancer are the metastasis of cancer cells and that most patients are prone to relapse and drug resistance after treatment. The present clinically available treatments, surgery, radiotherapy, and chemotherapy have little effect on metastasis of cancer cells, relapse, and drug resistance, or have only short-term effects, which makes it unable to achieve the long-term survival of the patients. At present, surgery excision is effective for about 10-20% of early patients, but it is almost ineffective for patients who have undergone metastasis. As radiotherapy can only treat local lesions, it is often used as adjunctive therapy before and after surgery and radical treatment of a few types of cancers. Though chemotherapy can be used for patients who have undergone metastasis, but due to its strong toxic and side effects and its tendency to produce short-term or long-term drug resistance, it can only have a significant short-term therapeutic effect on about 20-30% of patients. Even with a comprehensive treatment measure integrating the surgery, radiotherapy and chemotherapy, the long-term therapeutic effect, five-year survival rate, has been hovering at 20-30% for many years, with about 70-80% of patients dead within 5 years after treatment due to metastasis, relapse and drug resistance. Even in early cancer patients who did not show metastasis at the beginning, some of them died due to metastasis and relapse after treatment. New targeted drugs for tumors developed in recent years, including polypeptide, small molecules, protein factors, gene therapy and antibody drugs, can only prolong the survival time of patients by 3 to 9 months even when they are used in combination with chemotherapeutic drugs, and have no significant improvement for long-term five-year survival rate of the patients. Although the tumor immunotherapy emerging in the last two years, such as PD-1 monoclonal antibody drugs against the immune checkpoints and the CAR-T cell therapy, has shown some encouraging signs of long-term therapeutic efficacy, its overall effective rate to cancer patients can only reach about 20-30%, and there are still a large number of cancer patients who cannot get treated with truly effective drugs. Therefore, it is desired to develop new drugs that inhibit tumor metastasis, relapse, and drug resistance for improving the long-term therapeutic efficacy and prolonging survival time of cancer patients.

### Summary

In one aspect, the invention provides an isolated monoclonal antibody or antigen-binding fragment thereof against tumor stem cells, wherein the monoclonal antibody comprises a light chain variable region and a heavy chain variable region,
the light chain variable region comprises:
a VL CDR1 comprising an amino acid sequence set forth in SEQ ID NO: 2, or an amino acid sequence having 1 or 2 amino acid residue substitutions, deletions or additions relative to SEQ ID NO: 2,
a VL CDR2 comprising an amino acid sequence set forth in SEQ ID NO: 3, or an amino acid sequence having 1 or 2 amino acid residue substitutions, deletions or additions relative to SEQ ID NO: 3, and
a VL CDR3 comprising an amino acid sequence set forth in SEQ ID NO: 4, or an amino acid sequence having 1 or 2 amino acid residue substitutions, deletions or additions relative to SEQ ID NO: 4;
the heavy chain variable region comprises:
a VH CDR1 comprising an amino acid sequence set forth in SEQ ID NO:6, or an amino acid sequence having 1 or 2 amino acid residue substitutions, deletions or additions relative to SEQ ID NO:6,
a VH CDR2 comprising an amino acid sequence set forth in SEQ ID NO:7, or an amino acid sequence having 1 or 2 amino acid residue substitutions, deletions or additions relative to SEQ ID NO:7, such as a sequence set forth in SEQ ID NO: 13, and
a VH CDR3 comprising an amino acid sequence set forth in SEQ ID NO:8, or an amino acid sequence having 1 or 2 amino acid residue substitutions, deletions or additions relative to SEQ ID NO:8, such as a sequence set forth in SEQ ID NO:14.

In some embodiments wherein the monoclonal antibody comprises a light chain variable region and a heavy chain variable region,
the light chain variable region comprises:
a VL CDR1 comprising an amino acid sequence set forth in SEQ ID NO: 2,
a VL CDR2 comprising an amino acid sequence set forth in SEQ ID NO: 3, and
a VL CDR3 comprising an amino acid sequence set forth in SEQ ID NO:4;
the heavy chain variable region comprises:
a VH CDR1 comprising an amino acid sequence set forth in SEQ ID NO:6,
a VH CDR2 comprising an amino acid sequence set forth in SEQ ID NO: 13, and
a VH CDR3 comprising an amino acid sequence set forth in SEQ ID NO:8.

In some embodiments wherein the monoclonal antibody comprises a light chain variable region and a heavy chain variable region,
the light chain variable region comprises:
a VL CDR1 comprising an amino acid sequence set forth in SEQ ID NO: 2,
a VL CDR2 comprising an amino acid sequence set forth in SEQ ID NO: 3, and
a VL CDR3 comprising an amino acid sequence set forth in SEQ ID NO:4;
the heavy chain variable region comprises:
a VH CDR1 comprising an amino acid sequence set forth in SEQ ID NO:6,
a VH CDR2 comprising an amino acid sequence set forth in SEQ ID NO: 7, and
a VH CDR3 comprising an amino acid sequence set forth in SEQ ID NO: 14.

In some embodiments, the light chain variable region comprises an amino acid sequence set forth in SEQ ID NO: 1, or an amino acid sequence having at least 85%, at least 90%, at least 95% or higher sequence identity to SEQ ID NO: 1.

In some embodiments, the heavy chain variable region comprises an amino acid sequence set forth in SEQ ID NO:5, or an amino acid sequence having at least 85%, at least 90%, at least 95% or higher sequence identity to SEQ ID NO:5.

In some embodiments, the heavy chain variable region comprises an amino acid sequence set forth in one of the SEQ ID NOs:15-19 (corresponding to the variable regions of the humanized heavy chain versions H1-H5, respectively).

In some embodiments, the light chain variable region comprises an amino acid sequence set forth in one of the SEQ ID NOs:20-31 (corresponding to the variable regions of the humanized light chain versions LI-LI 2, respectively).

In some embodiments, the heavy chain variable region comprises an amino acid sequence set forth in SEQ ID NO:19, and the light chain variable region comprises an amino acid sequence set forth in SEQ ID NO: 25.

In some embodiments, the heavy chain variable region comprises an amino acid sequence set forth in SEQ ID NO:19, and the light chain variable region comprises an amino acid sequence set forth in SEQ ID NO: 30.

In some embodiments, the monoclonal antibody comprises a human heavy chain constant region, such as a human heavy chain constant region comprising an amino acid sequence set forth in SEQ ID NO:11.

In some embodiments, the monoclonal antibody comprises a human light chain constant region, such as a human light chain constant region comprising an amino acid sequence set forth in SEQ ID NO:12.

In some embodiments, the monoclonal antibody comprises a heavy chain having the amino acid sequence set forth in SEQ ID NO:9 and a light chain having an amino acid sequence set forth in SEQ ID NO:10.

In some embodiments, the monoclonal antibody comprises a heavy chain having an amino acid sequence set forth in one of SEQ ID NOs:32-36 (corresponding to the humanized heavy chain versions H1-H5, respectively) and a light chain having an amino acid sequence set forth in one of SEQ ID NOs: 37-48 (corresponding to the humanized light chain versions L1-L12, respectively).

In some embodiments, the monoclonal antibody comprises a heavy chain set forth in SEQ ID NO: 36 and a light chain set forth in SEQ ID NO: 47 (corresponding to the humanized antibody H5L 11).

In some embodiments, the monoclonal antibody comprises a heavy chain set forth in SEQ ID NO:36 and a light chain set forth in SEQ ID NO:42 (corresponding to the humanized antibody H5L6).

In another aspect, the present invention provides a monoclonal antibody or antigen-binding fragment thereof, wherein the monoclonal antibody is produced by the mouse hybridoma deposited in China General Microbiological Culture Collection Center on March 16, 2016 under the accession number of CGMCC NO. 12251.

In another aspect, the present invention provides a hybridoma deposited in the China General Microbiological Culture Collection Center on March 16, 2016 under the accession number of CGMCC NO. 12251.

In another aspect, the present invention provides a pharmaceutical composition comprising the monoclonal antibody or an antigen-binding fragment thereof of the invention, and a pharmaceutically acceptable carrier.

In some embodiments, the monoclonal antibody or antigen-binding fragment thereof is conjugated to a therapeutic moiety selected from the group consisting of a cytotoxin, a radioisotope, or a biologically active protein.

In another aspect, the present invention provides a method for treating a malignant tumor, preventing and/or treating metastasis or relapse of a malignant tumor in a patient, the method comprising administering to the patient an effective amount of the monoclonal antibody or antigen-binding fragment thereof of the invention or the pharmaceutical composition of the invention.

In some embodiments, the malignant tumor is selected from the group consisting of breast cancer, colorectal cancer, pancreatic cancer, prostatic cancer, liver cancer, lung cancer, and gastric cancer.

In some embodiments, the method further comprises administering to the patient other anti-tumor treatment means, such as administering a chemotherapeutic agent, an antibody targeting other tumor-specific antigens, or radiation therapy.

In another aspect, the present invention provides the use of the monoclonal antibody or antigen-binding fragment thereof of the invention or a pharmaceutical composition of the invention in preparing a medicament for treating a malignant tumor, preventing and/or treating metastasis or relapse of a malignant tumor.

In some embodiments, the malignant tumor is selected from the group consisting of breast cancer, colorectal cancer, pancreatic cancer, prostatic cancer, liver cancer, lung cancer, and gastric cancer.

In another aspect, the invention provides a method for detecting the presence of tumor stem cells in a biological sample, comprising:
a) contacting the biological sample with the monoclonal antibody or antigen-binding fragment thereof of the invention;
b) detecting the binding of the monoclonal antibody or antigen-binding fragment thereof of the invention to a target antigen in said biological sample, wherein the detected binding indicates the presence of the tumor stem cells in the biological sample.

In another aspect, the invention further provides a method for isolating tumor stem cells, the method comprising:
(a) providing a cell population suspected of containing the tumor stem cells;
(b) identifying a subpopulation of said cells that bind to the monoclonal antibody or antigen-binding fragment thereof of the invention; and
(c) isolating the subpopulation.

In some embodiments of the foregoing various aspects, the tumor stem cells are selected from the group consisting of breast cancer stem cells, colorectal cancer stem cells, pancreatic cancer stem cells, prostatic cancer stem cells, liver cancer stem cells, lung cancer stem cells, and gastric cancer stem cells.

In another aspect, the invention further provides a method for detecting the presence of a malignant tumor in a patient, comprising:
a) contacting a biological sample obtained from said patient with the monoclonal antibody or antigen-binding fragment thereof of the invention;
b) detecting the binding of the monoclonal antibody or antigen-binding fragment thereof of the invention to a target antigen in said biological sample, wherein the detected binding indicates the presence of a malignant tumor in said patient.

In another aspect, the invention further provides a method for prognosis of relapse or progression of a malignant tumor in a patient, the method comprising:
(a) isolating a biological sample comprising circulating cells from the patient;
(b) contacting said biological sample comprising the circulating cells with the monoclonal antibody or antigen-binding fragment thereof of the invention;
(c) identifying the presence of the circulating cells that bind to the monoclonal antibody or antigen-binding fragment thereof of the invention,
thereby obtaining prognosis of the relapse or progression of the malignant tumor in the patient.

In some embodiments, the progression of the malignant tumor comprises metastasis of the malignant tumor in the patient.

In some embodiments of the foregoing various aspects, the biological sample comprises a blood sample, a lymph sample, or any components thereof. In some embodiments, the malignant tumor is selected from the group consisting of breast cancer, colorectal cancer, pancreatic cancer, prostatic cancer, liver cancer, lung cancer, and gastric cancer.

In another aspect, the invention further provides an isolated nucleic acid molecule encoding the monoclonal antibody or antigen-binding fragment thereof of the invention.

In some embodiments, the nucleic acid molecule is operably linked to an expression regulatory sequence.

In another aspect, the invention further provides an expression vector comprising the nucleic acid molecule of the invention.

In another aspect, the invention further provides a host cell transformed with the nucleic acid molecule of the invention or the expression vector of the invention.

In another aspect, the invention provides a method of producing a monoclonal antibody or antigen-binding fragment thereof against human tumor stem cells, the method comprising:
(i) culturing a host cell of the invention in a condition suitable for expression of the nucleic acid molecule or expression vector of the invention, and
(ii) isolating and purifying the antibody or antigen-binding fragment thereof expressed by the nucleic acid molecule or expression vector.

### Description of Figures

FIG. 1. shows the detection of the expression of the monoclonal antibody Hetumomab target antigen on the surface of living cells of various tumor cells (some typical positive results) by live cell immunofluorescence technique.
FIG. 2. shows the immunohistochemical detection of the specific high expression of Hetumomab target antigen in human liver cancer, lung cancer, and gastric cancer tissues (some typical positive results).
FIG. 3. shows the immunological flow fluorescence detection of the substantial enrichment of cancer cells recognized by Hetumomab in the sphere cultured cells of various human tumor cell lines (some typical flow fluorescence atlas).
FIG. 4. shows the CCK8 detection of drug resistance of the Hetumomab+ cells (IC50) in various human tumor cells (such as liver cancer, lung cancer, and gastric cancer) recognized by Hetumomab.
FIG. 5. shows Hetumomab significantly inhibits the self-renewal ability (sphere-forming) of the tumor stem cells in various tumors (such as liver cancer, lung cancer, and gastric cancer).
FIG. 6. shows Hetumomab significantly inhibits the invasive ability of the tumor stem cells in various tumors (such as liver cancer, lung cancer, and gastric cancer).
FIG. 7. shows Hetumomab significantly inhibits the invasive ability of the tumor stem cells from multiple tumors.
FIG. 8. shows the in vivo tumor growth curve of the human liver cancer transplanted tumor Bel7402-V13 treated with Hetumomab, and the combination of Hetumomab and chemotherapy drug.
FIG. 9. shows the in vivo tumor volume inhibition rate of the human liver cancer transplanted tumor Bel7402-V13 treated with Hetumomab, and the combination of Hetumomab and chemotherapy drug (at the time of drug withdrawal).
FIG. 10. shows the in vivo tumor volume inhibition rate of the human liver cancer transplantation tumor Bel7402-V13 treated with Hetumomab, and the combination of Hetumomab and chemotherapy drug (one month after drug withdrawal).
FIG. 11. shows the survival curve of the mouse with the human liver cancer transplantation tumor Bel7402-V13 treated with Hetumomab, and the combination of Hetumomab and chemotherapy drug.
FIG. 12. shows the in vivo tumor growth curve of the human lung cancer transplantation tumor SPCA-1 treated with Hetumomab.
FIG. 13. shows the in vivo tumor growth curve of the human gastric cancer transplantation tumor SNU-5 treated with Hetumomab, and the combination of Hetumomab and chemotherapy drug.
FIG. 14. shows that the chimeric antibody Hetuximab binds to the same antigenic protein on the tumor stem cells as the parent antibody Hetumomab.
FIG. 15. shows that the chimeric antibody Hetuximab competes with the parent antibody Hetumomab for binding to the antigen.
FIG. 16. shows the alignment (partial) of the amino acid sequence of each version of the humanized heavy chain of Hetumomab, with Hetumomab corresponding to the abbreviation E21.
FIG. 17. shows the alignment (partial) of the amino acid sequence of each version of the humanized light chain of Hetumomab, with Hetumomab corresponding to the abbreviation E21.
FIG. 18. shows the glycosylation heterogeneity present in the H1 heavy chain series revealed by electrophoretic analysis.
FIG. 19. shows that A: glycosylation isomerism of H5 disappears; B: the combined glycosylation isomerism disappears according to the electrophoretic analysis when H5 was combined with L11, L6.
FIG. 20. shows a humanized antibody H5L11 with a purity greater than 95% obtained by the expression and purification from CHO cells. A: Reduced SDS-PAGE electrophoresis analysis shows that the purity of the purified antibody is greater than 95%; B: non-reduced SDS-PAGE electrophoresis analysis shows that the purity of the purified antibody is greater than 95%; and C: HPLC analysis shows that the purity of the purified antibody is greater than 95%.

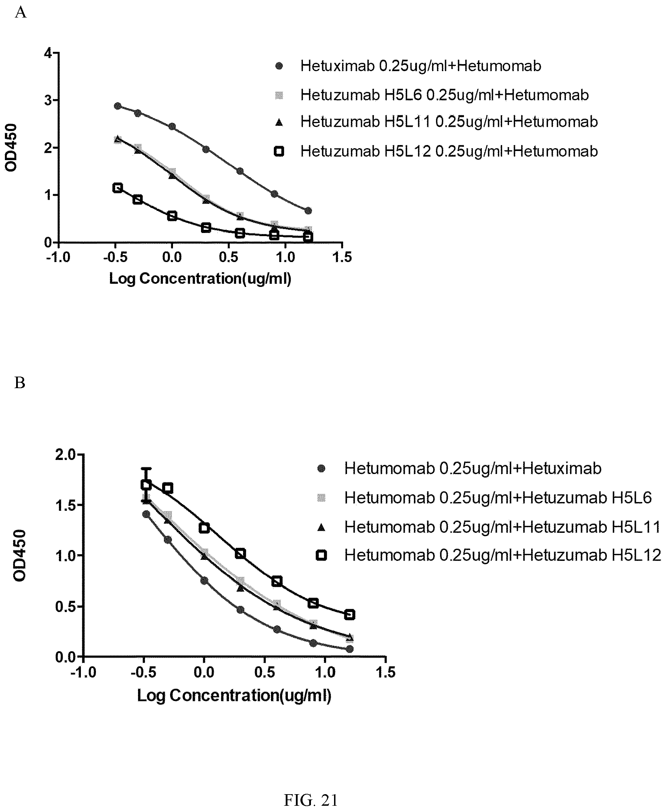

FIG. 21. shows that the parental mouse monoclonal antibody Hetumomab, the chimeric antibody Hetuximab and the humanized monoclonal antibody Hetuzumab compete for binding to the antigen. A: The parental mouse monoclonal antibody Hetumomab of different concentrations competitively inhibits binding of the 3 variants of Hetuzumab (H5L6, H5L11, H5L12) and the chimeric antibody Hetuximab to the antigen; B: The 3 variants of Hetuzumab of different concentrations (H5L6, H5L11, H5L12) and the chimeric antibody Hetuximab competitively inhibit binding of the parental mouse monoclonal antibody Hetumomab to the antigen.
FIG. 22. shows the in vivo tumor growth curve in mice with human liver cancer transplantation tumor treated with the monoclonal antibody Hetuzumab H5L11, and the combination of Hetuzumab H5L11 and chemotherapy drugs.

### Detailed Description

### I. Definitions

Unless defined otherwise, all technical and scientific terms used herein have the same meaning as is commonly understood by one of skill in the art to which the invention(s) belong. In addition, the terms and experimental procedures relating to protein and nucleic acid chemistry, molecular biology, cell and tissue culture, microbiology and immunology are those terms and common procedures widely used in the art. Meanwhile, for better understanding of the invention, definitions and explanations of relevant terms are provided below.

As used herein, "antibody" refers to immunoglobulins and immunoglobulin fragments, whether natural or partially or wholly synthetically, such as recombinantly, produced, including any fragment thereof containing at least a portion of the variable region of the immunoglobulin molecule that retains the binding specificity ability of the full-length immunoglobulin. Hence, an antibody includes any protein having a binding domain that is homologous or substantially homologous to an immunoglobulin antigen-binding domain (antibody combining site). Antibodies include antibody fragments, such as antibody fragment against tumor stem cells. As used herein, the term antibody, thus, includes synthetic antibodies, recombinantly produced antibodies, multispecific antibodies (e.g., bispecific antibodies), human antibodies, non-human antibodies, humanized antibodies, chimeric antibodies, intrabodies, and antibody fragments, such as, but not limited to, Fab fragments, Fab' fragments, F(ab')₂ fragments, Fv fragments, disulfide-linked Fvs (dsFv), Fd fragments, Fd' fragments, single-chain Fvs (scFv), single-chain Fabs (scFab), diabodies, anti-idiotypic (anti-Id) antibodies, or antigen-binding fragments of any of the above. Antibodies provided herein include members of any immunoglobulin type (e.g., IgG, IgM, IgD, IgE, IgAand IgY), any class (e.g. IgG1, IgG2, IgG3, IgG4, IgA1 and IgA2) or subclass (e.g., IgG2a and IgG2b).

As used herein, an "antibody fragment" or "antigen-binding fragment" of an antibody refers to any portion of a full-length antibody that is less than full length but contains at least a portion of the variable region of the antibody that binds antigen (e.g. one or more CDRs and/or one or more antibody combining sites) and thus retains the binding specificity, and at least a portion of the specific binding ability of the full-length antibody. Hence, an antigen-binding fragment refers to an antibody fragment that contains an antigen-binding portion that binds to the same antigen as the antibody from which the antibody fragment is derived. Antibody fragments include antibody derivatives produced by enzymatic treatment of full-length antibodies, as well as synthetically, e.g. recombinantly produced derivatives. An antibody fragment is included among antibodies. Examples of antibody fragments include, but are not limited to, Fab, Fab', F(ab')₂, single-chain Fv (scFv), Fv, dsFv, diabody, Fd and Fd' fragments and other fragments, including modified fragments (see, for example, Methods in Molecular Biology, Vol 207: Recombinant Antibodies for Cancer Therapy Methods and Protocols (2003); Chapter 1; p 3-25, Kipriyanov). The fragment can include multiple chains linked together, such as by disulfide bridges and/or by peptide linkers. An antibody fragment generally contains at least or about 50 amino acids and typically at least or about 200 amino acids. An antigen-binding fragment includes any antibody fragment that when inserted into an antibody framework (such as by replacing a corresponding region) results in an antibody that immunospecifically binds (i.e. exhibits Ka of at least or at least about 10⁷- 10⁸ M⁻¹) to the antigen.

As used herein, "monoclonal antibody" refers to a population of identical antibodies, meaning that each individual antibody molecule in a population of monoclonal antibodies is identical to the others. This property is in contrast to that of a polyclonal population of antibodies, which contains antibodies having a plurality of different sequences. Monoclonal antibodies can be produced by a number of well-known methods (Smith et al. (2004) J. Clin. Pathol. 57, 912-917; and Nelson et al., J Clin Pathol (2000), 53, 111-117). For example, monoclonal antibodies can be produced by immortalization of a B cell, for example through fusion with a myeloma cell to generate a hybridoma cell line or by infection of B cells with virus such as EBV. Recombinant technology also can be used to produce antibodies *in vitro* from clonal populations of host cells by transforming the host cells with plasmids carrying artificial sequences of nucleotides encoding the antibodies.

As used herein, the term "hybridoma" or "hybridoma cell" refers to a cell or cell line (typically a myeloma or lymphoma cell) produced by the fusion of antibody-producing lymphocytes and non-antibody-producing cancer cells. As is known to those of ordinary skill in the art, hybridoma may proliferate and continue to produce specific monoclonal antibodies. Methods for producing a hybridoma are known in the art (see, for example, Harlow & Lane, 1988). When referring to the term "hybridoma" or "hybridoma cell", it also includes subcloned and progeny cells of the hybridoma.

As used herein, a "conventional antibody" refers to an antibody that contains two heavy chains (which can be denoted H and H') and two light chains (which can be denoted L and L') and two antibody combining sites, where each heavy chain can be a full-length immunoglobulin heavy chain or any functional region thereof that retains antigen-binding capability (e.g. heavy chains include, but are not limited to, V_{H}, chains V_{H}-C_{H}1 chains and V_{H}-C_{H}1-C_{H}2-C_{H}3 chains), and each light chain can be a full-length light chain or any functional region of (e.g. light chains include, but are not limited to, V_{L} chains and V_{L}-C_{L} chains). Each heavy chain (H and H') pairs with one light chain (L and L', respectively)

As used herein, a full-length antibody is an antibody having two full-length heavy chains (e.g. V_{H}-C_{H}1-C_{H}2-C_{H}3 or V_{H}-C_{H}1-C_{H}2-C_{H}3-C_{H}4) and two full-length light chains (V_{L}-C_{L}) and hinge regions, such as human antibodies produced naturally by antibody secreting B cells and antibodies with the same domains that are synthetically produced.

As used herein, a dsFv refers to an Fv with an engineered intermolecular disulfide bond, which stabilizes the V_{H}-V_{L} pair.

As used herein, a Fab fragment is an antibody fragment that results from digestion of a full-length immunoglobulin with papain, or a fragment having the same structure that is produced synthetically, *e*.*g*. by recombinant methods. A Fab fragment contains a light chain (containing a V_{L} and C_{L}) and another chain containing a variable domain of a heavy chain (V_{H}) and one constant region domain of the heavy chain (C_{H}1).

As used herein, a F(ab')₂ fragment is an antibody fragment that results from digestion of an immunoglobulin with pepsin at pH 4.0-4.5, or a fragment having the same structure that is produced synthetically, *e*.*g*. by recombinant methods. The F(ab')₂ fragment essentially contains two Fab fragments where each heavy chain portion contains an additional few amino acids, including cysteine residues that form disulfide linkages joining the two fragments.

As used herein, a Fab' fragment is a fragment containing one half (one heavy chain and one light chain) of the F(ab')₂ fragment.

As used herein, an scFv fragment refers to an antibody fragment that contains a variable light chain (V_{L}) and variable heavy chain (V_{H}), covalently connected by a polypeptide linker in any order. The linker is of a length such that the two variable domains are bridged without substantial interference. Exemplary linkers are (Gly-Ser)ₙ residues with some Glu or Lys residues dispersed throughout to increase solubility.

The term "chimeric antibody" is intended to refer to antibodies in which the variable region sequences are derived from one species and the constant region sequences are derived from another species, such as an antibody in which the variable region sequences are derived from a rat antibody and the constant region sequences are derived from a human antibody.

"Humanized" antibody refers to forms of non-human (e.g. rat) antibodies that are chimeric immunoglobulins, immunoglobulin chains, or fragments thereof (such as Fv, Fab, Fab', F(ab')₂ or other antigen-binding subsequences of antibodies) that contain minimal sequence derived from non-human immunoglobulin. Preferably, humanized antibodies are human immunoglobulins (recipient antibody) in which residues from a complementary determining region (CDR) of the recipient are replaced by residues from a CDR of a non-human species (donor antibody) such as mouse, rat, or rabbit having the desired specificity, affinity, and capacity.

Furthermore, during humanization, it is also possible to mutate the amino acid residues within the CDR1, CDR2 and/or CDR3 regions of VH and/or VL, thereby improving one or more binding properties (e.g., affinity) of the antibody. A mutation, such as a PCR-mediated mutation, can be performed, the effect of which on antibody binding or other functional properties can be assessed using in vitro or in vivo tests as described herein. Typically, a conservative mutation is introduced. Such mutation can be an amino acid substitution, addition or deletion. In addition, the number of mutations within the CDRs typically does not exceed one or two. Thus, the humanized antibody of the invention also encompasses the antibody comprising one or two amino acid mutations within the CDRs.

As used herein, the term "epitope" refers to any antigenic determinant on an antigen to which the paratope of an antibody binds. Epitopic determinants typically contain chemically active surface groupings of molecules such as amino acids or sugar side chains and typically have specific three dimensional structural characteristics, as well as specific charge characteristics.

As used herein, a variable domain or variable region is a specific Ig domain of an antibody heavy or light chain that contains a sequence of amino acids that varies among different antibodies. Each light chain and each heavy chain has one variable region domain, V_{L} and V_{H}, respectively. The variable domains provide antigen specificity, and thus are responsible for antigen recognition. Each variable region contains CDRs that are part of the antigen-binding site domain and framework regions (FRs).

As used herein, "antigen-binding domain," "antigen-binding site," "antigen combining site" and "antibody combining site" are used synonymously to refer to a domain within an antibody that recognizes and physically interacts with cognate antigen. A native conventional full-length antibody molecule has two conventional antigen-binding sites, each containing portions of a heavy chain variable region and portions of a light chain variable region. A conventional antigen-binding site contains the loops that connect the anti-parallel beta strands within the variable region domains. The antigen combining sites can contain other portions of the variable region domains. Each conventional antigen-binding site contains three hypervariable regions from the heavy chain and three hypervariable regions from the light chain. The hypervariable regions also are called complementarity-determining regions (CDRs).

As used herein, "hypervariable region," "HV," "complementarity-determining region" and "CDR" and "antibody CDR" are used interchangeably to refer to one of a plurality of portions within each variable region that together form an antigen-binding site of an antibody. Each variable region domain contains three CDRs, named CDR1, CDR2 and CDR3. The three CDRs are non-contiguous along the linear amino acid sequence, but are proximate in the folded polypeptide. The CDRs are located within the loops that join the parallel strands of the beta sheets of the variable domain. As described herein, one of skill in the art knows and can identify the CDRs based on Kabat or Chothia numbering (see e.g., Kabat, E.A. et al. (1991) Sequences of Proteins of Immunological Interest, Fifth Edition, U. S. Department of Health and Human Services, NIH Publication No. 91-3242, and Chothia, C. et al. (1987) J. Mol. Biol. 196:901-917).

As used herein, framework regions (FRs) are the domains within the antibody variable region domains that are located within the beta sheets; the FR regions are comparatively more conserved, in terms of their amino acid sequences, than the hypervariable regions.

As used herein, a "constant region" domain is a domain in an antibody heavy or light chain that contains a sequence of amino acids that is comparatively more conserved than that of the variable region domain. In conventional full-length antibody molecules, each light chain has a single light chain constant region (C_{L}) domain and each heavy chain contains one or more heavy chain constant region (C_{H}) domains, which include, C_{H}1, C_{H}2, C_{H}3 and C_{H}4. Full-length IgA, IgD and IgG isotypes contain C_{H}1, C_{H}2, C_{H}3 and a hinge region, while IgE and IgM contain C_{H}1, C_{H}2, C_{H}3 and C_{H}4. C_{H}1 and C_{L} domains extend the Fab arm of the antibody molecule, thus contributing to the interaction with antigen and rotation of the antibody arms. Antibody constant regions can serve effector functions, such as, but not limited to, clearance of antigens, pathogens and toxins to which the antibody specifically binds, e.g., through interactions with various cells, biomolecules and tissues.

As used herein, a functional region of an antibody is a portion of the antibody that contains at least a V_{H}, V_{L}, C_{H} (*e*.*g*. C_{H}1, C_{H}2 or C_{H}3), C_{L} or hinge region domain of the antibody, or at least a functional region thereof.

As used herein, a functional region of a V_{H} domain is at least a portion of the full V_{H} domain that retains at least a portion of the binding specificity of the full V_{H} domain (*e*.*g*. by retaining one or more CDR of the full V_{H} domain), such that the functional region of the V_{H} domain, either alone or in combination with another antibody domain (*e*.*g*. V_{L} domain) or region thereof, binds to antigen. Exemplary functional regions of V_{H} domains are regions containing the CDR1, CDR2 and/or CDR3 of the V_{H} domain.

As used herein, a functional region of a V_{L} domain is at least a portion of the full V_{L} domain that retains at least a portion of the binding specificity of the full V_{L} domain (*e*.*g*. by retaining one or more CDRs of the full V_{L} domain), such that the function region of the V_{L} domain, either alone or in combination with another antibody domain (*e*.*g*. V_{H} domain) or region thereof, binds to antigen. Exemplary functional regions of V_{L} domains are regions containing the CDR1, CDR2 and/or CDR3 of the V_{L} domain.

As used herein, "specifically bind" or "immunospecifically bind" with respect to an antibody or antigen-binding fragment thereof are used interchangeably herein and refer to the ability of the antibody or antigen-binding fragment to form one or more noncovalent bonds with a cognate antigen, by noncovalent interactions between the antibody combining site(s) of the antibody and the antigen. The antigen can be an isolated antigen or presented in a tumor cell. Typically, an antibody that immunospecifically binds (or that specifically binds) to a antigen is one that binds to the antigen with an affinity constant Ka of about or 1×10⁷ M⁻¹ or 1x 10⁸ M⁻¹ or greater (or a dissociation constant (K_{d}) of 1x 10⁻⁷ M or 1×10⁻⁸ M or less). Affinity constants can be determined by standard kinetic methodology for antibody reactions, for example, immunoassays, surface plasmon resonance (SPR) (Rich and Myszka (2000) Curr. Opin. Biotechnol 11:54; Englebienne (1998) Analyst. 123:1599), isothermal titration calorimetry (ITC) or other kinetic interaction assays known in the art (see, *e*.*g*., Paul, ed., Fundamental Immunology, 2nd ed., Raven Press, New York, pages 332-336 (1989); see also U.S. Pat. No. 7,229,619 for a description of exemplary SPR and ITC methods for calculating the binding affinity of antibodies). Instrumentation and methods for real time detection and monitoring of binding rates are known and are commercially available (*e*.*g*., BiaCore 2000, Biacore AB, Upsala, Sweden and GE Healthcare Life Sciences; Malmqvist (2000) Biochem. Soc. Trans. 27:335).

The term "compete" or "competes", as used herein with regard to an antibody, means that a first antibody, or an antigen-binding fragment thereof, binds to an epitope in a manner sufficiently similar to the binding of a second antibody, or an antigen-binding fragment thereof, such that the result of binding of the first antibody with its cognate epitope is detectably decreased in the presence of the second antibody compared to the binding of the first antibody in the absence of the second antibody. Alternatively, where the binding of the second antibody to its epitope is also detectably decreased in the presence of the first antibody, can, but need not be the case. That is, a first antibody can inhibit the binding of a second antibody to its epitope without that second antibody inhibiting the binding of the first antibody to its respective epitope. However, where each antibody detectably inhibits the binding of the other antibody with its cognate epitope or ligand, whether to the same, greater, or lesser extent, the antibodies are said to "cross-compete" with each other for binding of their respective epitope(s). Both competing and cross-competing antibodies are encompassed by the present invention. Regardless of the mechanism by which such competition or cross-competition occurs (e.g., steric hindrance, conformational change, or binding to a common epitope, or fragment thereof), the skilled artisan would appreciate, based upon the teachings provided herein, that such competing and/or cross-competing antibodies are encompassed and can be useful for the methods disclosed herein.

As used herein, "polypeptide" refers to two or more amino acids covalently joined. The terms "polypeptide" and "protein" are used interchangeably herein.

An "isolated protein", "isolated polypeptide" or "isolated antibody" is a protein, polypeptide or antibody that by virtue of its origin or source of derivation (1) is not associated with naturally associated components that accompany it in its native state, (2) is free of other proteins from the same species, (3) is expressed by a cell from a different species, or (4) does not occur in nature. Thus, a polypeptide that is chemically synthesized or synthesized in a cellular system different from the cell from which it naturally originates will be "isolated" from its naturally associated components. A protein may also be rendered substantially free of naturally associated components by isolation, using protein purification techniques well known in the art.

In a peptide or protein, suitable conservative substitutions of amino acids are known to those of skill in this art and generally can be made without altering a biological activity of a resulting molecule. Those of skill in this art recognize that, in general, single amino acid substitutions in non-essential regions of a polypeptide do not substantially alter biological activity (see, e.g., Watson et al., Molecular Biology of the Gene, 4th Edition, 1987, The Benjamin/Cummings Pub. co., p.224).

As used herein, the terms "polynucleotide" and "nucleic acid molecule" refer to an oligomer or polymer containing at least two linked nucleotides or nucleotide derivatives, including a deoxyribonucleic acid (DNA) and a ribonucleic acid (RNA), joined together, typically by phosphodiester linkages.

As used herein, isolated nucleic acid molecule is one which is separated from other nucleic acid molecules which are present in the natural source of the nucleic acid molecule. An "isolated" nucleic acid molecule, such as a cDNA molecule, can be substantially free of other cellular material, or culture medium when produced by recombinant techniques, or substantially free of chemical precursors or other chemicals when chemically synthesized. Exemplary isolated nucleic acid molecules provided herein include isolated nucleic acid molecules encoding an antibody or antigen-binding fragments provided.

Sequence "identity" has an art-recognized meaning and the percentage of sequence identity between two nucleic acid or polypeptide molecules or regions can be calculated using published techniques. Sequence identity can be measured along the full length of a polynucleotide or polypeptide or along a region of the molecule. (See, e.g.: Computational Molecular Biology, Lesk, A.M., ed., Oxford University Press, New York, 1988; Biocomputing: Informatics and Genome Projects, Smith, D.W., ed., Academic Press, New York, 1993; Computer Analysis of Sequence Data, Part I, Griffin, A.M., and Griffin, H.G., eds., Humana Press, New Jersey, 1994; Sequence Analysis in Molecular Biology, von Heinje, G., Academic Press, 1987; and Sequence Analysis Primer, Gribskov, M. and Devereux, J., eds., M Stockton Press, New York, 1991). While there exist a number of methods to measure identity between two polynucleotides or polypeptides, the term "identity" is well known to skilled artisans (Carrillo, H. & Lipman, D., SIAM J Applied Math 48:1073 (1988)).

As used herein, "operably linked" with reference to nucleic acid sequences, regions, elements or domains means that the nucleic acid regions are functionally related to each other. For example, a promoter can be operably linked to nucleic acid encoding a polypeptide, whereby the promoter regulates or mediates the transcription of the nucleic acid.

As used herein, "expression" refers to the process by which polypeptides are produced by transcription and translation of polynucleotides. The level of expression of a polypeptide can be assessed using any method known in art, including, for example, methods of determining the amount of the polypeptide produced from the host cell. Such methods can include, but are not limited to, quantitation of the polypeptide in the cell lysate by ELISA, Coomassie blue staining following gel electrophoresis, Lowry protein assay and Bradford protein assay.

As used herein, a "host cell" is a cell that is used in to receive, maintain, reproduce and amplify a vector. A host cell also can be used to express the polypeptide encoded by the vector. The nucleic acid contained in the vector is replicated when the host cell divides, thereby amplifying the nucleic acids. The host cell may be a eukaryotic cell or a prokaryotic cell. Suitable host cells include, but are not limited to, CHO cells, various COS cells, HeLa cells, HEK cells such as HEK 293 cells.

Codon optimization refers to the replacement of at least one codon (eg, about or more than about 1, 2, 3, 4, 5, 10, 15, 20, 25, 50 or more codons) of a native sequence by a codon that is used more frequently or most frequently in the gene of the host cell, modifying the nucleic acid sequence while maintaining the native amino acid sequence to enhance expression in the host cell of interest. Different species show specific preferences for certain codons of a particular amino acid. Codon preference (difference in codon usage between organisms) is often associated with the efficiency of translation of messenger RNA (mRNA), which is believed to depend on the nature of the translated codon and the availability of specific transfer RNA (tRNA) molecules. The advantages of selected tRNAs within cells generally reflect the most frequently used codons for peptide synthesis. Therefore, genes can be customized to be best gene expressed in a given organism based on codon optimization. The codon usage table can be easily obtained, for example, in the Codon Usage Database available at www.kazusa.orjp/codon/, and these tables can be adjusted in different ways. See, Nakamura Y. et. al "Codon usage tabulated from the international DNA sequence databases: status for the year2000 Nucl.Acids Res, 28: 292 (2000).

As used herein, a "vector" is a replicable nucleic acid from which one or more heterologous proteins can be expressed when the vector is transformed into an appropriate host cell. Reference to a vector includes those vectors into which a nucleic acid encoding a polypeptide or fragment thereof can be introduced, typically by restriction digest and ligation. Reference to a vector also includes those vectors that contain nucleic acid encoding a polypeptide. The vector is used to introduce the nucleic acid encoding the polypeptide into the host cell for amplification of the nucleic acid or for expression/display of the polypeptide encoded by the nucleic acid. The vectors typically remain episomal, but can be designed to effect integration of a gene or portion thereof into a chromosome of the genome. Also contemplated are vectors that are artificial chromosomes, such as yeast artificial chromosomes and mammalian artificial chromosomes. Selection and use of such vehicles are well known to those of skill in the art.

As used herein, a vector also includes "virus vectors" or "viral vectors." Viral vectors are engineered viruses that are operatively linked to exogenous genes to transfer (as vehicles or shuttles) the exogenous genes into cells.

As used herein, an "expression vector" includes vectors capable of expressing DNA that is operatively linked with regulatory sequences, such as promoter regions, that are capable of effecting expression of such DNA fragments. Such additional segments can include promoter and terminator sequences, and optionally can include one or more origins of replication, one or more selectable markers, an enhancer, a polyadenylation signal, and the like. Expression vectors are generally derived from plasmid or viral DNA, or can contain elements of both. Thus, an expression vector refers to a recombinant DNA or RNA construct, such as a plasmid, a phage, recombinant virus or other vector that, upon introduction into an appropriate host cell, results in expression of the cloned DNA. Appropriate expression vectors are well known to those of skill in the art and include those that are replicable in eukaryotic cells and/or prokaryotic cells and those that remain episomal or those which integrate into the host cell genome.

As used herein, "treating" a subject with a disease or condition means that the subject's symptoms are partially or totally alleviated, or remain static following treatment. Hence treatment encompasses prophylaxis, therapy and/or cure. Prophylaxis refers to prevention of a potential disease and/or a prevention of worsening of symptoms or progression of a disease. Treatment also encompasses any pharmaceutical use of any antibody or antigen-binding fragment thereof provided or compositions provided herein.

As used herein, a "therapeutic effect" means an effect resulting from treatment of a subject that alters, typically improves or ameliorates the symptoms of a disease or condition or that cures a disease or condition.

As used herein, a "therapeutically effective amount" or a "therapeutically effective dose" refers to the quantity of an agent, compound, material, or composition containing a compound that is at least sufficient to produce a therapeutic effect following administration to a subject. Hence, it is the quantity necessary for preventing, curing, ameliorating, arresting or partially arresting a symptom of a disease or disorder.

As used herein, a "prophylactically effective amount" or a "prophylactically effective dose" refers to the quantity of an agent, compound, material, or composition containing a compound that when administered to a subject, will have the intended prophylactic effect, *e*.*g*., preventing or delaying the onset, or reoccurrence, of disease or symptoms, reducing the likelihood of the onset, or reoccurrence, of disease or symptoms, or reducing the incidence of viral infection. The full prophylactic effect does not necessarily occur by administration of one dose, and can occur only after administration of a series of doses. Thus, a prophylactically effective amount can be administered in one or more administrations.

As used herein, the term "patient" refers to a mammal, such as a human.

"Tumor stem cells" refer to a small group of cancer cells with sternness present in a tumor tissue, which have self-renewal ability, strong invasive ability, ability to tolerate chemotherapy drugs, and strong tumorigenic ability compared to ordinary cancer cells.

### II. Monoclonal Antibody against Tumor Stem Cells

In the present invention, a human tumor pluripotent stem cell line was used as an immunogen to immunize mice, and a monoclonal antibody Hetumomab was obtained by a classical hybridoma fusion technique. The mouse hybridoma cell line Hetumomab producing Hetumomab was deposited in China General Microbiological Culture Collection Center on March 16, 2016 under the accession number of CGMCC NO. 12251. (Example 1)

As such, the present invention provides a monoclonal antibody or antigen-binding fragment thereof, wherein the monoclonal antibody is produced by the mouse hybridoma deposited in China General Microbiological Culture Collection Center on March 16, 2016 under the accession number of CGMCC NO. 12251.

The present inventors have found that the target antigen of Hetumomab of the present invention is expressed on the surface of living cells of various human tumor cells, and is specifically highly expressed in various tumor tissues (with positive rates of 79%-94%). Hetumomab monoclonal antibody of the present invention is capable of enriching tumor cells carrying tumor stem cell markers such as ESA and CD90 from the sphere culture cells of various tumors, suggesting that Hetumomab is a monoclonal antibody specific for tumor stem cells (Example 2).

Further studies based on Hetumomab target antigen-positive tumor cells have shown that Hetumomab target antigen-positive tumor cells have stronger self-renewal ability, invasion ability, drug resistance and in vivo tumorigenicity compared to parental tumor cells and Hetumomab target antigen-negative tumor cells, which further proves that Hetumomab monoclonal antibody specifically targets tumor stem cells (Example 3). The present inventors further identified sequences of the light chain variable region and heavy chain variable region of the Hetumomab monoclonal antibody and the corresponding CDR sequences (Example 6). The human-mouse chimeric antibody Hetuximab was constructed by combining the variable region sequences with human light chain and heavy chain constant regions, respectively (Example 7). Experiments have shown that the chimeric antibody Hetuximab and the mouse antibody Hetumomab bind to the same antigenic epitope on the tumor stem cells and have the similar pharmacodynamic effect of inhibiting tumor stem cells (Examples 8-9).

The present inventors further humanized the Hetumomab monoclonal antibody to obtain various variants of humanized antibody Hetuzumab (Example 10). Experiments have shown that the humanized antibodies of Hetuzumab and the mouse antibody Hetumomab and the chimeric antibody Hetuximab bind to the same antigenic epitope on the tumor stem cells, and have the similar pharmacodynamic effect of inhibiting tumor stem cells (Examples 11-12).

As such, the present invention provides an isolated monoclonal antibody or antigen-binding fragment thereof against tumor stem cells, wherein the monoclonal antibody comprises a light chain variable region and a heavy chain variable region,
the light chain variable region comprises:
a VL CDR1 comprising an amino acid sequence set forth in SEQ ID NO: 2, or an amino acid sequence having 1 or 2 amino acid residue substitutions, deletions or additions relative to SEQ ID NO: 2,
a VL CDR2 comprising an amino acid sequence set forth in SEQ ID NO: 3, or an amino acid sequence having 1 or 2 amino acid residue substitutions, deletions or additions relative to SEQ ID NO: 3, and
a VL CDR3 comprising an amino acid sequence set forth in SEQ ID NO: 4, or an amino acid sequence having 1 or 2 amino acid residue substitutions, deletions or additions relative to SEQ ID NO: 4;
the heavy chain variable region comprises:
a VH CDR1 comprising an amino acid sequence set forth in SEQ ID NO:6, or an amino acid sequence having 1 or 2 amino acid residue substitutions, deletions or additions relative to SEQ ID NO:6,
a VH CDR2 comprising an amino acid sequence set forth in SEQ ID NO:7, or an amino acid sequence having 1 or 2 amino acid residue substitutions, deletions or additions relative to SEQ ID NO:7, such as a sequence set forth in SEQ ID NO:13, and
a VH CDR3 comprising an amino acid sequence set forth in SEQ ID NO:8, or an amino acid sequence having 1 or 2 amino acid residue substitutions, deletions or additions relative to SEQ ID NO:8, such as a sequence set forth in SEQ ID NO: 14.

In some embodiments, the monoclonal antibody comprises a light chain variable region and a heavy chain variable region,
the light chain variable region comprises:
a VL CDR1 comprising an amino acid sequence set forth in SEQ ID NO: 2,
a VL CDR2 comprising an amino acid sequence set forth in SEQ ID NO: 3, and
a VL CDR3 comprising an amino acid sequence set forth in SEQ ID NO:4;
the heavy chain variable region comprises:
a VH CDR1 comprising an amino acid sequence set forth in SEQ ID NO:6,
a VH CDR2 comprising an amino acid sequence set forth in SEQ ID NO: 13, and
a VH CDR3 comprising an amino acid sequence set forth in SEQ ID NO:8.

In some embodiments, the monoclonal antibody comprises a light chain variable region and a heavy chain variable region,
the light chain variable region comprises:
a VL CDR1 comprising an amino acid sequence set forth in SEQ ID NO: 2,
a VL CDR2 comprising an amino acid sequence set forth in SEQ ID NO: 3, and
a VL CDR3 comprising an amino acid sequence set forth in SEQ ID NO:4;
the heavy chain variable region comprises:
a VH CDR1 comprising an amino acid sequence set forth in SEQ ID NO:6,
a VH CDR2 comprising an amino acid sequence set forth in SEQ ID NO: 7, and
a VH CDR3 comprising an amino acid sequence set forth in SEQ ID NO:14.

In some embodiments, the monoclonal antibody is a humanized antibody.

In some embodiments, the light chain variable region comprises an amino acid sequence set forth in SEQ ID NO:1, or an amino acid sequence having at least 85%, at least 90%, at least 95% or higher sequence identity to SEQ ID NO: 1. In some embodiments, the light chain variable region comprises an amino acid sequence having about 90%, about 91%, about 92%, about 93%, about 94%, about 95%, about 96%, about 97%, about 98%, or about 99% sequence identity to SEQ ID NO: 1.

In some embodiments, the heavy chain variable region comprises an amino acid sequence set forth in SEQ ID NO:5, or an amino acid sequence having at least 85%, at least 90%, at least 95% or higher sequence identity to SEQ ID NO:5. In some embodiments, the heavy chain variable region comprises an amino acid sequence having about 90%, about 91%, about 92%, about 93%, about 94%, about 95%, about 96%, about 97%, about 98%, or about 99% sequence identity to SEQ ID NO:5.

In some embodiments, the heavy chain variable region comprises an amino acid sequence set forth in one of SEQ ID NOs: 15-19 (corresponding to the variable regions of the humanized heavy chain versions H1-H5, respectively).

In some embodiments, the light chain variable region comprises an amino acid sequence set forth in one of SEQ ID NOs:20-31 (corresponding to the variable regions of the humanized light chain versions L1-L12, respectively).

In some embodiments, the heavy chain variable region comprises an amino acid sequence set forth in SEQ ID NO:19, and the light chain variable region comprises an amino acid sequence set forth in SEQ ID NO: 25.

In some embodiments, the heavy chain variable region comprises an amino acid sequence set forth in SEQ ID NO:19, and the light chain variable region comprises an amino acid sequence set forth in SEQ ID NO: 30.

In some embodiments, the monoclonal antibody comprises a human heavy chain constant region, such as a human heavy chain constant region comprising an amino acid sequence set forth in SEQ ID NO:11.

In some embodiments, the monoclonal antibody comprises a human light chain constant region, such as a human light chain constant region comprising an amino acid sequence set forth in SEQ ID NO:12.

In some embodiments, the monoclonal antibody comprises a heavy chain having an amino acid sequence set forth in one of SEQ ID NOs:32-36 (corresponding to the humanized heavy chain versions H1-H5, respectively) and a light chain having an amino acid sequence set forth in one of SEQ ID NOs: 37-48 (corresponding to the humanized light chain versions L1-L12, respectively).

In some embodiments, the monoclonal antibody comprises a heavy chain set forth in SEQ ID NO: 36 and a light chain set forth in SEQ ID NO: 47 (corresponding to the humanized antibody H5L11).

In some embodiments, the monoclonal antibody comprises a heavy chain set forth in SEQ ID NO:36 and a light chain set forth in SEQ ID NO:42 (corresponding to the humanized antibody H5L6).

It is known in the art that in the case where the variable region has a glycosylation site, two adjacent protein bands for heavy chain or light chain will appear in the electrophoresis corresponding to different glycosylated forms of the antibody molecule (glycosylated isomer). Different glycosylated forms of antibody molecule may exhibit different pharmacokinetic and pharmacodynamic effects in humans. Therefore, antibodies in different glycosylated forms are difficult to be approved for marketing by drug approval authorities. Removal of excess variable region glycosylation sites will greatly facilitate the use of monoclonal antibodies in the preparation of human drugs. Thus, in some preferred embodiments, an isolated monoclonal antibody or antigen-binding fragment thereof of the invention does not comprise a glycosylation site, or the glycosylation site of the antibody is removed by mutation. For example, the glycosylation site in the heavy chain variable region set forth in SEQ ID NO: 19 and the glycosylation site in the heavy chain set forth in SEQ ID NO: 36 have been removed by mutation, and thus the heavy chain variable region set forth in SEQ ID NO: 19 and the heavy chain set forth in SEQ ID NO: 36 are preferred in the present invention.

In some embodiments, the isolated monoclonal antibody or antigen binding fragment thereof of the invention is derived from Hetumomab. In some embodiments, the isolated monoclonal antibody or antigen-binding fragment thereof of the invention binds to the same antigen on tumor stem cells as Hetumomab. In some embodiments, the isolated monoclonal antibody or antigen-binding fragment thereof of the invention binds to the same epitope on tumor stem cells as Hetumomab. In some embodiments, the isolated monoclonal antibody or antigen-binding fragment thereof of the invention competes with Hetumomab for binding to tumor stem cells.

In some embodiments, the isolated monoclonal antibody or antigen-binding fragment thereof of the invention specifically targets tumor stem cells. The tumor stem cells specifically targeted by the isolated monoclonal antibody or antigen-binding fragment thereof of the present invention include, but are not limited to, breast cancer stem cells, colorectal cancer stem cells, pancreatic cancer stem cells, prostatic cancer stem cells, liver cancer stem cells, lung cancer stem cells, and gastric cancer stem cells.

The invention further encompasses an isolated monoclonal antibody or antigen-binding fragment thereof that binds to the same antigen on tumor stem cells as Hetumomab. The invention further encompasses an isolated monoclonal antibody or antigen-binding fragment thereof that binds to the same epitope on the tumor stem cells as Hetumomab. The invention further encompasses an isolated monoclonal antibody or antigen-binding fragment thereof that competes with Hetumomab for binding to the tumor stem cell. The tumor stem cells include, but are not limited to, breast cancer stem cells, colorectal cancer stem cells, pancreatic cancer stem cells, prostatic cancer stem cells, liver cancer stem cells, lung cancer stem cells, and gastric cancer stem cells.

### III. Nucleic Acid, Vector and Method for Producing the Antibody

In another aspect, the invention provides an isolated nucleic acid molecule encoding the antibody or antigen-binding fragment thereof of the invention described above. In some embodiments, the nucleotide sequence of the nucleic acid molecule is codon optimized for the host cell for expression. In some embodiments, the nucleic acid molecule of the invention is operably linked to an expression regulatory sequence.

The invention further provides an expression vector comprising at least one nucleic acid molecule of the invention described above.

The invention further provides a host cell transformed with at least one nucleic acid molecule or expression vector of the invention described above.

In another aspect, the invention provides a method for producing the antibody or antigen-binding fragment thereof of the invention, comprising:
(i) culturing a host cell of the invention in a condition suitable for expression of the nucleic acid molecule or expression vector, and
(ii) isolating and purifying the antibody or antigen-binding fragment thereof expressed by the host cell.

The invention also relates to an isolated antibody or antigen-binding fragment thereof obtained by the method of the invention described above, which is capable of specifically targeting tumor stem cells.

### IV. Disease Treatment and/or Prevention

Tumor stem cells are a small group of cancer cells with stemness, which are present in tumor tissues and have the following biological characteristics: ability of self-renewal and replication, non-directional differentiation, high tumorigenicity, high invasion and metastasis ability, and insensitivity to either radiotherapy or chemotherapy. Due to the presence of tumor stem cells, tumors continuously grow, metastasize, and relapse. More important, tumor stem cells are resistant to almost all traditional chemotherapy drugs, radiotherapy and targeting drugs (including antibody-targeting drugs) that have been marketed in recent years. Tumor stem cells are in the G0 phase of the cell cycle and do not grow or proliferate. Chemotherapy and chemotherapy only affect cancer cells that are highly proliferating, but can not kill the tumor stem cells in G0 phase. Moreover, when a large number of rapidly growing cancer cells are killed by radiotherapy and chemotherapy, the tumor stem cells resistant to radiotherapy and chemotherapy are selected and enriched, leading to its greatly increased proportion. As tumor stem cells have strong ability of self-replication and metastasis, these tumor stem cells will rapidly differentiate and proliferate, grow and metastasize to various organs of the body to form a new metastatic lesion, and the cancer cells of this metastatic lesion are resistant to radiotherapy and chemotherapy. Tumor stem cells have been demonstrated as present in various malignancies such as breast cancer, colorectal cancer, pancreatic cancer, prostatic cancer, liver cancer, lung cancer, and gastric cancer. A cancer with higher malignancy may have more tumor stem cells, while the patient having higher proportion of tumor stem cells may have higher risk of metastasis and recurrence, and thus shorter survival period.

The present inventors have found that the monoclonal antibody of the present invention which specifically recognizes tumor stem cells can significantly inhibit the in vitro ability of self-renewal, invasion and drug resistance of various tumor stem cells (Example 4). Further experiments have shown that the monoclonal antibody of the present invention which specifically recognizes tumor stem cells is capable of inhibiting growth, metastasis and drug resistance of various tumor transplantation tumors in an animal model (Example 5). Therefore, the monoclonal antibody of the present invention may be used for treating a malignant tumor, preventing and/or treating the metastasis or relapse of the malignant tumor by targeting the tumor stem cells.

As such, the present invention provides a method for treating a malignant tumor, preventing and/or treating metastasis or relapse of a malignant tumor in a patient, the method comprising administering to the patient an effective amount of the antibody or antigen-binding fragment thereof of the invention against tumor stem cells. The malignant tumor that can be treated and/or prevented by the method of the invention includes, but is not limited to, breast cancer, colorectal cancer, pancreatic cancer, prostatic cancer, liver cancer, lung cancer, and gastric cancer.

### V. Pharmaceutical Composition

The present invention further provides a pharmaceutical composition comprising the monoclonal antibody or antigen-binding fragment thereof against tumor stem cells of the invention, and a pharmaceutically acceptable carrier. The pharmaceutical composition is used for treating a malignant tumor, preventing and/or treating metastasis or recurrence of a malignant tumor in a patient.

As used herein, "pharmaceutically acceptable carrier" includes any and all solvents, dispersion media, coatings, antibacterial and antifungal agents, isotonic and absorption delaying agents, and the like that are physiologically compatible. Preferably, the carrier is suitable for intravenous, intramuscular, subcutaneous, parenteral, spinal or epidermal administration (e.g., by injection or infusion). Depending on the route of administration, the active compound, i.e., antibody, or conjugate, may be coated in a material to protect the compound from the action of acids and other natural conditions that may inactivate the compound.

A pharmaceutical composition of the invention also may include a pharmaceutically acceptable anti-oxidant. Examples of pharmaceutically acceptable antioxidants include: (1) water soluble antioxidants, such as ascorbic acid, cysteine hydrochloride, sodium bisulfate, sodium metabisulfite, sodium sulfite and the like; (2) oil-soluble antioxidants, such as ascorbyl palmitate, butylated hydroxyanisole (BHA), butylated hydroxytoluene (BHT), lecithin, propyl gallate, alpha-tocopherol, and the like; and (3) metal chelating agents, such as citric acid, ethylenediamine tetraacetic acid (EDTA), sorbitol, tartaric acid, phosphoric acid, and the like.

These compositions may also contain adjuvants such as preservatives, wetting agents, emulsifying agents and dispersing agents.

Prevention of presence of microorganisms may be ensured both by sterilization procedures, supra, and by the inclusion of various antibacterial and antifungal agents, for example, paraben, chlorobutanol, phenol sorbic acid, and the like. It may also be desirable to include isotonic agents, such as sugars, sodium chloride, and the like into the compositions. In addition, prolonged absorption of the injectable pharmaceutical form may be brought about by the inclusion of agents which delay absorption such as aluminum monostearate and gelatin.

Pharmaceutically acceptable carriers include sterile aqueous solutions or dispersions and sterile powders for the extemporaneous preparation of sterile injectable solutions or dispersion. The use of such media and agents for pharmaceutically active substances is known in the art. Except insofar as any conventional media or agent is incompatible with the active compound, use thereof in the pharmaceutical compositions of the invention is contemplated. Supplementary active compounds can also be incorporated into the compositions.

Therapeutic compositions typically must be sterile and stable under the conditions of manufacture and storage. The composition can be formulated as a solution, microemulsion, liposome, or other ordered structure suitable to high drug concentration. The carrier can be a solvent or dispersion medium containing, for example, water, ethanol, polyol (for example, glycerol, propylene glycol, and liquid polyethylene glycol, and the like), and suitable mixtures thereof. The proper fluidity can be maintained, for example, by the use of a coating such as lecithin, by the maintenance of the required particle size in the case of dispersion and by the use of surfactants.

Sterile injectable solutions can be prepared by incorporating the active compound in the required amount in an appropriate solvent with one or a combination of ingredients enumerated above, as required, followed by sterilization microfiltration. Generally, dispersions are prepared by incorporating the active compound into a sterile vehicle that contains a basic dispersion medium and the required other ingredients from those enumerated above. In the case of sterile powders for the preparation of sterile injectable solutions, the preferred methods of preparation are vacuum drying and freeze-drying (lyophilization) that yield a powder of the active ingredient plus any additional desired ingredient from a previously sterile-filtered solution thereof.

The amount of active ingredient which can be combined with a carrier material to produce a single dosage form will vary depending upon the subject being treated, and the particular mode of administration. The amount of active ingredient which can be combined with a carrier material to produce a single dosage form will generally be that amount of the composition which produces a therapeutic effect. Generally, this amount will range from about 0.01% to about 99% of active ingredient, preferably from about 0.1% to about 70%, most preferably from about 1 %to about 30 %of active ingredient in combination with a pharmaceutically acceptable carrier.

Dosage regimens are adjusted to provide the optimum desired response (e.g., a therapeutic response). For example, a single bolus may be administered, several divided doses may be administered over time or the dose may be proportionally reduced or increased as indicated by the exigencies of the therapeutic situation. It is especially advantageous to formulate parenteral compositions in dosage unit form for ease of administration and uniformity of dosage. Dosage unit form as used herein refers to physically discrete units suited as unitary dosages for the subjects to be treated; each unit contains a predetermined quantity of active compound calculated to produce the desired therapeutic effect in association with the required pharmaceutical carrier. The specification for the dosage unit forms of the invention are dictated by and directly dependent on (a) the unique characteristics of the active compound and the particular therapeutic effect to be achieved, and (b) the limitations inherent in the art of compounding such an active compound for the treatment of sensitivity in individuals.

For administration of the antibody molecule, the dosage ranges from about 0.0001 to 100 mg/kg, and more usually 0.01 to 20 mg/kg, of the subject body weight. For example dosages can be 0.3 mg/kg body weight, 1 mg/kg body weight, 3 mg/kg body weight, 5 mg/kg body weight, 10 mg/kg body weight or 20 mg/kg body weight or within the range of 1-20 mg/kg. An exemplary treatment regime entails administration once per week, once every 2 weeks, once every 3 weeks, once every 4 weeks, once a month, once every 3 months or once every 3 to 6 months, or with a short administration interval at the beginning (such as once per week to once every 3 weeks), and then an extended interval later (such as once a month to once every 3 to 6 months).

Alternatively, antibody against tumor stem cells can be administered as a sustained release formulation, in which case less frequent administration is required. Dosage and frequency vary depending on the half-life of the antibody in the patient. In general, human antibodies show the longest half life, followed by humanized antibodies, chimeric antibodies, and nonhuman antibodies. The dosage and frequency of administration can vary depending on whether the treatment is prophylactic or therapeutic. In prophylactic applications, a relatively low dosage is administered at relatively infrequent intervals over a long period of time. Some patients continue to receive treatment for the rest of their lives. In therapeutic applications, a relatively high dosage at relatively short intervals is sometimes required until progression of the disease is reduced or terminated, and preferably until the patient shows partial or complete amelioration of symptoms of disease. Thereafter, the patient can be administered a prophylactic regime.

Actual dosage levels of the active ingredients in the pharmaceutical compositions of the present invention may be varied so as to obtain an amount of the active ingredient which is effective to achieve the desired therapeutic response for a particular patient, composition, and mode of administration, without being toxic to the patient. The selected dosage level will depend upon a variety of pharmacokinetic factors including the activity of the particular compositions of the present invention employed, or the ester, salt or amide thereof, the route of administration, the time of administration, the rate of excretion of the particular compound being employed, the duration of the treatment, other drugs, compounds and/or materials used in combination with the particular compositions employed, the age, sex, weight, condition, general health and prior medical history of the patient being treated, and like factors well known in the medical arts.

A "therapeutically effective amount" of the antibody or antigen-binding fragment thereof of the invention preferably results in a decrease in severity of disease symptoms, an increase in frequency and duration of disease symptom-free periods, or a prevention of impairment or disability due to the disease affliction. For example, for the treatment of tumors, a "therapeutically effective amount " preferably inhibits cell growth or tumor growth by at least about 10%, at least about 20%, more preferably by at least about 40%, even more preferably by at least about 60%, and still more preferably by at least about 80% relative to untreated subjects. The ability to inhibit tumor growth can be evaluated in an animal model system predictive of efficacy in human tumors. Alternatively, this property of a composition can be evaluated by examining the ability of the compound to inhibit cell growth; such inhibition can be determined in vitro by assays known to the skilled practitioner. A therapeutically effective amount of a therapeutic compound can decrease tumor size, or otherwise ameliorate symptoms in a subject. One of ordinary skill in the art would be able to determine such amounts based on such factors as the subject's size, the severity of the subject's symptoms, and the particular composition or route of administration selected.

The antibody or antigen-binding fragment thereof of the invention or the pharmaceutical composition of the invention can be administered via one or more routes of administration using one or more of a variety of methods known in the art. As will be appreciated by the skilled artisan, the route and/or mode of administration will vary depending upon the desired results. Preferred routes of administration for PDL1-binding molecules of the invention include intravenous, intramuscular, intradermal, intraperitoneal, subcutaneous, spinal or other parenteral routes of administration, for example by injection or infusion. The phrase "parenteral administration" as used herein means modes of administration other than enteral and topical administration, usually by injection, and includes, without limitation, intravenous, intramuscular, intraarterial, intrathecal, intracapsular, intraorbital, intracardiac, intradermal, intraperitoneal, transtracheal, subcutaneous, subcuticular, intraarticular, subcapsular, subarachnoid, intraspinal, epidural and intrasternal injection and infusion.

Alternatively, the antibody or antigen-binding fragment thereof against tumor stem cells of the present invention or the pharmaceutical composition of the present invention can be administered via a non-parenteral route, such as a topical, epidermal or mucosal route of administration, for example, intranasally, orally, vaginally, rectally, sublingually or topically.

The active compounds can be prepared with carriers that will protect the compound against rapid release, such as a controlled release formulation, including implants, transdermal patches, and microencapsulated delivery systems. Biodegradable, biocompatible polymers can be used, such as ethylene vinyl acetate, polyanhydrides, polyglycolic acid, collagen, polyorthoesters, and polylactic acid. Many methods for the preparation of such formulations are patented or generally known to those skilled in the art. See, e.g., Sustained and Controlled Release Drug Delivery Systems, J.R. Robinson, ed., Marcel Dekker, Inc., New York, 1978.

Therapeutic compositions can be administered with medical devices known in the art. For example, in a preferred embodiment, a therapeutic composition of the invention can be administered with a needleless hypodermic injection device, such as the devices disclosed in U.S. Patent Nos. 5,399,163; 5,383,851; 5,312,335; 5,064,413; 4,941,880; 4,790,824; or 4,596,556. Examples of well-known implants and modules useful in the present invention include: U.S. Patent No. 4,487,603, which discloses an implantable micro-infusion pump for dispensing medication at a controlled rate; U.S. Patent No. 4,486, 194, which discloses a therapeutic device for administering medicaments through the skin; U.S. Patent No. 4,447,233, which discloses a medication infusion pump for delivering medication at a precise infusion rate; U.S. Patent No. 4,447,224, which discloses a variable flow implantable infusion apparatus for continuous drug delivery; U.S. Patent No. 4,439,196, which discloses an osmotic drug delivery system having multi-chamber compartments; and U.S. Patent No. 4,475,196, which discloses an osmotic drug delivery system. These patents are incorporated herein by reference. Many other such implants, delivery systems, and modules are known to those skilled in the art.

In certain embodiments, the antibody of the invention against tumor stem cells can be formulated to ensure proper distribution in vivo. For example, the blood-brain barrier (BBB) excludes many highly hydrophilic compounds. To ensure that the therapeutic compounds of the invention cross the BBB (if desired), they can be formulated, for example, in liposomes. For methods of manufacturing liposomes, see, e.g., U.S. Patents 4,522,811; 5,374,548; and 5,399,331. The liposomes may comprise one or more moieties which are selectively transported into specific cells or organs, thus enhance targeted drug delivery (see, e.g., V. V. Ranade (1989) J. Clin. Pharmacol. 29:685). Exemplary targeting moieties include folate or biotin (see, e.g., U.S. Patent 5,416,016 to Low et al); mannosides (Umezawa et al., (1988) Biochem. Biophys. Res. Commun. 153:1038): antibodies (P.G. Bloeman et al. (1995) FEBS Lett. 357:140; M. Owais et al. (1995) Antimicrob. Agents Chemother. 39:180); surfactant protein A receptor (Briscoe et al. (1995) Am. J. Physiol. 1233: 134); pl20 (Schreier et al. (1994) J Biol. Chem. 269:9090): see also K. Keinanen; M.L. Laukkanen (1994) FEBS Lett. 346:123; JJ. Killion; LJ. Fidler (1994) Immunomethods 4:273.

The antibody or antigen-binding fragment thereof of the present invention against tumor stem cells in the pharmaceutical composition may also be conjugated to a therapeutic moiety such as a cytotoxin, a radioisotope or a biologically active protein.

A cytotoxin includes any agent that is detrimental to (e.g., kills) cells. Examples include taxol, cytochalasin B, gramicidin D, ethidium bromide, emetine, mitomycin, etoposide, tenoposide, vincristine, vinblastine, colchicin, doxorubicin, daunorubicin, dihydroxy anthracin dione, mitoxantrone, mithramycin, actinomycin D, 1-dehydrotestosterone, glucocorticoids, procaine, tetracaine, lidocaine, propranolol, and puromycin and analogs or homologs thereof.

Therapeutic agents that can be conjugated also include, for example, antimetabolites (e.g., methotrexate, 6-mercaptopurine, 6-thioguanine, cytarabine, 5-fluorouracil decarbazine), alkylating agents (e.g., mechlorethamine, thioepa chlorambucil, melphalan, carmustine (BSNU) and lomustine (CCNU), cyclothosphamide, busulfan, dibromomannitol, streptozotocin, mitomycin C, and cis-dichlorodiamine platinum (II) (DDP) cisplatin), anthracyclines (e.g., daunorubicin (formerly daunomycin) and doxorubicin), antibiotics (e.g., dactinomycin (formerly actinomycin), bleomycin, mithramycin, and anthramycin (AMC)), and anti-mitotic agents (e.g., vincristine and vinblastine).

Other preferred examples of therapeutic cytotoxins which can be conjugated to the antibody against tumor stem cells of the present invention include duocarmycins, calicheamicins, maytansines and auristatins, and derivatives thereof.

Cytotoxins can be conjugated to the antibody against tumor stem cells of the invention using linker technology available in the art. Examples of linker types that have been used to conjugate a cytotoxin to antibody against tumor stem cells of the invention include, but are not limited to, hydrazones, thioethers, esters, disulfides and peptide-containing linkers. A linker can be chosen that is, for example, susceptible to cleavage by low pH within the lysosomal compartment or susceptible to cleavage by proteases, such as proteases preferentially expressed in tumor tissue such as cathepsins (e.g., cathepsins B, C, D).

For further discussion of types of cytotoxins, linkers and methods for conjugating therapeutic agents to antibodies, see Saito, G. et al. (2003) Adv. Drug Deliv. Rev. 55:199-215; Trail, PA et al. (2003) Cancer. Immunol. Immunother. 52:328-337; Payne, G. (2003) Cancer Cell 3: 207-212; Allen, T. M. (2002) Nat. Rev. Cancer 2: 750-763; Pastan, I. and Kreitman, R. J. (2002) Curr. Opin. Investig. Drugs 3: 1089-1091; Senter, P. D. and Springer, C. J. (2001) Adv. Drug Deliv. Rev. 53: 247-264.

The antibody against tumor stem cells of the invention may also be conjugated to a radioisotope to produce a cytotoxic radiopharmaceutical, also known as a radioactive antibody conjugate. Examples of radioactive isotopes that can be conjugated to antibodies for use diagnostically or therapeutically include, but are not limited to, iodine¹³¹, indium¹¹¹, yttrium⁹⁰ and lutetium¹⁷⁷. Methods for preparing a radioactive antibody conjugate have been established in the art.

The antibody against tumor stem cells of the invention may also be conjugated to to a protein with desired biological activity to modify a given biological response. Such proteins may include, for example, an enzymatically active toxin, or active fragment thereof, such as abrin, ricin A, pseudomonas exotoxin, or diphtheria toxin; a protein such as tumor necrosis factor or interferon-y; or, biological response modifiers such as, for example, lymphokines, interleukin-1 ("IL-1"), interleukin-2 ("IL-2"), interleukin-6 ("IL-6"), granulocyte macrophage colony stimulating factor ("GM-CSF"), granulocyte colony stimulating factor ("G-CSF"), or other immune factors such as IFN.

Techniques for conjugating such therapeutic moieties to antibody molecules are well known, see, for example, Arnon et al., "Monoclonal Antibodies For Immunotargeting Of Drugs In Cancer Therapy", Monoclonal Antibodies And Cancer Therapy, Reisfeld et al. (ed.), pp. 243-56 (Alan R. Liss, Inc. 1985); Hellstrom et al., "Antibodies For Drug Delivery", Controlled Drug Delivery (2nd Ed.), Robinson et al. (ed.), pp. 623-53 (Marcel Dekker, Inc. 1987); Thorpe, "Antibody Carriers Of Cytotoxic Agents In Cancer Therapy: A Review", Monoclonal Antibodies' 84: Biological And Clinical Applications, Pinchera et al. (ed.), pp. 475-506 (1985); "Analysis, Results, And Future Prospective Of therapeutic Use Of Radiolabeled Antibody In Cancer Therapy", Monoclonal Antibodies For Cancer Detection And Therapy, Baldwin et al. (ed.), pp. 303-16 (Academic Press 1985), and Thorpe et al., "The Preparation And Cytotoxic Properties Of Antibody-Toxin Conjugates, "Immunol. Rev., 62: 119-58 (1982).

### VI. Combination therapy

The antibody or pharmaceutical composition of the present invention against tumor stem cells may be administered in combination with a chemotherapeutic agent or an antibody targeting other tumor antigens. Example 5 of the present application demonstrates that the monoclonal antibody of the present invention in combination with a chemotherapeutic agent has a synergistic effect in the treatment of a tumor. Without being bound by any theory, it is believed that the antibodies against tumor stem cells of the present invention are capable of inhibiting drug resistance of tumors, thereby enabling synergistic effects when administered in combination with chemotherapeutic agents or antibodies targeting other tumor antigens.

The chemotherapeutic agents or the antibody targeting other tumor antigens which can be used in combination with the antibody of the present invention or the pharmaceutical composition of the present invention are not particularly limited. Examples of such chemotherapeutic agents and antibodies targeting other tumor antigens include, but are not limited to, ifosfamide, cyclophosphamide, dacarbazine, temozolomide, nimustine, busulfan, melphalan, enocitabine, capecitabine, carmofur, cladribine, gemcitabine, cytarabine, tegafur, tegafur-uracil, TS-1, doxifluridine, nelarabine, hydroxyurea, Fluorouracil, Fludarabine, pemetrexed, pentostatin, mercaptopurine, methotrexate, Irinotecan, etoposide, Eribulin, Sobuzoxane, docetaxel, paclitaxel, vinorelbine, Vincristine, Vindesine, Vinblastine, actinomycin D, Aclarubicin, amrubicin, idarubicin, epirubicin, Zinostatin, daunorubicin, doxorubicin, pirarubicin, bleomycin, peplomycin, mitomycin C, mitoxantrone, oxaliplatin, carboplatin, cisplatin, nedaplatin, anastrozole, exemestane, ethinyl estradiol, Chlormadinone, goserelin, tamoxifen, dexamethasone, bicalutamide, toremifene, flutamide, prednisolone, fosfestrol, Mitotane, methyltestosterone, Leuprorelin, letrozole, methylmedroxyprogesterone, ibritumomab tiuxetan, Imatinib, everolimus, Erlotinib, Gefitinib, Sunitinib, cetuximab, Sorafenib, Dasatinib, Tamibarotene, Trastuzumab, retinoic acid, Keytruda, bevacizumab, Bortezomib, and Lapatinib. In a specific embodiment, the chemotherapeutic agent is a platinum-containing chemotherapeutic agent, such as cisplatin.

The antibodies of the invention and the chemotherapeutic agents or antibodies targeting other tumor antigens can be administered in one administration or administered separately. When administered separately (in respective different administration regimens), they can be administered sequentially without intervals or administered at predetermined intervals.

The doses of the antibody of the present invention and the chemotherapeutic agent or antibody targeting other tumor antigens in the pharmaceutical composition of the present invention are not particularly limited. As described above, the dose of the antibody of the present invention can be determined by referring to the dose when the antibody is used alone. The chemotherapeutic agent and the antibody targeting other tumor antigens can be used according to the dose indicated by the respective drug or the dose may be reduced (considering the effect combined with the antibody of the present invention).

The antibody of the invention or the pharmaceutical composition of the invention may also be combined with radiotherapy, for example, administering to the patient ionizing radiation before, during, and/or after the administration of the antibody or pharmaceutical composition of the invention.

### VII. Detection and Purification of Tumor Stem Cells

As described herein, the monoclonal antibody of the invention specifically recognizes tumor stem cells. As such the invention further provides a method for detecting the presence of tumor stem cells in a biological sample, comprising:
a) contacting the biological sample with the monoclonal antibody or antigen-binding fragment thereof of the invention;
b) detecting the binding of the monoclonal antibody or antigen-binding fragment thereof of the invention to a target antigen in said biological sample, wherein the detected binding indicates the presence of tumor stem cells in the biological sample.

In some embodiments, the tumor stem cells are selected from the group consisting of breast cancer stem cells, colorectal cancer stem cells, pancreatic cancer stem cells, prostatic cancer stem cells, liver cancer stem cells, lung cancer stem cells, and gastric cancer stem cells.

In some embodiments of the above detection methods of the invention, the monoclonal antibodies or antigen-binding fragments thereof of the invention are further conjugated with fluorescent dyes, chemicals, polypeptides, enzymes, isotopes, tags and the like which are used for detection or can be detected by other reagents.

Methods for detecting antibody-antigen binding are known in the art, such as ELISA and the like.

The invention further provides a method for isolating tumor stem cells, the method comprising:
(a) providing a cell population suspected of containing tumor stem cells;
(b) identifying a subpopulation of said cells that bind to the monoclonal antibody or antigen-binding fragment thereof of the invention; and
(c) isolating the subpopulation.

For example, tumor stem cells may be isolated by flow cytometry.

### VIII. Diagnosis and Prognosis

As mentioned in this disclosure, the target antigen of the monoclonal antibody of the present invention is expressed on the surface of various human living tumor cells, and is specifically highly expressed in various tumor tissues (positive rates: 79% - 94%).

As such, the invention further provides a method for detecting the presence of a malignant tumor in a patient, comprising:
a) contacting a biological sample obtained from said patient with the monoclonal antibody or antigen-binding fragment thereof of the invention;
b) detecting the binding of the monoclonal antibody or antigen-binding fragment thereof of the invention to a target antigen in said biological sample, wherein the detected binding indicates the presence of a malignant tumor in said patient.

The invention further provides a method for prognosis of relapse or progression of a malignant tumor in a patient, the method comprising:
(a) isolating a biological sample comprising circulating cells from the patient;
(b) contacting said biological sample comprising the circulating cells with the monoclonal antibody or antigen-binding fragment thereof of the invention;
(c) identifying the presence of the circulating cells that bind to the monoclonal antibody or antigen-binding fragment thereof of the invention,
thereby prognosing the relapse or progression of the malignant tumor in the patient.

In some embodiments, the progression of the malignant tumor comprises metastasis of the malignant tumor in the patient.

The identified presence of circulating cells that bind to the monoclonal antibody or antigen-binding fragment thereof of the invention suggests a high risk of relapse or progression of the malignant tumor in the patient.

In some embodiments, the biological sample comprises a blood sample, a lymph sample, or components thereof.

In some embodiments, the malignant tumor is selected from the group consisting of breast cancer, colorectal cancer, pancreatic cancer, prostatic cancer, liver cancer, lung cancer, and gastric cancer.

In some embodiments of the above methods of the invention, the monoclonal antibodies or antigen-binding fragments thereof of the invention are further conjugated with fluorescent dyes, chemicals, polypeptides, enzymes, isotopes, tags and the like which are used for detection or can be detected by other reagents.

Methods for detecting the antibody-antigen binding are known in the art, such as ELISA and the like.

### IX. Kit

Also included within the scope of the invention is a kit for use in the method of the invention, which comprises the monoclonal antibody or antigen-binding fragments thereof of the invention, and instructions for use. The kit may further comprise at least one additional detection reagent for detecting the presence of the monoclonal antibody of the invention. The kit generally comprises a label indicating the intended use and/or method for use of the contents of the kit. The term label includes any writing, or recorded material supplied on or with the kit, or which otherwise accompanies the kit.

### Examples

A further understanding of the present invention may be obtained by reference to the specific examples set forth herein, which are only intended to illustrate the invention, and are not intended to limit the scope of the invention. It is apparent that various modifications and variations may be made to the present invention without departing from the spirit of the invention, and such modifications and variations are therefore also within the scope of the present invention.

### Example 1 Preparation of Mouse Monoclonal Antibody Hetumomab

### 1. Preparation of Mouse Monoclonal Antibody Library Against Human Tumor Pluripotent Stem Cells

This example employs a human tumor pluripotent stem cell line T3A-A3 as an immunogen (Liu H, et al., Cell Death and Disease. 2013, 4: e857). The human tumor stem cell line was isolated from a liver cancer tissue surgically resected from a primary liver cancer patient, and can be subcultured in vitro for a long time. The cell line has been passaged for more than 100 times and the cells are still growing rapidly and retain properties of stem cells. The cell line expresses markers of various stem cells, has the ability of self-renewal of stem cells, and the potential to directionally differentiate into different tumor cells; and also has the properties of a tumor, and has the ability of tumorigenesis and metastasis. The cell line has been cultured in vitro for a long time with the same properties maintained, and has strong tumorigenic and metastatic ability in immunodeficient mice.

The liver cancer stem cell (human tumor pluripotent stem cell) line T3A-A3 obtained by expanding culture were fixed with paraformaldehyde, and normal Balb/c mice were immunized, once every 2-4 weeks, about 1×10⁷ cells each time. Long-term immunization was carried out until the titer of serum against human liver cancer stem cell (human tumor pluripotent stem cell) line T3A-A3 of the immunized mouse determined by conventional cellular immunochemistry achieved above 1:50000. Mouse spleen cells and mouse myeloma cells SP2/0 were fused by conventional PEG-mediated fusion to form hybridoma secreting mouse monoclonal antibodies. Hybridoma monoclones were prepared by a conventional methylcellulose plate method, and after its growth, each monoclone was picked up to a 96-well plate and cultured, thereby obtaining a hybridoma clone library containing a large amount of monoclonal antibodies against human liver cancer stem cell (human tumor pluripotent stem cell) line T3A-A3. The culture supernatant of each hybridoma clone in the 96-well plate was collected for use in the next determination and screening process.

### 2. Screening of Mouse Monoclonal Antibody Hetumomab Against Human Tumor Pluripotent Stem Cells

The serum-free suspension medium was DMEM/F12 (1:1) culture medium containing 20 ng/mL EGF, 20 ng/mL bFGF, B27 added at a ratio of 1:50, 10 ng/mL LIF, 2 mmol/mL glutamine, 1 µ/mL Heparin. The cells were washed once before culturing with the serum-free medium. The sphere cells of human liver cancer stem cell (human tumor pluripotent stem cell) line T3A-A3 cultured in the serum-free suspension medium were gently blown into single cells and seeded into a 96-well plate at 2000 cells/well. After culturing for 24 h in the serum-free medium, the cells were washed with PBS containing 1% BSA, and incubated for 2 hours at room temperature after adding 100µL culture supernatant of one hybridoma clone to each well. After washing with PBS containing 1% BSA for 5 times, biotin-labeled anti-mouse secondary antibody was added and reacted at room temperature for 30 minutes. After washing again with PBS containing 1% BSA for 5 times, Cy3-labeled Avidin was added and reacted for 30 minutes at room temperature. After washing with PBS containing 1% BSA for 5 times, fluorescence microscopy was performed under a fluorescence microscope to determine the reaction of each monoclonal antibody hybridoma supernatant in the monoclonal antibody library with the human liver cancer stem cell (human tumor pluripotent stem cell) line T3A-A3. It can be observed that some cells had fluorescent staining, which made them determined as positive, and mouse monoclonal antibodies capable of binding to the membrane surface antigens of the human liver cancer stem cell (human tumor pluripotent stem cell) line T3A-A3 were initially screened out.

Further, sphere cells of the human liver cancer cell line (Yan Li, Zhao-You Tang, Sheng-Long Ye, Yin-Kun Liu, Jie CHEN, Qiong Xue, Jun Chen, Dong-Mei Gao, Wei-Hua Bao, Establishment of cell clones with different metastatic potential from the metastatic hepatocellular carcinoma cell line MHCC97, World Journal of Gastroenterology (English Edition). 2001, 7(05):630-636) cultured in the serum-free suspension medium were selected for repeating the above steps again. Microscopic examination was used to determine the reaction of the hybridoma monoclonal antibody supernatant in the monoclonal antibody library with the human liver cancer stem cells (as MHCC97L sphere cells are rich in liver cancer stem cells), so as to obtain mouse monoclonal antibodies capable of binding to the membrane surface antigens of the liver cancer stem cells.

One mouse monoclonal antibody Hetumomab was screened out from the mouse monoclonal antibody library against human tumor pluripotent stem cells, which not only bound to the membrane surface antigens of the human tumor pluripotent stem cell line T3A-A3, but also to the membrane surface antigens of stem cells of the human liver cancer MHCC97L cell line (MHCC97L sphere cells), demonstrating that the mouse monoclonal antibody Hetumomab was able to recognize human liver cancer stem cells from different sources. The mouse monoclonal antibody Hetumomab was selected for further characterization and pharmaceutical efficacy studies.

Mouse hybridoma cells secreting the Hetumomab monoclonal antibody were deposited in the China General Microbiological Culture Collection Center (CGMCC) (Institute of Microbiology of Chinese Academy of Sciences, Building 3, No. 1 Beichen West Road, Chaoyang District, Beijing) on March 16, 2016 under the accession number of CGMCC NO. 12251.

Hetumomab hybridoma cells were expanded to collect the antibody-containing supernatant. Southern Biotech's monoclonal antibody subclass detection kit was used to identify the type and subclass of the monoclonal antibody, and Sigma's ELISA secondary antibody was used to detect antibody production of the supernatant. The experimental results showed that Hetumomab is an antibody with an IgG1 heavy chain, a κ-type light chain.

The hybridoma cells secreting Hetumomab were expanded in vitro, and after the cells were grown to 80%, the serum-free medium was refreshed to continue to culture for 4-5 days, and the serum-free supernatant with secreted antibody was then collected. Hetumomab was purified using an anti-Protein G purification column. The purity of the isolated and purified Hetumomab monoclonal antibody was determined by 10% SDS-PAGE after Coomassie blue staining. The result showed that Hetumomab had a heavy chain with molecular weight of about 47 kDa and a light chain with molecular weight of about 26 kDa, which are consistent with theoretical molecular weights of the heavy and light chain of a general IgG antibody. Through scan and analysis, it was determined that the purity of the electrophoresis target band was above 95%, and the purity of the purified Hetumomab monoclonal antibody met the requirements of the subsequent experiments.

### Example 2 Specific Expression of Hetumomab Target Antigen in Various Tumor Cells and Tissues

### 1. Target antigen of Hetumomab is expressed in a variety of cancer cell lines of human liver cancer, lung cancer, gastric cancer, and is expressed on the surface of living cells.

The expression of monoclonal antibody Hetumomab target antigen on the surface of living cells in various human tumor cell lines such as liver cancer, lung cancer and gastric cancer cell lines was detected by conventional viable cell immunofluorescence staining. The specific technical methods are as follows: cells were grown on slides or inoculated in a 96-well culture plate (4×10³ cells/well) and grown to 60% to 70% of the full plate, then washed with serum-free culture fluid twice, washed with PBS once. Primary antibody (hybridoma supernatant or purified antibody) was added (with mouse anti-α-tublin antibody (dilution: 1:1000) as control for cell permeability, and the normal mouse IgG, SP2/0 supernatant, PBS as negative control), and then incubated at room temperature for 1 h; washed with the live cell washing liquid (PBS containing 1% BSA) for 5 times and 5 min each time, and then fixed for 15 min with 4% paraformaldehyde at room temperature; washed with fix cell washing liquid (PBS containing 0.1% BSA, 0.05% tween-20) for 5 times and 5 min each time. 100 µl secondary antibody was added and incubated for 30 min at room temperature devoid of light; washed with fixed cell washing liquid for 5 times and 4 min each time; blocked with 50 µl PBS containing 10 µg/ml DAPI, 50% glycerol; then observed under the microscope. Membrane fluorescence staining observed in some cells was deemed as positive.

It was found that the target antigen of Hetumomab can be expressed on the surface of living cells of the following cell lines of human liver cancer, lung cancer, and gastric cancer (purchased from the Cell Resource Center of the Institute of Basic Medical Sciences, Chinese Academy of Medical Sciences):
Human liver cancer cell line: MHCC97L, Bel7402-V13;
Human lung cancer cell line: A549, SPCA-1;
Human gastric cancer cell line: SNU-5, BGC-823.

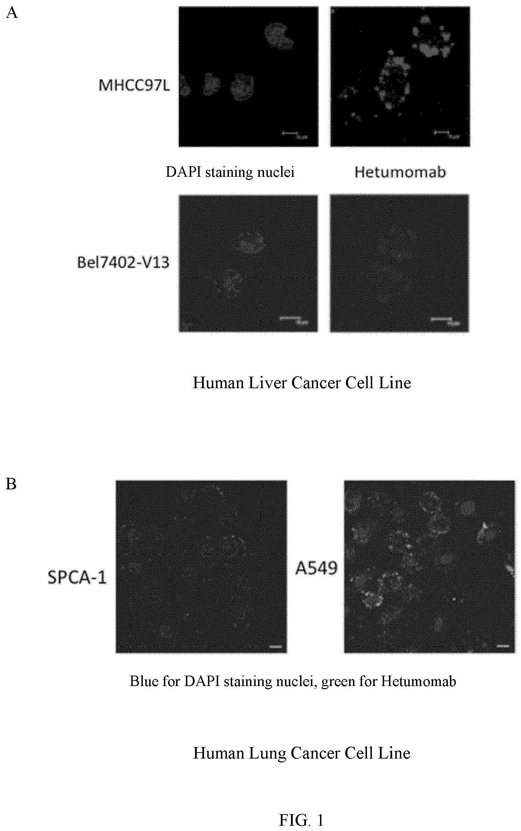

Some typical positive results are shown in FIG. 1.

The above results indicate that the target antigen of Hetumomab is expressed in various human tumors, such as liver cancer, lung cancer, and gastric cancer cell lines, and is expressed on the membrane surface of living cancer cells.

### 2. The target antigen of Hetumomab is specifically highly expressed in human liver cancer, lung cancer, and gastric cancer tissues.

By using conventional immunohistochemical techniques, the expression of the target antigen of Hetumomab in many human liver cancer, lung cancer, gastric cancer patients and other related tissues was detected by taking Hetumomab as the primary antibody, and the anti-mouse antibody as the secondary antibody.

The specific technical method is briefly described as follows: performing common deparaffin to the tissue section slide; pouring a citrate buffer (pH 6.0) into a antigen restoration box, and placing the slide into the box, and then placing the restoration box in boiling water, and heating it in water bath for 30 min and naturally cooling at room temperature for 2 h; washing with PBS for 3 min × 3 times, spin-drying the water on the slide, and then immediately drawing circles with a histochemical pen along the tissues; adding a drop of endogenous peroxidase blocking solution to each tissue, incubating for 20 min at room temperature, washing with PBS for 3min × 3 times, shaking off the washing liquid, and adding a drop of normal animal serum (sheep serum) for blocking, and incubating for 20 min at room temperature; shaking off the blocking serum, adding the primary antibody to each tissue point, placing the slide in a humid box, incubating overnight at 4 °C; shaking off the primary antibody, washing with PBS for 3 min × 3 times, shaking off the washing liquid, adding a secondary antibody biotin - antibody against mouse antibody, incubating for 20 min at room temperature; washing with PBS for 3 min × 5 times, shaking off the washing liquid, adding a drop of Avidin-HRP, incubating for 10 min at room temperature; washing with PBS for 3 min × 3 times, shaking off the washing liquid, adding a drop of freshly prepared DAB, and then observing under a microscope while strictly timing, and after color develops, stopping the reaction by rinsing with water; performing hematoxylin counterstaining for 5 min, and treating with 1% hydrochloric acid - 75% alcohol for 2 seconds, and rinsing with water; mounting the section with Neutral balsam after dehydration, and observing under a microscope.

By using Hetumomab as the primary antibody, the expression of the target antigen of Hetumomab was detected in human liver cancer tissues of 120 cases, para-carcinoma tissues of 20 cases, normal liver tissues of 10 cases, hepatitis tissues of 10 cases and hepatic cirrhosis tissues of 40 cases. The experimental results (Table 1) showed that the target antigen of Hetumomab was specifically expressed in 79.17% (95/120) of human liver cancer tissues, but not expressed in para-carcinoma tissues, normal liver tissues, hepatitis tissues and hepatic cirrhosis tissues, which indicates that the target antigen of Hetumomab is specifically highly expressed in human liver cancer tissues.

**Table 1. Detection of target antigen of Monoclonal Antibody Hetumomab in Human liver cancer Tissues by Immunohistochemistry**

| Type of Tissue | Total Number of Cases | Number of Positive Cases | Proportion of Positive Cases (%) |
|---|---|---|---|
| Liver Cancer Tissue | 120 | 95 | 79.17 |
| Liver Cancer Para-carcinoma Tissue | 20 | 0 | 0 |
| Normal Liver Tissue | 20 | 0 | 0 |
| Hepatitis | 10 | 0 | 0 |
| Hepatic Cirrhosis | 40 | 0 | 0 |

By using Hetumomab as the primary antibody, the expression of the target antigen of Hetumomab was detected in human lung cancer tissues of 160 cases, para-carcinoma tissues of 32 cases, and normal lung tissues of 3 cases. The experimental results (Table 2) showed that the target antigen of Hetumomab was specifically expressed in 82.5% (132/160) of human lung cancer tissues, but only expressed in 6.25% of the para-carcinoma tissues, and not expressed in normal lung tissues, which indicates that the target antigen of Hetumomab is specifically highly expressed in human lung cancer tissues.

**Table 2. Detection of target antigen of Hetumomab in Human Lung Cancer Tissues by Immunohistochemistry**

| Type of Tissue | Total Number of Cases | Number of Positive Cases | Proportion of Positive Cases (%) |
|---|---|---|---|
| Lung Cancer Tissue | 160 | 132 | 82.5 |
| Lung Cancer Para-carcinoma Tissue | 32 | 2 | 6.25 |
| Normal Lung Tissue | 3 | 0 | 0 |

By using Hetumomab as the primary antibody, the expression of the target antigen of Hetumomab was detected in human gastric cancer tissues of 110 cases, para-carcinoma tissues of 20 cases, and normal gastric tissues of 3 cases. The experimental results (Table 3) showed that the target antigen of Hetumomab was specifically expressed in 93.64% (103/110) of human gastric cancer tissues, but not expressed in the para-carcinoma tissues and normal gastric tissues, which indicates that the target antigen of Hetumomab is specifically highly expressed in human gastric cancer tissues.

**Table 3. Detection of target antigens of Monoclonal Antibody Hetumomab in Human Gastric Tissues by Immunohistochemistry**

| Type of Tissue | Total Number of Cases | Number of Positive Cases | Proportion of Positive Cases (%) |
|---|---|---|---|
| Gastric Cancer Tissue | 110 | 103 | 93.64 |
| Gastric Cancer Para-carcinoma Tissue | 20 | 0 | 0 |
| Normal Gastric Tissue | 3 | 0 | 0 |

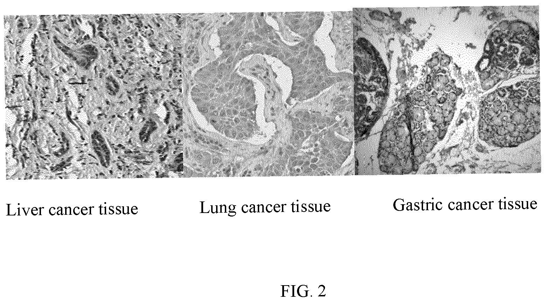

Photos of some typical positive results of the above immunohistochemistry results are shown in FIG. 2.

The above results indicate that the target antigen of Hetumomab has specific high expression in various cancer tissues, such as liver cancer, lung cancer, and gastric cancer, of most cancer patients.

### Example 3 Recognition of Tumor Stem Cells by Monoclonal Antibody Hetumomab

### 1. The cancer cells recognized by Hetumomab are enriched in the sphere-forming culture of the cancer cells.

According to a large number of literature reports, a serum-free suspension culture, i.e., a sphere (sphere-forming) culture, can enrich tumor stem cells (Reynolds, B.A. and S. Weiss, "Clonal and population analyses demonstrate that an EGF-responsive mammalian embryonic CNS precursor is a stem cell." Dev Biol, 1996. 175(1): p. 1-13; Fang, N., et al., "pH responsive adhesion of phospholipid vesicle on poly(acrylic acid) cushion grafted to poly(ethylene terephthalate) surface. "Colloids Surf B Biointerfaces, 2005. 42(3-4): p. 245-52.; and Tirino, V, et al., "The role of CD133 in the identification and characterisation of tumour-initiating cells in non-small-cell lung cancer. "Eur J Cardiothorac Surg, 2009. 36(3): p. 446-53.). Therefore, whether the cancer cells recognized by Hetumomab are enriched in the sphere culture may be used to determine whether the cells recognized by Hetumomab are related to tumor stem cells.

The human liver cancer cell line Bel7402-V13 and MHCC97L cells were cultured for 5 days in a serum-free manner, and the Hetumomab⁺ cells in the parental cells and in the cells from sphere culture were detected by viable cell flow fluorescence cytometry. The experimental results (Table 4) showed that the ratio of Hetumomab⁺ cells in Bel7402-V13 sphere cells was 8.92%, which was 3.96 times enriched relative to the ratio of 2.25% in the parental cells; and the ratio of Hetumomab⁺ cells in MHCC97L sphere cells was 7.51%, which was 2.18 times enriched relative to the ratio of 3.45% in the parental cells. That is, after serum-free culture, Hetumomab⁺ cells of the liver cancer cell line were enriched.

**Table 4. Detection of Enrichment of Liver Cancer cells Recognized by Monoclonal Antibody Hetumomab in Sphere Culture of Liver Cancer Cells by Flow Immunofluorescence Techniques**

| Cell Line | Parental Cells | Cells from Sphere Culture | Fold Enrichment |
|---|---|---|---|
| Bel7402-V13 | 2.25% | 8.92% | 3.96 |
| MHCC97L | 3.45% | 7.51% | 2.18 |

The human lung cancer cell line SPCA-1 and A549 cells were cultured for 5 days in a serum-free manner, and the Hetumomab⁺ cells in the parental cells and in the cells from sphere culture were detected by viable cell flow fluorescence cytometry. The experimental results (Table 5) showed that the ratio of Hetumomab⁺ cells in SPCA-1 sphere cells was 7.34%, which was 4.22 times enriched relative to the ratio of 1.74% in the parental cells; and the ratio of Hetumomab⁺ cells in A549 sphere cells was 13.30%, which was 1.84 times enriched relative to the ratio of 7.23% in the parental cells. That is, after the serum-free culture, Hetumomab⁺ cells of the lung cancer cell line were enriched.

**Table 5. Detection of Enrichment of Lung Cancer Cells Recognized by Monoclonal Antibody Hetumomab in Sphere Culture of Lung Cancer Cells by Flow Immunofluorescence Techniques**

| Cell Line | Parental Cells | Cells from Sphere Culture | Fold Enrichment |
|---|---|---|---|
| SPCA-1 | 1.74% | 7.34% | 4.22 |
| A549 | 7.23% | 13.30% | 1.84 |

The human gastric cancer cell line SNU-5 and BGC-823 cells were cultured for 5 days in a serum-free manner, and the Hetumomab⁺ cells in the parental cells and in the cells from sphere culture were detected by viable cell flow fluorescence cytometry. The experimental results (Table 6) showed that the ratio of Hetumomab⁺ cells in SNU-5 sphere cells was 7.19%, which was 2.13 times enriched relative to the ratio of 3.38% in the parental cells; and the ratio of Hetumomab⁺ cells in BGC-823 sphere cells was 7.45%, which was 2.53 times enriched relative to the ratio of 2.95% in the parental cells. That is, after the serum-free culture, Hetumomab⁺ cells of the gastric cancer cell line were enriched.

**Table 6. Detection of Enrichment of Gastric Cancer Cells Recognized by Monoclonal Antibody Hetumomab in Sphere Culture of Gastric Cancer Cells by Flow Immunofluorescence Techniques**

| Cell Line | Parental Cells | Cells from Sphere Culture | Fold Enrichment |
|---|---|---|---|
| SNU-5 | 3.38% | 7.19% | 2.13 |
| BGC-823 | 2.95% | 7.45% | 2.53 |

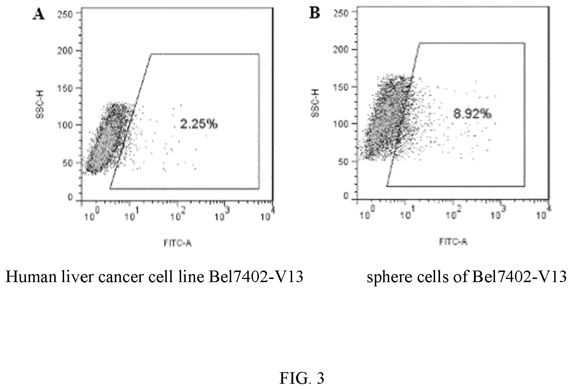

Some typical figures of the above flow immunofluorescence results are shown in FIG. 3.

These results indicate that cancer cells recognized by Hetumomab are significantly enriched in the sphere cultuere of a variety of human tumor cell lines, such as liver cancer, lung cancer, and gastric cancer cell lines.

### 2. Hetumomab recognizes ESA- and CD90-positive cancer stem cells.

At present, many articles (Yamashita T, et al. EpCAMpositive hepatocellular carcinoma cells are tumor-initiating cells with stem/progenitor cell features. Gastroenterology. 2009,136(3): 1012-1024.; and Yang ZF. Identification of local and circulating cancer stem cells in human liver cancer. Hepatology. 2008,47(3):919-928.) have demonstrated that ESA and CD90 are the surface markers of tumor stem cells of some liver cancer cells. In order to detect liver cancer stem cells in human liver cancer cell line Bel7402-V13 cells reconginized by monoclonal antibody Hetumomab, which are also ESA and CD90 marker positive, we used two-color flow fluorimetry to stain the human liver cancer cell line Bel7402-V13 cells after culture for 5 days in a serum-free medium.

The results (Table 7) showed that the ratio of cells recognized by Hetumomab was 8.52%, and the expression ratio of ESA⁺ stem cells was 9.02%, and the co-staining ratio of the two was 3.63%. That is, Hetumomab recognized 40.2% of ESA⁺ stem cells. Human liver cancer MHCC97L cells cultured for 5 days in a serum-free medium were stained. The results (Table 7) showed that the ratio of cells recognized by Hetumomab was 2.38%, and the expression ratio of CD90⁺ stem cells was 4.54%, and the co-staining ratio of the two was 2.01%. That is, Hetumomab recognized 44.3% of CD90⁺ stem cells.

**Table 7. Co-staining of Monoclonal Antibody Hetumomab with Liver Cancer Stem Cell Surface Markers ESA or CD90 in Liver Cancer Cells by Two-color Flow Fluorimetry**

| Cell Line | Hetumomab⁺ | Liver Cancer Stem Cell Surface Marker⁺ | Co-staining |
|---|---|---|---|
| Bel7402-V13 | 8.52% | 9.02%(ESA) | 3.63% |
| MHCC97L | 2.38% | 4.54%(CD90) | 2.01% |

These results indicate that Hetumomab recognizes human tumor stem cells positive for markers such as ESA or CD90.

### 3. Cancer cells recognized by monoclonal antibody Hetumomab have stronger self-renewal ability.

The self-renewal ability, strong invasive ability, ability to tolerate chemotherapeutic drugs and strong tumorigenicity are important basic features of tumor stem cells distinguishing from ordinary tumor cells. Therefore, in order to further verify whether the cells recognized by Hetumomab have the characteristics of tumor stem cells, Hetumomab⁺ cells in various human tumor cells were sorted to detect self-renewal ability, invasion ability, drug resistance and in vivo tumorigenicity.

The main manifestation of the self-renewal ability of tumor stem cells is the ability to form sphere cells in a serum-free medium, which is called asymmetric division, that is, when one cell divides into two daughter cells, one of the daughter cells maintains the same features as the parental cell, while the other daughter cell may continue to divide, forming normal daughter cells. Therefore, self-renewal ability may be determined by detecting the ability of Hetumomab⁺ cells to form spheres in a serum-free medium.

Hetumomab⁺ cells, parental cells and Hetumomab⁻ cells were isolated from human liver cancer Bel7402-V13 sphere cells cultured for 5 days by flow sorting. The sorted cells were seeded at 500 cells/well in semi-solid sphere medium containing 0.8% methylcellulose (DMEM/F12 (1:1) culture solution containing 0.8% methylcellulose, 20 ng/mLEGF, 20 ng/mL bFGF, B27 added at a ratio of 1:50, 10ng/mL LIF, 2 mmol/mL glutamine, 1 u/mL Heparin), and cultured in a ultra-low adhesion 24-well plate, to observe the number of spheres formed from each group of cells. The results (Table 8) showed that, the number of spheres formed from Hetumomab+ cells, parental cells and Hetumomab⁻ cells in serum-free medium was 262±8.5, 168±5.6, and 98±5.6, respectively, that is, the sphere-forming rate of Hetumomab+ cells was significantly higher than the other two cells (*p* < 0.05). Therefore, Hetumomab⁺ cells have stronger self-renewal ability than the parental cells and Hetumomab⁻ cells.

**Table 8 Comparison of self-renewal ability of Hetumomab⁺ cells, parental cells and Hetumomab⁻ cells in liver cancer cells**

| Cell line: Bel7402-V13 | Hetumomab ⁺ Cells | Parental Cells | Hetumomab ⁻ Cells |
|---|---|---|---|
| Number of spheres formed per 500 cells | 262±8.5 | 168±5.6 | 98±5.6 |

Hetumomab⁺ cells, parental cells and Hetumomab⁻ cells were isolated from human lung cancer SPCA-1 sphere cells by flow sorting. The sorted cells were seeded at 500 cells/well in semi-solid sphere medium containing 0.8% methylcellulose, and cultured in an ultra-low adhesion 24-well plate to observe the number of spheres formed from each group of cells. The results (Table 9) showed that, the number of spheres formed from Hetumomab+ cells, parental cells and Hetumomab⁻ cells in serum-free medium was 165.7±6.0, 127±5.6, and 83.7±4.7, respectively, that is, the sphere-forming rate of Hetumomab+ cells was significantly higher than the other two cells (*p* < 0.05). Therefore, Hetumomab⁺ cells have stronger self-renewal ability than the parental cells and Hetumomab⁻ cells.

**Table 9 Comparison of self-renewal ability of Hetumomab⁺ cells, parental cells and Hetumomab⁻ cells in lung cancer cells**

| Cell line: SPCA-1 | Hetumomab ⁺ cells | Parental Cells | Hetumomab ⁻ cells |
|---|---|---|---|
| Number of spheres formed per 500 cells | 165.7±6.0 | 127±5.6 | 83.7±4.7 |

Hetumomab⁺ cells, parental cells and Hetumomab⁻ cells were isolated from human gastric cancer SNU-5 sphere cells by flow sorting. The sorted cells were seeded at 500 cells/well in semi-solid sphere medium containing 0.8% methylcellulose, and cultured in an ultra-low adhesion 24-well plate to observe the number of spheres formed from each group of cells. The results (Table 10) showed that, the number of spheres formed from Hetumomab+ cells, parental cells and Hetumomab⁻ cells in serum-free medium was 24±1.4, 15.5±0.7, and 11.5±0.7, respectively, that is, the sphere-forming rate of Hetumomab+ cells was significantly higher than the other two cells (*p* < 0.05). Therefore, Hetumomab⁺ cells have stronger self-renewal ability than the parental cells and Hetumomab⁻ cells.

**Table 10 Comparison of self-renewal ability of Hetumomab⁺ cells, parental cells and Hetumomab⁻ cells in gastric cancer cells**

| Cell line: SNU-5 | Hetumomab ⁺ cells | Parental Cells | Hetumomab ⁻ cells |
|---|---|---|---|
| Number of spheres formed per 500 cells | 60.7±4.2 | 27.0±2.6 | 18.0±2.0 |

These results indicate that a variety of cancer cells (liver cancer, lung cancer, gastric cancer) recognized by Hetumomab have stronger self-renewal ability, that is, having one of the main characteristics of tumor stem cells: high self-renewal ability.

### 4. Cancer cells recognized by monoclonal antibody Hetumomab have stronger invasion ability.

The self-renewal ability, strong invasive ability, ability to tolerate chemotherapeutic drugs and strong tumorigenicity are important basic features of tumor stem cells distinguishing from ordinary tumor cells. Therefore, in order to further verify whether the cells recognized by Hetumomab have the characteristics of tumor stem cells, Hetumomab⁺ cells in various human tumor cells were sorted to detect self-renewal ability, invasion ability, drug resistance and in vivo tumorigenicity.

Hetumomab⁺ cells, parental cells and Hetumomab⁻ cells were isolated from human liver cancer Bel7402-V13 sphere cells cultured for 5 days by flow sorting. The sorted cells were seeded in the same amount in a Transwell chamber previously coated with Matrigel gel, and fixed after 24 hours, to observe the number of cells invading through the membrane under a microscope. The results (Table 11) showed that, the number of cells invading through the membrane in Hetumomab+cells, parental cells and Hetumomab⁻ cells was 308±9.5, 210±10.7 and 132±14.7 per field of vision, respectively, that is, the number of Hetumomab+ cells invading through the membrane was significantly higher than the other two cells (*p* < 0.05). Therefore, Hetumomab⁺ cells have stronger invasion ability than the parental cells and Hetumomab⁻ cells.

**Table 11 Comparison of invasion ability of Hetumomab⁺ cells, parental cells and Hetumomab⁻ cells in liver cancer cells**

| Cell line: Bel7402-V13 | Hetumomab⁺ cells | Parental Cells | Hetumomab⁻ cells |
|---|---|---|---|
| Invasive Cell | 308±9.5 | 210±10.7 | 132±14.7 |

Hetumomab⁺ cells, parental cells and Hetumomab⁻ cells were isolated from the cultured human lung cancer SPCA-1 sphere cells by flow sorting. The sorted cells were seeded in the same amount in a Transwell chamber previously coated with Matrigel gel, and fixed after 24 hours, to observe the number of cells invading through the membrane under a microscope. The results (Table 12) showed that, the number of cells invading through the membrane in the Hetumomab+ cells, parental cells and Hetumomab⁻ cells was 222±11.5, 193.7±5.7 and 154.3±12.1 per field of vision, respectively, that is, the number of Hetumomab+ cells invading through the membrane was significantly higher than the other two cells (*p* < 0.05). Therefore, Hetumomab⁺ cells have stronger invasion ability than the parental cells and Hetumomab⁻ cells.

**Table 12 Comparison of invasion ability of Hetumomab⁺ cells, parental cells and Hetumomab⁻ cells in lung cancer cells**

| Cell line: SPCA-1 | Hetumomab⁺ cells | Parental Cells | Hetumomab⁻ cells |
|---|---|---|---|
| Invasive Cell | 222±11.5 | 193.7±5.7 | 154.3±12.1 |

Hetumomab⁺ cells, parental cells and Hetumomab⁻ cells were isolated from the cultured human gastric cancer SNU-5 sphere cells by flow sorting. The sorted cells were seeded in the same amount in a Transwell chamber previously coated with Matrigel gel, and fixed after 24 hours, to observe the number of cells invading through the membrane under a microscope. The results (Table 13) showed that, the number of cells invading through the membrane in the Hetumomab+ cells, parental cells and Hetumomab⁻ cells was 247.5±19.1, 142.5±9.2 and 145.0±11.3 per field of vision, respectively, that is, the number of Hetumomab+ cells invading through the membrane was significantly higher than the other two cells (*p* < 0.05). Therefore, Hetumomab⁺ cells have stronger invasion ability than the parental cells and Hetumomab⁻ cells.

**Table 13 Comparison of invasion ability of Hetumomab⁺ cells, parental cells and Hetumomab⁻ cells in gastric cancer cells**

| Cell line: SNU-5 | Hetumomab ⁺ cells | Parental Cells | Hetumomab⁻ cells |
|---|---|---|---|
| Invasive Cell | 214.0±12.5 | 156.7±7.2 | 48.0±7.5 |

These results indicate that a variety of cancer cells (liver cancer, lung cancer, gastric cancer) recognized by Hetumomab have stronger invasion ability, that is, having one of the main characteristics of tumor stem cells: high invasion ability.

### 5. Cancer cells recognized by monoclonal antibody Hetumomab have stronger ability of tolerating chemotherapeutic drugs.

The self-renewal ability, strong invasive ability, ability to tolerate chemotherapeutic drugs and strong tumorigenicity are important basic features of tumor stem cells distinguishing from ordinary tumor cells. Therefore, in order to further verify whether the cells recognized by Hetumomab have the characteristics of tumor stem cells, Hetumomab⁺ cells in various human tumor cells were sorted to detect self-renewal ability, invasion ability, drug resistance and in vivo tumorigenicity.

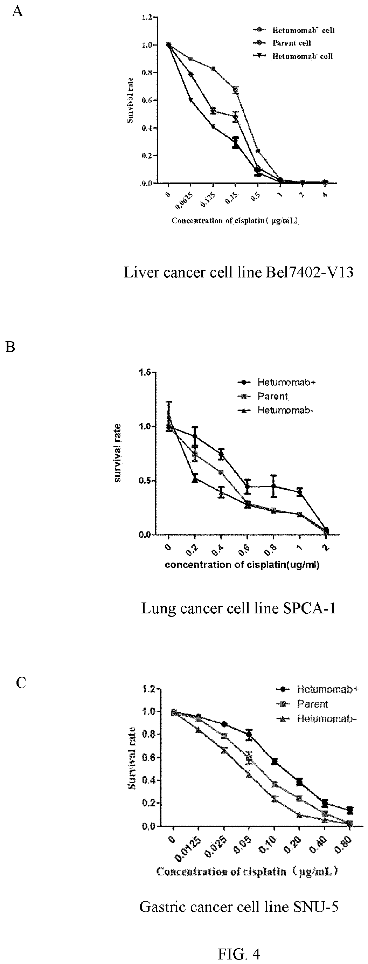

In order to detect the drug resistance of Hetumomab⁺ liver cancer cells, Hetumomab⁺ cells, parental cells and Hetumomab⁻ cells obtained by flow sorting sphere cells of human liver cancer cells Bel7402-V13 were seeded at 5000 cells/well in a 96-well plate. Each group of cells was cultured in a complete medium containing 8 different concentrations, i.e., 0µg/mL, 0.0625ug/mL, 0.125µg/mL, 0.25µg/mL, 0.5µg/mL, 1µg/mL, 2µg/mL, and 4 µg/mL, of cisplatin. The medium was changed once every 3 days, and after 7 days, the OD value was measured by the CCK8 method to determine the IC50 reflecting drug resistance. The experimental results (Table 14, FIG. 4) showed that the IC50 of Hetumomab⁺ cells, parental cells and Hetumomab⁻ cells were 0.739 µg/mL, 0.502 µg/mL and 0.313 µg/mL, respectively, that is, the drug resistance of Hetumomab⁺ cells was significantly higher than that of the parents cells and Hetumomab⁻ cells, and the difference was statistically significant (*p*<0.05). Therefore, Hetumomab⁺ liver cancer cells have stronger chemotherapeutic drug resistance than the parental cells and Hetumomab⁻ cells.

**Table 14 Comparison of chemotherapeutic drug resistance of Hetumomab⁺ cells, parental cells and Hetumomab⁻ cells in liver cancer cells**

| Cell line: Bel7402-V13 | IC50 (µg/mL) |
|---|---|
| Hetumomab⁺ cells | 0.739 |
| Parental Cells | 0.502 |
| Hetumomab⁻ cells | 0.313 |

In order to detect the drug resistance of Hetumomab⁺ lung cancer cells, Hetumomab⁺ cells, parental cells and Hetumomab⁻ cells obtained by flow sorting sphere cells of human lung cancer cells SPCA-1 were seeded at 5000 cells/well in a 96-well plate. Each group of cells was cultured in a complete medium containing 7 different concentrations, i.e., 0 µg/mL, 0.2 µg/mL, 0.4 µg/mL, 0.6 µg/mL, 0.8 µg/mL, 1 µg/mL, and 2 µg/mL, of cisplatin, and then the OD value was measured by the CCK8 method to determine the IC50 reflecting drug resistance. The experimental results (Table 15, FIG. 4) showed that the IC50 of Hetumomab⁺ cells, parental cells and Hetumomab⁻ cells were 0.707 µg/mL, 0.513 µg/mL and 0.180 µg/mL, respectively, that is, the drug resistance of Hetumomab⁺ cells was significantly higher than that of the parents cells and Hetumomab⁻ cells, and the difference was statistically significant (*p*<0.05). Therefore, Hetumomab⁺ lung cancer cells have stronger chemotherapeutic drug resistance than the parental cells and Hetumomab⁻ cells.

**Table 15 Comparison of chemotherapeutic drug resistance of Hetumomab⁺ cells, parental cells and Hetumomab⁻ cells in lung cancer cells**

| Cell line: SPCA-1 | IC50 (µg/mL) |
|---|---|
| Hetumomab⁺ cells | 0.707 |
| Parental Cells | 0.513 |
| Hetumomab⁻ cells | 0.180 |

In order to detect the drug resistance of Hetumomab⁺ gastric cancer cells, Hetumomab⁺ cells, parental cells and Hetumomab⁻ cells obtained by flow sorting sphere cells of human gastric cancer cells SNU-5 were seeded at 5000 cells/well in a 96-well plate. Each group of cells was cultured in a complete medium containing 8 different concentrations, i.e., 0 µg/mL, 0.0125 µg/mL, 0.025 µg/mL, 0.05 µg/mL, 0.1 µg/mL, 0.2 µg/mL, 0.4 µg/m, and 0.8 µg/mL, of cisplatin, and then the OD value was measured by the CCK8 method to determine the IC50 reflecting drug resistance. The experimental results (Table 16, FIG. 4) showed that the IC50 of Hetumomab⁺ cells, parental cells and Hetumomab⁻ cells were 0.285 µmol/L, 0.155 µmol/L and 0.094 µmo|/L, respectively, that is, the drug resistance of Hetumomab⁺ cells was significantly higher than that of the parents cells and Hetumomab⁻ cells, and the difference was statistically significant (*p*<0.05). Therefore, Hetumomab⁺ gastric cancer cells have stronger chemotherapeutic drug resistance than the parental cells and Hetumomab⁻ cells.

**Table 16 Comparison of chemotherapeutic drug resistance of Hetumomab⁺ cells, parental cells and Hetumomab⁻ cells in gastric cancer cells**

| Cell line: SNU-5 | IC50 (µmol/L) |
|---|---|
| Hetumomab⁺ cells | 0.294 |
| Parental Cells | 0.151 |
| Hetumomab⁻ cells | 0.092 |

These results indicate that a variety of cancer cells (liver cancer, lung cancer, gastric cancer) recognized by Hetumomab have stronger chemotherapeutic drug resistance, that is, having one of the main characteristics of tumor stem cells: high drug resistance.

### 6. Cancer cells recognized by monoclonal antibody Hetumomab have stronger in vivo tumorigenicity.

The self-renewal ability, strong invasive ability, ability to tolerate chemotherapeutic drugs and strong tumorigenicity are important basic features of tumor stem cells distinguishing from ordinary tumor cells. Therefore, in order to further verify whether the cells recognized by Hetumomab have the characteristics of tumor stem cells, Hetumomab⁺ cells in various human tumor cells were sorted to detect self-renewal ability, invasion ability, drug resistance and in vivo tumorigenicity.

The "gold standard" for testing whether a cell is a cancer stem cell is its strong in vivo tumorigenicity. Therefore, Hetumomab⁺ cells, parental cells and Hetumomab⁻ cells obtained by flow sorting sphere cells of human liver cancer cells Bel7402-V13 were inoculated subcutaneously into 4-weeks-old nude mice, respectively, to observe the in vivo tumorigenicity for a long time. As a result, as shown in Table 17, 1×10⁴ Hetumomab⁺ cells resulted in tumorigenesis in mice at 3 weeks of inoculation, whereas 1×10⁵ parental cells were required to result in tumorigenesis after 3 weeks of inoculation, while the Hetumomab⁻ cells were not able to cause tumorigenesis during the whole observation period. The results of this classical experiment indicate that the in vivo tumorigenicity of Hetumomab⁺ cells is significantly stronger than that of the parental cells and Hetumomab⁻ cells. It is suggested that Hetumomab⁺ cells have the characteristics of tumor stem cells, high tumorigenicity, which is consistent with the "gold standard" for identifying tumor stem cells. Therefore, the cells recognized by Hetumomab are tumor stem cells of liver cancer.

**Table 17 Comparison of in vivo tumorigenicity of Hetumomab⁺ cells, parental cells and Hetumomab⁻ cells in liver cancer cells Bel7402-V13 (number of animals carrying tumor)**

| Tumorigenicity Grouping | Number of cells injected | Time after injection (week) | | | | |
|---|---|---|---|---|---|---|
| | | 1 | 2 | 3 | 4 | 5 |
| Hetumomab ⁺ cells | 2×10³ | 0/6 | 0/6 | 0/6 | 0/6 | 0/6 |
| | 1×10⁴ | 0/6 | 0/6 | 1/6 | 2/6 | 2/6 |
| | 2×10⁴ | 0/6 | 1/6 | 2/6 | 3/6 | 3/6 |
| Parental Cells | 2×10⁴ | 0/6 | 0/6 | 0/6 | 0/6 | 0/6 |
| | 6×10⁴ | 0/6 | 0/6 | 0/6 | 1/6 | 1/6 |
| | 1×10⁵ | 0/6 | 0/6 | 1/6 | 1/6 | 1/6 |
| Hetumomab⁻ cells | 2×10⁴ | 0/6 | 0/6 | 0/6 | 0/6 | 0/6 |
| | 6×10⁴ | 0/6 | 0/6 | 0/6 | 0/6 | 0/6 |
| | 1×10⁵ | 0/6 | 0/6 | 0/6 | 0/6 | 0/6 |

Hetumomab⁺ cells, parental cells and Hetumomab⁻ cells obtained by flow sorting sphere cells of human gastric cells SNU-5 were inoculated subcutaneously into 4-weeks-old nude mice, respectively, to observe the in vivo tumorigenicity for a long time. As a result, as shown in Table 18, 2×10³ Hetumomab⁺ cells resulted in in vivo tumorigenesis in half of the mice at 3 months of inoculation, whereas 2×10³ parental cells failed to initiate tumorigenesis after 4 months of inoculation, while the Hetumomab⁻ cells were not able to cause tumorigenesis during the whole observation period. The results of this classical experiment indicate that the in vivo tumorigenicity of Hetumomab⁺ cells is significantly stronger than that of the parental cells and Hetumomab⁻ cells. It is suggested that Hetumomab⁺ cells have the characteristics of tumor stem cells, high tumorigenicity, which is consistent with the "gold standard" for identifying tumor stem cells. Therefore, the cells recognized by Hetumomab are tumor stem cells of gastric cancer.

**Table 18 Comparison of in vivo tumorigenicity of Hetumomab⁺ cells, parental cells and Hetumomab⁻ cells in gastric cells SNU-5 (number of animals carrying tumor)**

| Tumorigenicity Grouping | Number of cells injected | Time after injection (month) | | | |
|---|---|---|---|---|---|
| | | 1 | 2 | 3 | 4 |
| Hetumomab⁺ cells | 2×10⁴ | 0/6 | 4/6 | 6/6 | 6/6 |
| | 2×10³ | 0/6 | 2/6 | 3/6 | 3/6 |
| | 2×10² | 0/6 | 1/6 | 1/6 | 1/6 |
| Hetumomab⁻ cells | 2×10⁴ | 0/6 | 0/6 | 0/6 | 0/6 |
| | 2×10³ | 0/6 | 0/6 | 0/6 | 0/6 |
| | 2×10² | 0/6 | 0/6 | 0/6 | 0/6 |
| Parental Cells | 2×10⁴ | 0/6 | 1/6 | 2/6 | 2/6 |
| | 2×10³ | 0/6 | 0/6 | 0/6 | 0/6 |
| | 2×10² | 0/6 | 0/6 | 0/6 | 0/6 |

These results indicate that a variety of cancer cells (liver cancer, lung cancer, gastric cancer) recognized by Hetumomab have stronger in vivo tumorigenicity, that is, having one of the main characteristics of tumor stem cells: high in vivo tumorigenicity.

### Example 4 Monoclonal Antibody Hetumomab can inhibit the self-renewal, invasion and drug resistance of tumor stem cells

### 1. Hetumomab significantly inhibits the self-renewal ability (one of the main characteristics of tumor stem cells) of the tumor stem cells in various tumors (liver cancer, lung cancer, and gastric cancer).

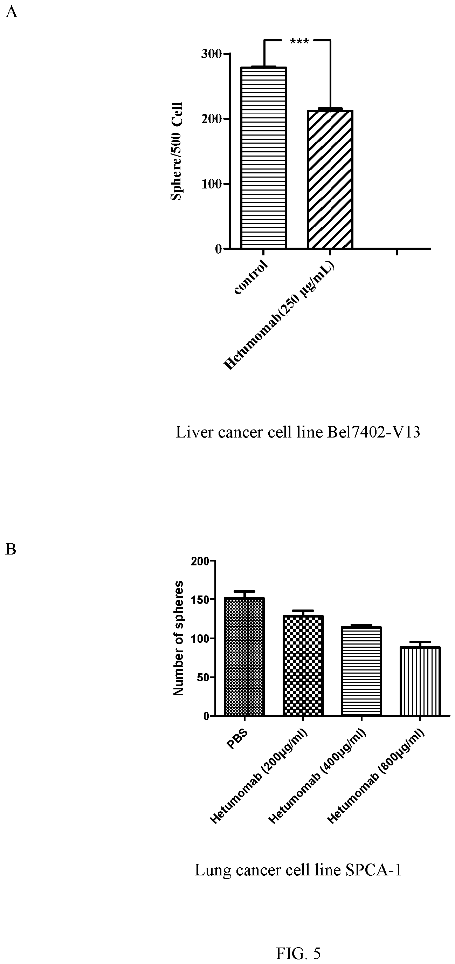

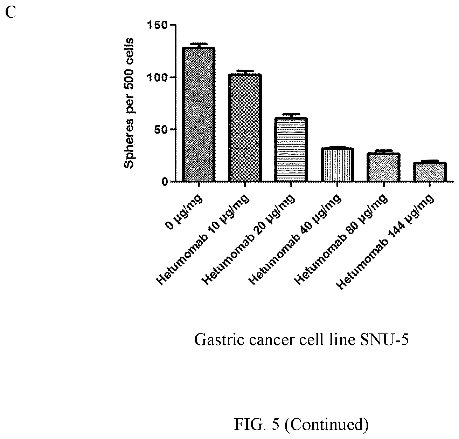

The main manifestation of the self-renewal ability of tumor stem cells is the ability to form spheres in serum-free medium, which is called asymmetric division, that is, when one cell divides into two daughter cells, one of the daughter cells maintains the same features as the parental cell, while the other daughter cell may continue to divide, forming normal daughter cells. In order to prove whether Hetumomab is a functional monoclonal antibody capable of directly inhibiting liver cancer stem cells, a single-cell suspension was prepared from spheres of the human liver cancer cell line Bel7402-V13 obtained by culturing in a serum-free medium for 5 days. And then Hetumomab (250 µg/mL) was used as the experimental group, and the PBS was used as a negative control group to incubate with the cells at 37°C for 2 h, during which the cells were mixed with the antibody or the negative control once every half an hour. 500 cells in each group were inoculated into a semi-solid sphere medium (containing EGF, LIF, bFGF, etc.) containing 0.8% methylcellulose, and cultured in an ultra-low adhesion 24-well plate. 1-1.5 mL liquid medium was added every other day, and the number of spheres formed from the two groups was observed after 14 days. The experimental results (FIG. 5) showed that the number of spheres formed in the experimental group was 212±2.8, while the number of spheres formed in the negative control group was 278.5±0.7, which was significantly higher than that in the experimental group. The inhibition ratio of Hetumomab to the sphere-formation of Bel7402-V13 cells was 23.9%, p<0.05, with statistical difference. The results showed that Hetumomab can directly act on liver cancer stem cells and inhibit their self-renewal ability.

In order to prove whether Hetumomab is a functional monoclonal antibody capable of directly inhibiting lung cancer stem cells, the same method was used to detect the inhibitory effect of Hetumomab on the sphere-formation of human lung cancer cell line SPCA-1. The results (as shown in Table 19 and FIG. 5) showed that number of formed spheres from the highest concentration of antibody in the experimental group was 88.3±7.2, while that from the negative control group was 151.3 ± 9.1, which was significantly higher than the experimental group, and inhibition ratio of Hetumomab to the sphere-formation of SPCA-1 cells was 41.6%, p < 0.01, with statistical difference. The results showed that Hetumomab can directly act on lung cancer stem cells and inhibit their self-renewal ability.

**Table 19 Detection of inhibitory effect of Hetumomab on the sphere-formation of human lung cancer cell line SPCA-1**

| | | | | |
|---|---|---|---|---|
| Concentration of antibody | 0 µg/ml | 200 µg/ml | 400 µg/ml | 800 µg/ml |
| Number of formed spheres | 151.3±9.1 | 128.3±7.1 | 113.7±3.8 | 88.3±7.2 |

In order to prove whether Hetumomab is a functional monoclonal antibody capable of directly inhibiting gastric cancer stem cells, the same method was used to detect the inhibitory effect of Hetumomab on the sphere-formation of tumor stem cell subgroup of CD44 positive cells of human gastric cancer cell line SNU-5. The results (as shown in Table 20 and FIG. 5) showed that number of formed spheres from the highest concentration of antibody in the experimental group was 18±2.0, while that from the negative control group was 128 ± 4.0, which was significantly higher than the experimental group, and inhibition ratio of Hetumomab to the sphere-formation of SNU-5 CD44⁺ cells was 85.9%, *p* < 0.01, with statistical difference. The results showed that Hetumomab can directly act on gastric cancer stem cells and inhibit their self-renewal ability.

**Table 20 Detection of inhibitory effect of Hetumomab on the sphere-formation of CD44⁺ cells of human gastric cancer cell line SNU-5**

| | | | | | | |
|---|---|---|---|---|---|---|
| Concentration of antibody | 0 µg/ml | 10 µg/ml | 20 µg/ml | 40 µg/ml | 80 µg/ml | 144 µg/ml |
| Number of formed spheres | 128±4.0 | 103±3.5 | 60±4.2 | 32±1.5 | 27±2.4 | 18±2.0 |

These results indicate that Hetumomab can directly inhibit the self-renewal ability of various cancer cells (liver cancer, lung cancer, gastric cancer), and that Hetumomab can not only recognize the target tumor stem cells, but also is a functional (therapeutic) monoclonal antibody capable of directly inhibiting tumor stem cells.

### 2. Hetumomab significantly inhibits the invasion ability (the second main characteristics of tumor stem cells) of the tumor stem cells in various tumors (liver cancer, lung cancer, and gastric cancer).

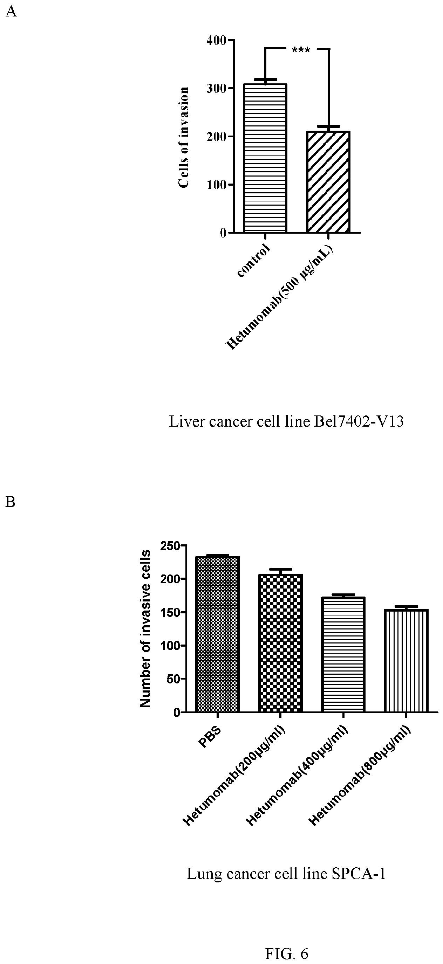

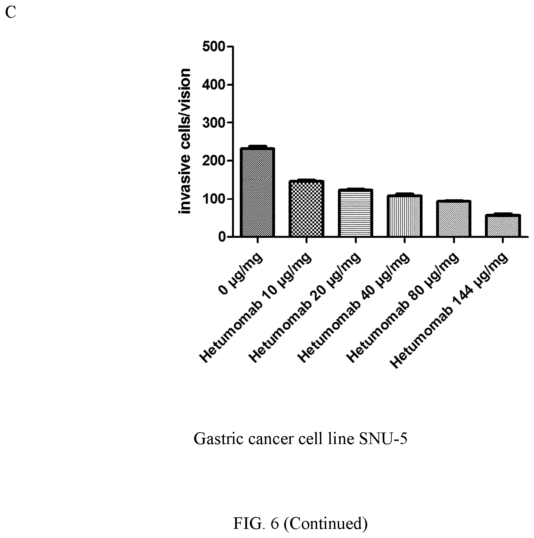

High invasiveness is another important biological feature of cancer stem cells. In order to prove whether Hetumomab is a functional monoclonal antibody capable of directly inhibiting liver cancer stem cells, Transwell invasion experiment was used to analyze the ability of monoclonal antibody Hetumomab to inhibit the invasion of liver cancer cells. The experimental results (FIG. 6) showed that the number of invasive cells in the PBS negative control group was (301.0±16.3)/field of vision, and the number of invasive cells in Hetumomab (0.5 mg/ml) group was (148±16.4)/field of vision. The results showed that the invasion ability of the cells was significantly weakened by the direct action of Hetumomab, and the inhibition rate of Hetumomab to the invasoin of Bel7402-V13 sphere cells was 50.8%, *p*<0.05, with statistical difference. The results showed that Hetumomab can directly act on liver cancer stem cells and inhibit their invasion ability.

In order to prove whether Hetumomab is a functional monoclonal antibody capable of directly inhibiting lung cancer stem cells, the same method was used to detect the inhibitory effect of Hetumomab on the invasion of human lung cancer cell line SPCA-1. The experimental results (Table 21, FIG. 6) showed that the number of invasive cells in the PBS negative control group was (232.3±3.1)/field of vision, and the number of invasive cells in Hetumomab group was (153.0±6.1)/field of vision. The results showed that the invasion ability of the cells was significantly weakened by the direct action of Hetumomab, and the inhibition rate of Hetumomab to the invasoin of SPCA-1 sphere cells was 34.1%, *p*<0.05, with statistical difference. The results showed that Hetumomab can directly act on lung cancer stem cells and inhibit their invasion ability.

**Table 21 Detection of inhibitory effect of Hetumomab on the invasion of human lung cancer cell line SPCA-1**

| | | | | |
|---|---|---|---|---|
| Concentration of antibody | 0 µg/ml | 200 µg/ml | 400 µg/ml | 800 µg/ml |
| Cells/Field of Vision | 232.3±3.1 | 205.7±8.5 | 171.3±4.9 | 153.0±6.1 |

In order to prove whether Hetumomab is a functional monoclonal antibody capable of directly inhibiting gastric cancer stem cells, the same method was used to detect the inhibitory effect of Hetumomab on the invasion of tumor stem cell subgroup of CD44 positive cells of human gastric cancer cell line SNU-5. The experimental results (Table 22, FIG. 6) showed that the number of invasive cells in the PBS negative control group was (231±7.0)/field of vision, and the number of invasive cells in Hetumomab group was (56±4.0)/field of vision. The results showed that the invasion ability of the cells was significantly weakened by the direct action of Hetumomab, and the inhibition rate of Hetumomab to the invasoin of SNU-5 CD44⁺ cells was 75.8%, *p*<0.05, with statistical difference. The results showed that Hetumomab can directly act on gastric cancer stem cells and inhibit their invasion ability.

**Table 21 Detection of inhibitory effect of Hetumomab on the invasion of CD44⁺ cells of human gastric cancer cell line SNU-5**

| | | | | | | |
|---|---|---|---|---|---|---|
| Concentration of antibody | 0 µg/ml | 10 µg/ml | 20 µg/ml | 40 µg/ml | 80 µg/ml | 144 µg/ml |
| Cells/Field of Vision | 231±7.0 | 146±3.5 | 124±3.0 | 108±5.0 | 93±2.5 | 56±4.0 |

These results indicate that Hetumomab can directly inhibit the invasion ability of various cancer cells (liver cancer, lung cancer, gastric cancer), and that Hetumomab can not only recognize the target tumor stem cells, but also is a functional (therapeutic) monoclonal antibody capable of directly inhibiting the tumor stem cells.

### 3. Hetumomab significantly inhibits the chemotherapeutic drug resistance (the third main characteristics of tumor stem cells) of the tumor stem cells in various tumors (liver cancer, lung cancer, and gastric cancer).

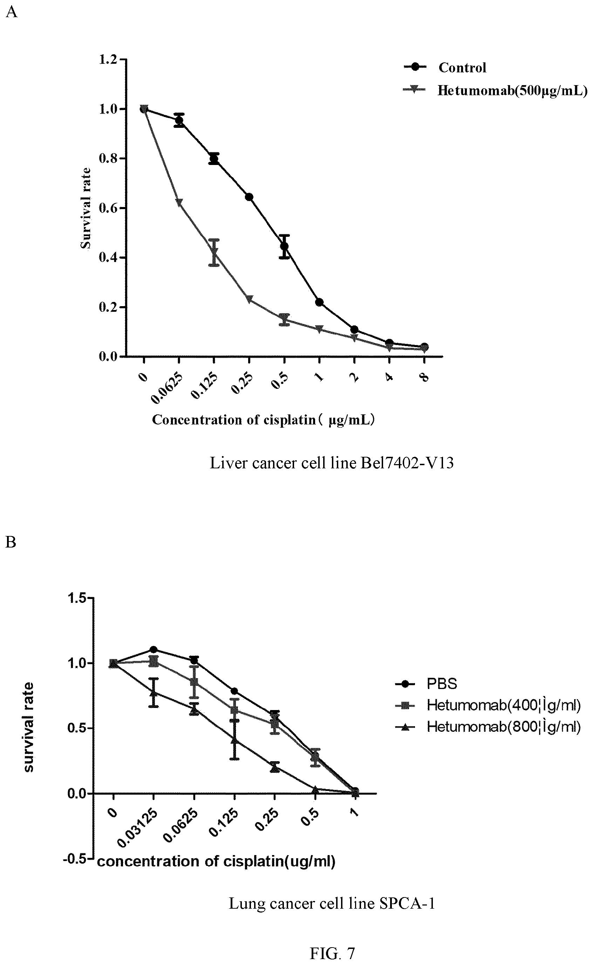

Drug resistance is one of the biological features of tumor stem cells. To demonstrate whether Hetumomab is a functional monoclonal antibody capable of directly inhibiting liver cancer stem cells, Bel7402-V13 sphere cells cultured for 5 days were seeded in a 96-well plate at 5000 cells/well, and the purified monoclonal antibody Hetumomab (0.5mg/mL) and PBS were added in the wells respectively, and after incubating at 37°C in 5% CO₂ incubator for 24h, the antibody-containing medium was removed. Each group of cells was cultured with a medium containing 9 different concentrations, i.e., 0, 0.0625, 0.125, 0.25, 0.5, 1, 2, 4, and 8 µg/mL, of cisplatin. The complete medium containing cisplatin was replaced once every 48 hours. After 5 days, CCK-8 reagent was added according to the instructions of CCK-8 kit. OD450 absorbance values were measured, and the IC50 values for each group were calculated. The experimental results (FIG. 7) showed that the drug resistance of the cells directly treated with Hetumomab was significantly decreased, with the IC50 value of 0.334 µg/mL, while the IC50 value of the control group was 0.9 µg/mL, and the drug resistance of cells directly acted by Hetumomab was significantly lower than that of the control group. The results showed that Hetumomab can directly act on liver cancer stem cells and inhibit their drug resistance.

In order to prove whether Hetumomab is a functional monoclonal antibody capable of directly inhibiting lung cancer stem cells, the same method was used to detect the inhibitory effect of Hetumomab on the drug resistance of human lung cancer cell line SPCA-1. The experimental results (FIG. 7) showed that the drug resistance of the cells directly treated with Hetumomab was significantly decreased, with the lowest IC50 value of 0.136 µg/mL, while the IC50 value of the control group was 0.351 µg/mL, and the drug resistance of cells directly acted by Hetumomab was significantly lower than that of the control group. The results showed that Hetumomab can directly act on lung cancer stem cells and inhibit their drug resistance ability.

These results indicate that Hetumomab can directly inhibit the chemotherapeutic drug resistance of various cancer cells (liver cancer, lung cancer), and that Hetumomab can not only recognize the target tumor stem cells, but also is a functional (therapeutic) monoclonal antibody against tumor stem cells capable of directly inhibiting the tumor stem cells.

### Example 5 Hetumomab has the pharmaceutical effect of inhibiting the growth of transplanted tumors and synergistic effect with chemotherapy in animals

The above experimental results demonstrated that Hetumomab is a monoclonal antibody that targets a variety of tumor stem cells; and the results of in vitro pharmaceutical studies show that Hetumomab can significantly inhibit the self-renewal, invasion ability and drug resistance of various tumor stem cells. In order to further clarify the in vivo effect of Hetumomab on the growth, metastasis and drug resistance of various tumors, a variety of human tumor animal models were used to evaluate the in vivo pharmaceutical effect of Hetumomab on the growth, metastasis and drug resistance of various tumors.

### 1. Hetumomab significantly inhibits the in vivo growth of human liver cancer transplanted tumors, and can synergically enhance therapeutic efficacy of chemotherapy, and significantly prolong the survival time, with significant anti-tumor pharmaceutical effect.

An experimental study on the treatment of liver cancer in nude mice by Hetumomab was carried out to observe and compare the therapeutic efficacy of the monoclonal antibody alone, the chemotherapeutic drugs alone and the combination of the monoclonal antibody and the chemotherapeutic drugs in treating liver cancer, to determine the in vivo effect of Hetumomab on the growth and drug resistance of liver cancer, and to explore the optimal treatment of liver cancer by targeting liver cancer stem cells.

Bel7402-V13 sphere cells were inoculated subcutaneouly to nude mice at 30,000 cells/mouse. The nude mice were randomly divided into 6 groups (6 per group) as follows: the chemotherapy group (0.3mg/kg cisplatin, 6 mice); the high dose antibody group (10 mg/kg, 6 mice); the high dose antibody + chemotherapy group (6 mice); the low dose antibody group (2.5 mg/kg, 6 mice); the low dose antibody + chemotherapy group (6 mice); and the PBS group (6 mice). The treatments were started the next day after the cells were inoculated, twice a week, and terminated after 5 weeks. The major and minor diameters of the subcutaneously transplanted tumor were measured twice a week. The tumor volume was calculated with the formula V = (π / 6) × (major diameter × minor diameter × minor diameter), and the changes in tumor volume and growth rate of each group were observed. After drug withdrawal, the growth of the transplanted tumors was observed again, and the tumor size was recorded. Tumor growth rate = (Vₜ - V₀) / number of days, where Vₜ is the tumor volume at each measurement, and V₀ is the tumor volume before administration (V₀ is the tumor volume when the administration was stopped).

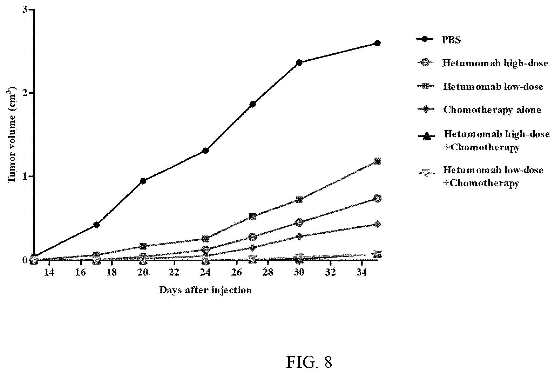

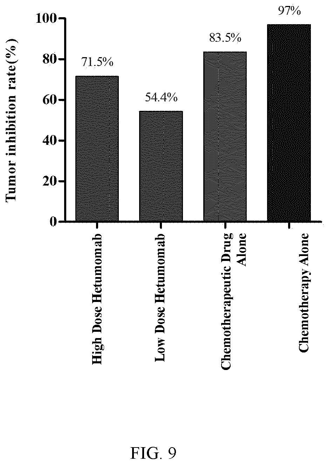

The growth curve of transplanted tumors in mice is shown in FIG. 8, which shows that Hetumomab can significantly inhibit the growth of transplanted tumors in nude mice. And with the increase of the dose of the antibody, the inhibition rate of the transplanted tumors gradually increased, which showed a dose-dependent relationship. The experimental results are shown in FIG. 9. When the drug was discontinued after 5 week treatment, the inhibition rates of the high and low dose Hetumomab group on the transplanted tumors were 71.5% and 54.4%, respectively, while the inhibition rate of the chemotherapy group was 83.5%, and the inhibition rate of the groups with high dose antibody, low dose antibody combined with chemotherapeutic drug were similar, about 97%. The results suggested that the monoclonal antibody combined with the chemotherapeutic drug showed a better therapeutic effect in the treatment of the transplanted tumors, compared with the monoclonal antibody alone and the chemotherapeutic drug alone.

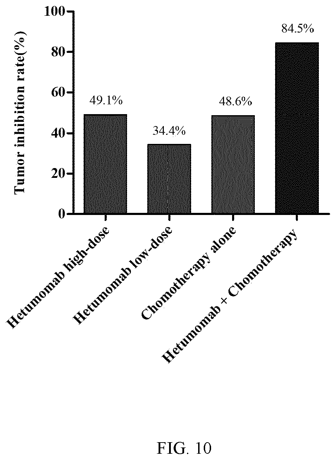

After one month of drug withdrawal, some mice died. At this time point, the inhibition rate of each group is shown in FIG. 10. The inhibition rates of high dose and low dose monoclonal antibody groups on the transplanted tumors were 49.1% and 34.4%, respectively, while the inhibition rates of the groups of high and low dose monoclonal antibody combined with chemotherapeutic drugs were similar, at about 84.5%, but the inhibition rate of the chemotherapy group was 48.6%. The results suggested that the inhibition rate of the monoclonal antibody combined with the chemotherapeutic drug group on the transplanted tumors in the mice was higher than that of other treatment groups, *P* <0.05, with statistically significant difference. The volume of the transplanted tumor at one month after drug withdrawal was compared with that at the time of drug withdrawal. The tumor growth rate of the monoclonal antibody combined chemotherapeutic drug group was 0.051 cm³/day, which was 4.7 times lower than that of the PBS control group (0.239 cm³/day). The tumor growth rate of the combination group was 2.9, 3.6, and 3.1 times lower than those of the high and low dose monoclonal antibody groups and the chemotherapy group (0.148 cm³/day, 0.185 cm³/day, and 0.154 cm³/day), respectively. This result showed that the method for administering the combination of the monoclonal antibody and the chemotherapeutic drugs may effectively inhibit tumor growth and recurrence.

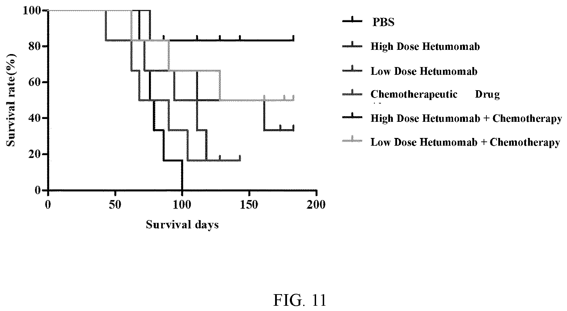

The entire treatment and observation lasted six months. The six-month survival curve of the mice (FIG. 11) showed that there was a statistically significant difference in the survival curves of the six groups of mice, *P* <0.05. This shows that the survival status of mice in the monoclonal antibody combined with chemotherapy group was significantly better than that in the PBS control group, the monoclonal antibody group and the chemotherapy group. It is suggested that monoclonal antibody combined with chemotherapy may prolong the survival of mice.

The above results show that Hetumomab alone may significantly inhibit the growth of human liver cancer transplanted tumors and has a significant pharmaceutical effect of inhibiting the liver cancer. The combinations of Hetumomab and the chemotherapeutic drug all showed a high inhibition rate against transplanted tumors, which could significantly inhibit tumor growth, with a better treatment effect than that of the antibody alone group and the chemotherapeutic drug alone, which indicates that the monoclonal antibody combined with the chemotherapeutic drug may effectively inhibit the tumor growth and reduce the chemotherapeutic drug resistance. The survival time of mice in the combination therapy group was significantly longer than that in the antibody group and that in the chemotherapeutic drug group, which indicates that Hetumomab combined with chemotherapeutic drug may not only treat the tumor growth, but also prolong the survival time of mice. It is possible that the death of the mice caused by the in vivo metastasis of tumors was significantly inhibited.

### 2. Hetumomab significantly inhibits the growth of human lung cancer transplanted tumors in vivo and has a significant anti-tumor pharmaceutical effect.

An experimental study on the in vivo treatment of lung cancer in nude mice by Hetumomab was performed, and the therapeutic efficacy of the treatment of lung cancer by the monoclonal antibody alone and the chemotherapeutic drugs alone was observed.

Specifically, the sphere cells of the human lung cancer cell line SPCA-1 were harvested and inoculated in nude mice at 2.5×10⁵ cells/mouse. The mice were divided into following 5 groups, with 5 mice in each group: the PBS control group; the chemotherapy group (cisplatin 0.3 mg/kg); the high dose antibody Hetumomab group (40 mg/kg); the medium dose antibody Hetumomab group (10 mg/kg); and the low dose Hetumomab group (2.5 mg/kg). The antibody treatment was started on the second day after inoculation of lung cancer cells. The experimental groups and the control group were treated by intraperitoneal injection. The treatments were performed for 28 days after inoculation. The chemotherapeutic drug group was treated twice a week. The major and minor diameters of the subcutaneously transplanted tumors were measured twice a week. The tumor volume was calculated with the formula V = (π / 6) × (major diameter × minor diameter × minor diameter). The change in tumor volume in each group was observed.

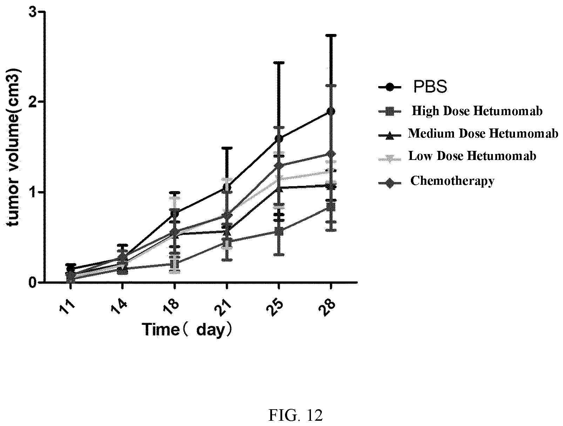

When the treatment was stopped 28 days after inoculation, the tumor volume growth of the mice was observed and measured, and the inhibition rate was calculated. The growth curve of the transplanted tumors in the mice is shown in FIG. 12, and the tumor volume inhibition rate is shown in Table 22. Hetumomab may significantly inhibit the growth of transplanted tumors in nude mice, with its inhibition rate increased along with the increase of the dose of the antibody. At the time of drug withdrawal, the inhibition rates of the groups of high dose, medium dose and low dose monoclonal antibody Hetumomab on the transplanted tumors were 55.97%, 43.56%, and 35.58%, respectively, while the inhibition rate of the chemotherapeutic drug group was only 24.91%.

The above results show that Hetumomab alone may significantly inhibit the growth of human lung cancer transplanted tumors and has a significant pharmaceutical effect on lung cancer.

**Table 22 Inhibitory effect of Hetumomab on the growth of human lung cancer SPCA-1 transplanted tumors within animals**

| Group | Tumor Volume Inhibition Rate (%) |
|---|---|
| PBS | |
| Chemotherapeutic Drug Alone | 24.91 |
| High Dose Hetumomab | 55.97 |
| Medium Dose Hetumomab | 43.56 |
| Low Dose Hetumomab | 35.58 |

### 3. Hetumomab significantly inhibits the in vivo growth of human gastric cancer transplanted tumors, and can synergically enhance therapeutic efficacy of chemotherapy, and significantly prolong the survival time, with significant anti-tumor pharmaceutical effect.

An experimental study on the treatment of gastric cancer in nude mice by Hetumomab was carried out. The therapeutic efficacy of the monoclonal antibody alone, the chemotherapeutic drugs alone and the combinantion of the monoclonal antibody and the chemotherapeutic drugs in treating lung cancer was observed and compared. It was analyzed whether Hetumomab can inhibit the growth of gastric tumors in vivo and synergically enhance therapeutic effect of chemotherapy.

Specifically, the sphere cells of the human gastric cancer cell line SNU-5-V13 were harvested and inoculated in nude mice at 2.5 × 10⁵ cells/mouse. The mice were divided into following 7 groups, with 8 mice in each group: the PBS control group; the mouse IgG control group; the chemotherapy group (cisplatin 0.3 mg/kg); the high dose antibody Hetumomab group (20 mg/kg); the low dose Hetumomab group (1.25 mg/kg); the high dose Hetumomab + chemotherapeutic drug group; and the low dose Hetumomab + chemotherapeutic drug group. The antibody treatment was started on the second day after inoculation of gastric cancer cells. The experimental groups and the control group were treated by intraperitoneal injection. The treatments were performed for one month. The chemotherapeutic drug group was treated for 4 weeks, twice a week. The major and minor diameters of the subcutaneously transplanted tumor were measured twice a week. The tumor volume was calculated with the formula V = (π / 6) × (major diameter × minor diameter × minor diameter). The change in tumor volume in each group was observed.

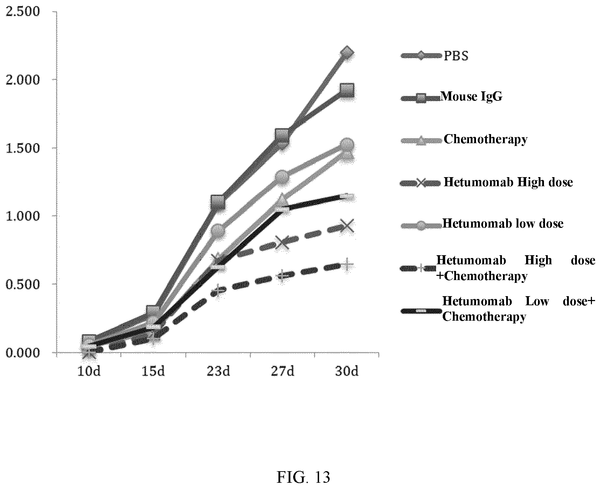

When the treatments were stopped one month after inoculation, the tumor volume growth of the mice was observed and measured, and the inhibition rate was calculated. The growth curve of the transplanted tumors in the mice is shown in FIG. 13, and the tumor volume inhibition rate is shown in Table 23. Hetumomab can significantly inhibit the growth of transplanted tumors in nude mice, with its inhibition rate on the transplanted tumors increased along with the increase of the dose of the antibody. At the time of drug withdrawal, the inhibition rates of the groups of high dose and low dose monoclonal antibody Hetumomab on the transplanted tumors were 57.62%, and 30.68% respectively, while the inhibition rate of the chemotherapeutic drug group was only 33.16%. The inhibition rates of the groups of high- dose + chemotherapeutic drug and low-dose + chemotherapeutic drug were 70.68% and 47.93%, respectively. The results suggested that the inhibitory rates of the combinations of monoclonal antibody and chemotherapeutic drug on the transplanted tumors in the mice were higher than those of chemotherapeutic drug group and the antibody group, with *p* <0.05, and that the combinations of the monoclonal antibody and chemotherapeutic drug showed better therapeutic effect in the treatment of the transplanted tumors.

The above results show that Hetumomab alone may significantly inhibit the growth of human gastric cancer transplanted tumors and has a significant pharmaceutical effect of inhibiting gastric cancer. The combinations of Hetumomab and chemotherapeutic drug all showed a high inhibition rate against transplanted tumors, could significantly inhibit the tumor growth, thus exhibited a better treatment effect than that of the antibody alone group and the chemotherapeutic drug alone group, which indicates that the monoclonal antibody combined with the chemotherapeutic drug may effectively inhibit the tumor growth and reduce the chemotherapeutic drug resistance.

**Table 23 Inhibitory effect of Hetumomab on the growth of human gastric cancer SNU-5 transplanted tumors within animals**

| Grouping | Tumor Volume Inhibition Rate (%) |
|---|---|
| PBS | |
| Mouse IgG | 12.47 |
| Chemotherapeutic Drug Alone | 33.16 |
| High Dose Hetumomab | 57.62 |
| Low Dose Hetumomab | 30.68 |
| High Dose Hetumomab + Chemotherapeutic Drug | 70.68 |
| Low Dose Hetumomab + Chemotherapeutic Drug | 47.93 |

The the above series of animal experiment results of inhibiting tumors demonstrated that Hetumomab has a significant inhibitory effect (pharmacodynamic effect) on the growth and drug resistance of a variety of human tumors (such as liver cancer, lung cancer, and gastric cancer) in vivo, and has important application value for treating the growth, metastasis and drug resistance of many tumors.

### Example 6 Cloning of the variable region sequences of mouse monoclonal antibody Hetumomab and sequence analysis of its complementarity determining regions

In this example, hybridoma cells of the mouse monoclonal antibody Hetumomab with subtype IgG1 and Kappa light chain was used. The cells were lysed with Trizol reagent to extract the total RNA. After precipitation with isopropyl alcohol and washing with ethanol, RNA electrophoresis and UV spectrophotometry were used to determine concentration, purity, and integrity of RNA. As a result, total RNA from Hetumomab hybridoma cells was obtained at a concentration of 2.5 µg/µl and OD260/OD280 = 1.8. They were reverse transcribed into first chain cDNA by conventional methods and diluted twice to be used as the template for PCR amplification.

Appropriate variable region primer combinations were designed for subsequent routine PCR reactions to amplify the variable region gene sequences of antibody which were cloned into the ZT4-Blunt vector and transformed into E. coli DH5α competent cells. Positive clones were selected for sequencing. The amino acid sequence of the light chain variable region of Hetumomab is shown in SEQ ID NO: 1, and the amino acid sequence of the heavy chain variable region is shown in SEQ ID NO: 5.

The amino acid sequence of the light chain variable region of Hetumomab (SEQ ID NO: 1) is: 
DIVMTQAAFSNPVTLGTSASISCRSSKSLLHSNGITYLYWFLQKPGQSPQLLIYQM
SNLASGVPDRFSSSGSGTDFTLRISRVEAEDVGVYYCAQNLELYTFGGGTKLEIK
 where the VL-CDR1 (SEQ ID NO: 2: RSSKSLLHSNGITYLY), VL-CDR2 (SEQ ID NO: 3: QMSNLAS), and VL-CDR3 (SEQ ID NO: 4: AQNLELYT) are underlined respectively.

The amino acid sequence of the heavy chain variable region of Hetumomab (SEQ ID NO: 5) is: 
QVTLKESGPGILQPSQTLSLTCSFSGFSLSTSGMGVSWIRQPSGKGLEWLAHIYW
DDDKRYNPSLKSRLTISKDSSNNQVFLKITSVDTADTATYYCARSFYYYANNSFAYWG
QGTLVTVSS
 where the VH-CDR1(SEQ ID NO:6: TSGMGVS), VH-CDR2(SEQ ID NO:7: HIYWDDDKRYNPSLKS), VH-CDR3(SEQ ID NO:8: SFYYYANNSFAY) are underlined respectively.

### Example 7 Construction, expression and purification of a human-mouse chimeric antibody Hetuximab derived from the mouse monoclonal antibody Hetumomab

In this example, the light and heavy chain variable region gene fragments of the mouse monoclonal antibody Hetumomab were cloned into the transient expression vectors containing the human IgG1 CH (heavy chain constant region gene) and human IgG CK (light chain constant region gene), then transfected into eukaryotic cells. The human-mouse chimeric antibody variant of Hetumomab was purified from the culture supernatant of the transfected cells by conventional Protein A affinity chromatography and named as Hetuximab.

The heavy chain and light chain antibody variable region gene fragments of Hetumomab were amplified by PCR in large sacle and cloned into the corresponding restriction sites of pKN009 (containing human IgG1 CH coding sequence) and pKN019 (containing human IgG CK coding sequence) transient expression vectors, transformed into E. coli, and positive clones were selected. The positive clones were further sequenced for identification. As a result, a human-mouse chimeric antibody Hetuximab derived from Hetumomab and its expression vectors were obtained. The specific protein sequences of the chimeric antibody were as follows:

The amino acid sequence of the heavy chain of chimeric antibody Hetuximab (SEQ ID NO: 9) is: 
QVTLKESGPGILQPSQTLSLTCSFSGFSLSTSGMGVSWIRQPSGKGLEWLAHIYW
DDDKRYNPSLKSRLTISKDSSNNQVFLKITSVDTADTATYYCARSFYYYANNSFAYWG
QGTLVTVSSASTKGPSVFPLAPSSKSTSGGTAALGCLVKDYFPEPVTVSWNSGALTSG
VHTFPAVLQSSGLYSLSSVVTVPSSSLGTQTYICNVNHKPSNTKVDKKVEPKSCDKTH
TCPPCPAPELLGGPSVFLFPPKPKDTLMISRTPEVTCVVVDVSHEDPEVKFNWYVDGV
EVHNAKTKPREEQYNSTYRVVSVLTVLHQDWLNGKEYKCKVSNKALPAPIEKTISKA
KGQPREPQVYTLPPSREEMTKNQVSLTCLVKGFYPSDIAVEWESNGQPENNYKTTPPV
LDSDGSFFLYSKLTVDKSRWQQGNVFSCSVMHEALHNHYTQKSLSLSPGK
 where the VH-CDR1 (SEQ ID NO:6), VH-CDR2 (SEQ ID NO:7), VH-CD3(SEQ ID NO:8), human IgG1 CH (SEQ ID NO: 11) are underlined respectively.

The amino acid sequence of the light chain of Hetuximab chimeric antibody (SEQ ID NO: 10) is: 
DIVMTQAAFSNPVTLGTSASISCRSSKSLLHSNGITYLYWFLQKPGQSPQLLIYQM
SNLASGVPDRFSSSGSGTDFTLRISRVEAEDVGVYYCAQNLELYTFGGGTKLEIKRTV
AAPSVFIFPPSDEQLKSGTASVVCLLNNFYPREAKVQWKVDNALQSGNSQESVTEQD
SKDSTYSLSSTLTLSKADYEKHKVYACEVTHQGLSSPVTKSFNRGEC
 where the VL-CDR1 (SEQ ID NO:2), VL-CDR2 (SEQ ID NO:3), VL-CDR3 (SEQ ID NO:4), human IgG C (SEQ ID NO:12) are underlined respectively.

The light and heavy chain chimeric antibody gene expression vectors verified were conventionally transfected into eukaryotic HEK-293 cells using liposome transfection to transiently express the chimeric antibody. The supernatants were collected for preliminary purification using a Protein A affinity chromatography column, and then impurities were removed using cation exchange chromatography. The purity of the purified product was verified by SDS-PAGE electrophoresis. As a result, a human-mouse chimeric antibody Hetuximab derived from Hetumomab with a purity >95% and 2 mg/ml were obtained.

### Example 8 The chimeric antibody Hetuximab recognizes and binds to the same epitope of the same antigenic protein as the parental mouse monoclonal antibody and has the same specificity and comparable affinity.

### 1. Western Blotting was used to demonstrate that the chimeric antibody Hetuximab and the parent mouse monoclonal antibody Hetumomab recognize and bind to the same antigen protein and have the same specificity.

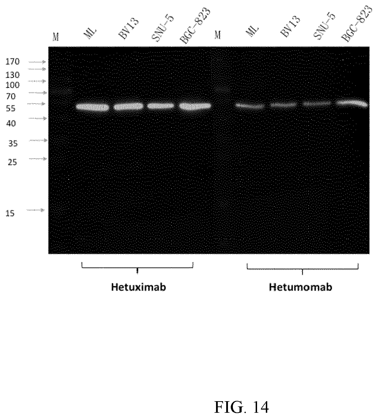

Total proteins of 4 kinds of Hetumomab target antigen-positive human tumor cells SNU-5, BGC-823, MHCC-97L, BEL7402 V13 were extracted by conventional methods, and the protein concentrations were quantified by a BCA kit. An appropriate amount of the protein sample (20 µg) was separated by SDS-polyacrylamide gel electrophoresis and transferred to a PVDF membrane using a semi-dry electroporator. The PVDF membrane was then placed in blocking solution (TBST / 5% skimmed milk powder) and incubated on a horizontal shaker at room temperature for 1 h. Then the primary antibody (the chimeric antibody Hetuximab and the parental mouse monoclonal antibody Hetumomab, each 2 ug/ml, diluted with the blocking solution), and incubated at 4 °C overnight. The membrane was then rinsed 5 times with TBST for 5 min each. Then the secondary antibody (1:2000 to 1:5000 dilution) against human IgG Fc-HRP or against mouse IgG Fc-HRP was added accordingly and incubated at room temperature for 1 h. The membrane was then rinsed 3 times with TBST for 5 min each, and then rinsed with PBS 3 times. Visualization was conducted. The results are shown in FIG. 14.

The results showed that the chimeric antibody Hetuximab and the parental mouse monoclonal antibody Hetumomab recognize a same protein molecule. Because they both specifically bound to the antigen protein and did not generate any bands for other proteins of human eukaryotic cells, the chimeric antibody Hetuximab and the parental mouse monoclonal antibody Hetumomab have the same specificity.

### 2. Immunohistochemistry was used to demonstrate that the chimeric antibody Hetuximab and the parent mouse monoclonal antibody Hetumomab recognize and bind to the same antigen protein and have the same specificity.

Using the aforementioned conventional immunohistochemical techniques, with Hetumomab used as the primary antibody and anti-mouse antibody used as the secondary antibody, and with the chimeric antibody Hetuximab used as the primary antibody and the anti-human antibody used as the secondary antibody, 3 Hetumomab target antigen-positive tissue sections from human liver cancer, lung cancer, and gastric cancer patients and 3 negative tissue sections of human liver cancer, lung cancer, and gastric cancer patients were detected.

The experimental results showed that the chimeric antibody Hetuximab and the parental mouse monoclonal antibody Hetumomab could both positively stain the 3 Hetumomab target antigen-positive tissue sections from human liver cancer, lung cancer, and gastric cancer patients, but failed to stain the 3 negative tissue sections from human liver cancer, lung cancer, and gastric cancer patients, indicating that the chimeric antibody Hetuximab and the parent mouse monoclonal antibody Hetumomab recognized and bound to a same antigen protein, and did not recognize other proteins in human tissues, and thus have the same specificity.

### 3. Competitive inhibition cell ELISA was performed to demonstrate that the chimeric antibody Hetuximab and the parental mouse monoclonal antibody Hetumomab recognize and bind to the same epitope of the same antigen protein and have comparable affinity.

MHCC-97L cells were seeded in a 96-well culture plate (4×10³ cells/well), and experiments were performed when the cells grew to 90% to 100%. After discarding the culture medium in the wells, the plate was washed with PBS containing 0.05% Tween-20, 300 µl/well, 1 min× 5 times. The samples were then added based on the following design in duplicate, 100 µl/well, and incubated at 37°C for 1.5 h. After discarding the liquid from the wells, the plate was washed with PBS containing 0.05% Tween-20, 300 µl/well, 1 min x 5 times. Corresponding secondary antibodies against human IgG Fc-HRP (nonreactive with mouse IgG Fc) or against mouse IgG Fc-HRP (nonreactive with human IgG Fc) 1:5000 diluted was then added, 100 µl/well, and incubated for 1 h at 37 °C. After discarding the liquid in the wells, the plate was washed with PBS containing 0.05% Tween-20, 300 µl / well, 1 min× 3 times, and then washed again with pure water for two times. After shaking off the pure water, TMB was then added, 100 µl/well, and then incubated at 37 °C for 30 min for visualization; and then 2M H₂SO₄ was added, 50 µl/well. After that, the OD450 was measured by microplate reader.

When the anti-human IgG Fc-HRP (not reactive with mouse IgG Fc) was used as the secondary antibody, the results are shown in the following table:

**Table 24**

| **Antibody Sample** | **Average OD450** | **Antibody Sample** | **Average OD450** | **Antibody Sample** | **Average OD450** |
|---|---|---|---|---|---|
| 0.25µg/ml Hetuximab+0µg/ml Hetumomab | 2.901 | 0.25µg/ml Hetuximab | 2.743 | 0.25µg/ml Hetumomab | 0.082 |
| 0.25µg/ml Hetuximab+ 0.0825µg/ml Hetumomab | 2.644 | 0.3325µg/ml Hetuximab | 2.747 | 0.3325µg/ml Hetuximab | 0.075 |
| 0.25µg/ml Hetuximab+ 0.25µg/ml Hetumomab | 2.544 | 0.5µg/ml Hetuximab | 2.785 | 0.5µg/ml Hetumomab | 0.082 |
| 0.25µg/ml Hetuximab+ 0.75µg/ml Hetumomab | 2.257 | 1 µg/ml Hetuximab | 2.983 | 1µg/ml Hetumomab | 0.112 |
| 0.25µg/ml Hetuximab+ 1.75 µg/ml Hetumomab | 1.750 | 2µg/ml Hetuximab | 3.103 | 2µg/ml Hetumomab | 0.133 |
| 0.25µg/ml Hetuximab+ 3.75µg/ml Hetumomab | 1.344 | 4µg/ml Hetuximab | 3.127 | 4µg/ml Hetumomab | 0.161 |
| 0.25µg/ml Hetuximab+ 7.75 µg/ml Hetumomab | 0.865 | 8µg/ml Hetuximab | 3.192 | 8µg/ml Hetumomab | 0.177 |
| 0.25µg/ml Hetuximab+ 15.75µg/ml Hetumomab | 0.604 | 16µg/ml Hetuximab | 3.227 | 16µg/ml Hetumomab | 0.210 |

When the anti-mouse IgG Fc-HRP (not reactive with human IgG Fc) was used as the secondary antibody, the results are shown in the following table:

**Table 25**

| **Antibody Sample** | **Average OD450** | **Antibody Sample** | **Average OD450** | **Antibody Sample** | **Average OD450** |
|---|---|---|---|---|---|
| 0.25µg/ml Hetumomab + 0µg/ml Hetuximab | 2.278 | 0.25µg/ml Hetuximab | 0.065 | 0.25µg/ml Hetumomab | 2.168 |
| 0.25µg/ml Hetumomab + 0.0825µg/ml Hetuximab | 1.964 | 0.3325µg/ml Hetuximab | 0.042 | 0.3325µg/ml Hetuximab | 2.234 |
| 0.25µg/ml Hetumomab + 0.25 µg/ml Hetuximab | 1.772 | 0.5 µg/ml Hetuximab | 0.031 | 0.5 µg/ml Hetumomab | 2.361 |
| 0.25µg/ml Hetumomab + 0.75 µg/ml Hetuximab | 1.178 | 1µg/ml Hetuximab | 0.050 | 1µg/ml Hetumomab | 2.523 |
| 0.25µg/ml Hetumomab + 1.75 µg/ml Hetuximab | 0.757 | 2µg/ml Hetuximab | 0.043 | 2µg/ml Hetumomab | 2.674 |
| 0.25µg/ml Hetumomab + 3.75µg/ml Hetuximab | 0.527 | 4µg/ml Hetuximab | 0.043 | 4µg/ml Hetumomab | 2.702 |
| 0.25µg/ml Hetumomab + 7.75µg/ml Hetuximab | 0.324 | 8µg/ml Hetuximab | 0.046 | 8µg/ml Hetumomab | 2.726 |
| 0.25µg/ml Hetumomab + 15.75 µg/ml Hetuximab | 0.208 | 16µg/ml Hetuximab | 0.044 | 16µg/ml Hetumomab | 2.818 |

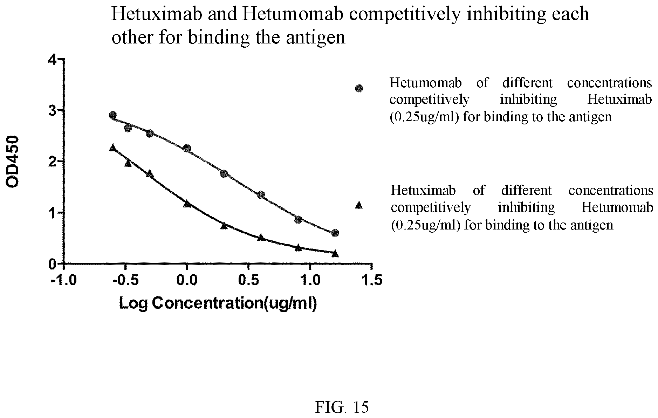

The original results of Tables 24 and 25 are plotted and shown in FIG. 15. It can be seen from FIG. 15 that the chimeric antibody Hetuximab and the parental mouse monoclonal antibody Hetumomab significantly competed with each other to inhibit the binding of the other one to the target antigen on the surface of the target cell MHCC-97L cells, which indicates that the chimeric antibody Hetuximab and the parental mouse monoclonal antibody Hetumomab recognize and bind to the same epitope of the same antigen protein, and they show similar competitive inhibition efficiency, suggesting that the chimeric antibody Hetuximab and the parental mouse monoclonal antibody Hetumomab have comparable affinity.

### Example 9 The chimeric antibody Hetuximab and the parental mouse monoclonal antibody recognize the same tumor stem cells and have the same pharmaceutical effect of inhibiting tumor stem cells.

### 1. The chimeric antibody Hetuximab and the parental mouse monoclonal antibody Hetumomab recognize the same group of tumor stem cells.

As described above, four cell lines, SNU-5, BGC-823, MHCC-97L, and BEL7402 V13, were used to harvest both parental cells and tumor-stem-cell-rich sphere cells. Hetuximab (using anti-human IgG Fc as the secondary antibody) and Hetumomab (using anti-mouse IgG Fc as the secondary antibody) were used respectively for flow immunofluorescence experiments, the proportion of positive cells in the parental cells and the tumor-stem-cell-rich sphere cells of the above 4 cell lines, and their fold enrichment in the sphere cells were detected. The results are as follows:

**Table 26. Comparison of the positive rates of Hetuximab and Hetumomab in different tumor cells and the fold enrichment in sphere cells.**

| | *Hetuximab* | | | *Hetumomab* | | |
|---|---|---|---|---|---|---|
| | Parental Cells | Sphere Cells | Fold Enrichment | Parent Cells | Sphere Cells | Fold Enrichment |
| SNU-5 | 7.92% | 11% | 1.4 | 6.91% | 9.75% | 1.4 |
| BGC-823 | 5.5% | 6.8% | 1.2 | 4.3% | 5% | 1.2 |
| BEL7402 V13 | 8.15% | 11.9% | 1.5 | 7.59% | 12% | 1.6 |
| MHCC-97L | 5.18% | 11.8% | 2.3 | 7.38% | 15.98% | 2.2 |

From the above results, it can be seen that the two antibodies have very consistent positive rate and enrichment in the 4 kinds of tumor cells and tumor-stem-cell-rich sphere cells. Therefore, the chimeric antibody Hetuximab and the parent mouse monoclonal antibody Hetumomab recognize the same group of tumor stem cells.

### 2. The chimeric antibody Hetuximab and the parental mouse monoclonal antibody have the same pharmaceutical effect of inhibiting tumor stem cells in vitro.

With the same process described above, by using the tumor-stem-cell-rich sphere cells of the four cell lines, i.e., the SNU-5, BGC-823, MHCC-97L, BEL7402 V13 cell lines, and by using Hetuximab and Hetumomab to perform conventional sphere-forming inhibition experiments, the pharmaceutical effects of inhibiting tumor stem cells of Hetuximab and Hetumomab on tumor-stem-cell-rich sphere cells of the above four cell lines were detected. The results are shown in the table below:

**Table 27. Hetuximab and Hetumomab's sphere-forming inhibition rate for the 4 cell lines**

| **SNU-5** | | **Concentration of antibody ug/ml** | **Average number of formed spheres** | **Inhibition Rate (%)** |
|---|---|---|---|---|
| | **Hetuximab** | 144 | 114.5 | 46.2 |
| | | 80 | 134 | 37.1 |
| | | 40 | 145.5 | 31.7 |
| | | 20 | 161.5 | 24.2 |
| | | 10 | 175 | 17.8 |
| | | 0 | 213 | - |
| | **Hetumomab** | 144 | 119 | 43.1 |
| | | 80 | 130 | 37.8 |
| | | 40 | 146 | 30.1 |
| | | 20 | 161 | 23.0 |
| | | 10 | 176.5 | 15.6 |
| | | 0 | 213 | - |
| | | | | |

| **BEL7402 V13** | | **Concentration of antibody ug/ml** | **Average number of formed spheres** | **Inhibition Rate (%)** |
|---|---|---|---|---|
| | **Hetuximab** | 144 | 106 | 53.3 |
| | | 80 | 124 | 45.4 |
| | | 40 | 143.5 | 36.8 |
| | | 20 | 169.5 | 25.3 |
| | | 10 | 190 | 16.3 |
| | | 0 | 227 | - |
| | **Hetumomab** | 144 | 106.5 | 53.1 |
| | | 80 | 126.5 | 44.3 |
| | | 40 | 142.5 | 37.2 |
| | | 20 | 171 | 24.7 |
| | | 10 | 193.5 | 14.8 |
| | | 0 | 227 | - |
| | | | | |

| **MHCC-97L** | | **Concentration of antibody ug/ml** | **Average number of formed spheres** | **Inhibition Rate (%)** |
|---|---|---|---|---|
| | **Hetuximab** | 144 | 120 | 48.3 |
| | | 80 | 140 | 39.7 |
| | | 40 | 152 | 34.5 |
| | | 20 | 169.5 | 26.9 |
| | | 10 | 191.5 | 17.5 |
| | | 0 | 232 | - |
| | **Hetumomab** | 144 | 121.5 | 47.6 |
| | | 80 | 141 | 39.2 |
| | | 40 | 155 | 33.2 |
| | | 20 | 170 | 26.7 |
| | | 10 | 194 | 16.4 |
| | | 0 | 232 | - |

| **BGC-823** | | **Concentration of antibody ug/ml** | **Average number of formed spheres** | **Inhibition Rate (%)** |
|---|---|---|---|---|
| | **Hetuximab** | 144 | 132 | 44.3 |
| | | 80 | 148 | 37.6 |
| | | 40 | 161.5 | 31.9 |
| | | 20 | 183 | 22.8 |
| | | 10 | 201 | 15.2 |
| | | 0 | 237 | - |
| | **Hetumomab** | 144 | 137.5 | 42.0 |
| | | 80 | 150 | 36.7 |
| | | 40 | 162 | 31.6 |
| | | 20 | 183 | 22.8 |
| | | 10 | 206 | 13.1 |
| | | 0 | 237 | - |

From the above results, it can be seen that the two antibodies at each individual concentration gradients have very consistent efficiency in inhibiting sphere-forming of tumor stem cells of the 4 kinds of tumor cell lines. Therefore, the chimeric antibody Hetuximab and the parent mouse monoclonal antibody Hetumomab have the same pharmaceutical effect of inhibiting tumor stem cells in vitro.

### Example 10 Humanization of the variable region sequences of mouse monoclonal antibody Hetumomab and modification of glycosylation sites

In this example, based on the variable region protein sequences of the mouse monoclonal antibody Hetumomab, glycosylation site modification and humanization of the Hetumomab were performed by sequence design, gene synthesis, cloning, and of eukaryotic cell transfection.

In the sequence library of the human antibody germline genes (http://www2.mrc-lmb.cam.ac.uk/vbase/alignments2.php#VHEX), based on the results of homology comparison, the most similar human antibody template was found. Finally, VH2 (3-1) was selected as the basic template for CDR transplantation, and Hetumomab VH CDR-1, VH CDR-2, and VH CDR-3 were transplanted into its FR framework. According to the sequence of Hetumomab VH CDR-3, the human germline JH4 (WGQGTLVTVSS) was selected as the sequence of the J region. Then a heavy chain variable region protein sequence with fully humanized heavy chain variable region FR was designed and named as Hetuzumab H1. The H1 version achieved full humanization in the framework region.

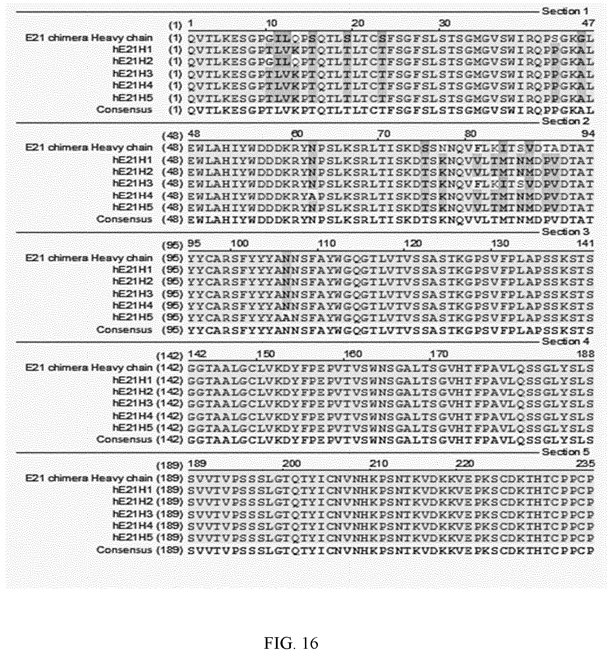

Based on the H1 heavy chain sequence, two other heavy chain mutants were designed as initial humanization: H2 and H3. The degree of humanization of these versions was in descending order. The specific sequences are shown in FIG. 16. Combining with the light chain of the chimeric antibody Hetuximab, it was found by cell ELISA that all three heavy chain mutants (H1, H2, H3) retained the affinity of the chimeric antibody Hetuximab. Since H1 was the most humanized version in which the framework regions were all humanized, H1 was finally determined as the preferred humanization molecular template.

Three mutants were also designed as the initial humanization of the light chain. Based on the results of homology comparison, the most similar human antibody template was found in the human antibody germline library (http://www2.mrc-lmb.cam.ac.uk/vbase/alignments2.php #VHEX). VK II (O11) and JK1 were selected as basic templates, and Hetumomab VL CDR-1, VL CDR-2, and VL CDR-3 were transplanted into their FR frameworks. A light chain variable region protein sequence with the light chain variable region FR fully humanized was designed and named as Hetuzumab L1. The L1 version achieved full humanization in the framework region.

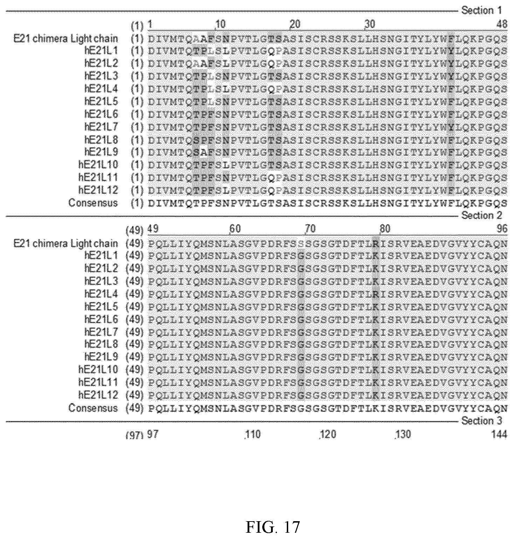

Based on the L1 with fully humanized FR region, L2 and L3 were designed, and the degree of humanization of the three version was also in descending order. However, by combining with the heavy chain of the chimeric antibody Hetuximab, it was found using a cellular ELISA method that the affinity of the three light chain mutants was greatly reduced. In order to find out which key amino acids were mutated during humanization, back mutations were performed, and nine different mutants (L4-L12) were prepared. The specific sequences are shown in FIG. 17.

By analyzing the affinity using the cellular ELISA method, it was found that the phenylalanine at the 9th and 42th positions (F9 and F42) of the light chain must be back mutated, and the affinity of the sixth mutant (L6) retaining these two mouse-derived amino acid residues were recovered. The affinity of the higher humanized version L11 based on L6 was close to that of L6, and the affinity of L12 with the 11th Asn of the light chain humanized was also similar to that of L11, but other mutant versions were different. Therefore, L6, L11, and L12 were finally selected for optimization screening of the best light chain humanized version.

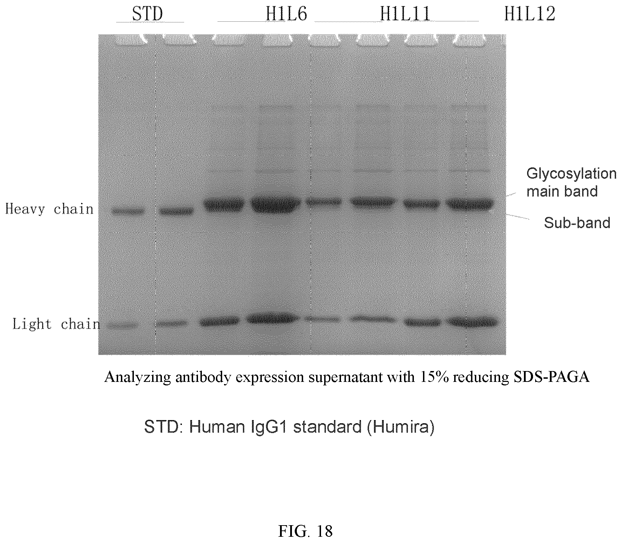

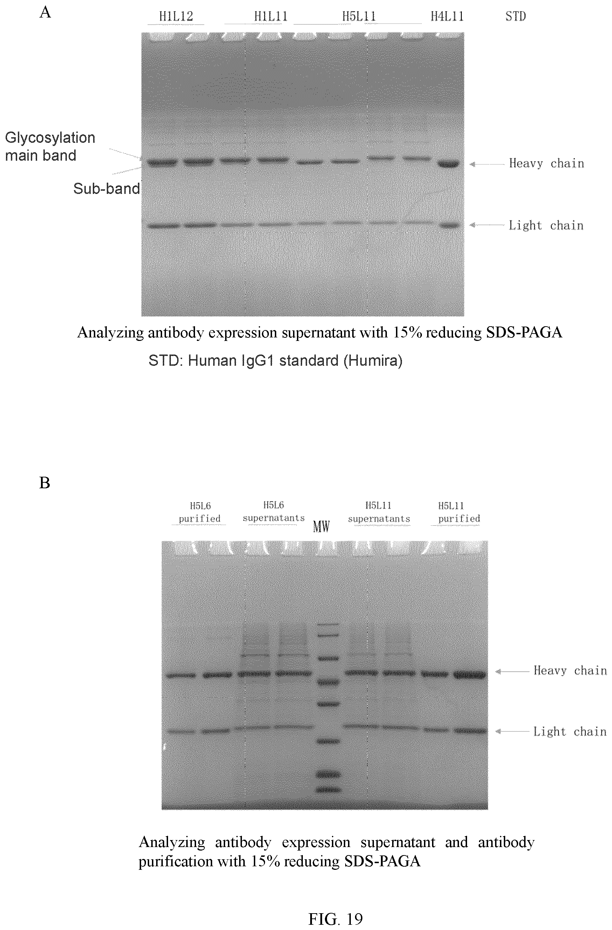

However, glycosylated isomers of Hetuzumab H1 heavy chain were confirmed by antibody sequence analysis and reductive electrophoresis analysis. In the analysis of H1 heavy chain expression products by electrophoresis, H1L6, H1L11, and H1L12 were all shown with weak sub-bands visible below the main band of the heavy chain. The analysis indicated that the heavy chain may have heterogeneous glycosylation which results in glycosylated isomers (FIG. 18). This may cause serious quality problems of uniformity, which is very unfavorable for drug development. The sequence analysis showed that N62 (located in CDR2) and N107 (located in CDR3) were two possible glycosylation sites. Alanine mutations were performed at these two sites, and the two new humanized versions of the heavy chain as obtained were designed: N62A as H4 (its CDR2 sequence shown in SEQ ID NO: 13), and N107A as H5 (its CDR3 sequence shown in SEQ ID NO: 14). H4 and H5 were combined with L11, respectively, and analyzed by electrophoresis after expression. It was found that the glycosylation isomerism of H5 (HI N107A) disappeared, and it was found by cell ELISA found that the H5L11 had maintained approximately comparable affinity; and the glycosylation isomerism of H4 (HI N62A) still existed and the affinity decreased (FIG. 19A). Furthermore, the combination of H5 with L11 and L6 confirmed that the glycosylation isomerism disappeared, and the heavy chain shown a uniform band (FIG. 19B). Therefore, H5 (HI N107A) is the finally selected variable region sequence.

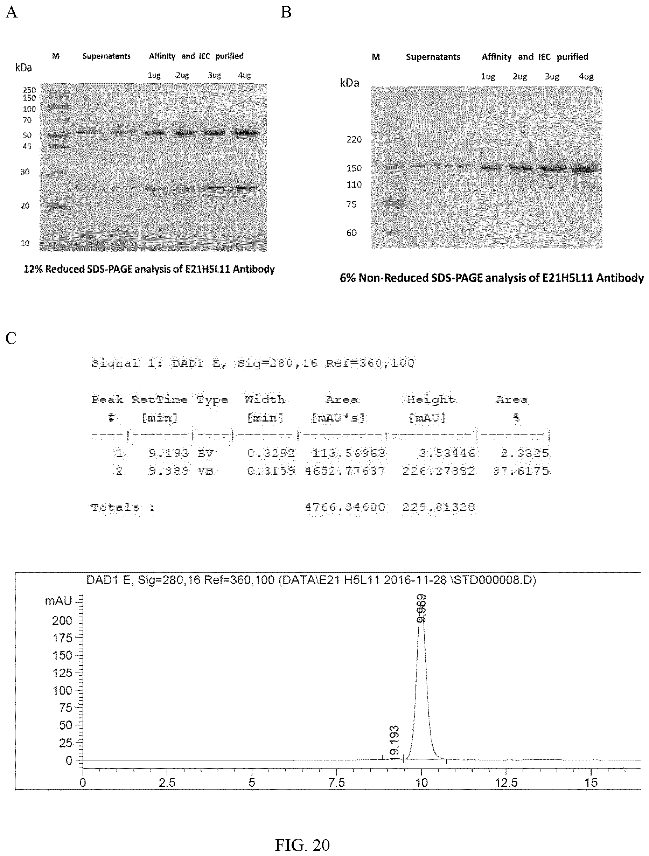

To determine the best humanized version, three candidate humanized combinations (H5L6, H5L11, H5L12) were selected to prepare purified antibodies. The H5L6, H5L11, and H5L12 light and heavy chain coding sequences were cloned into a eukaryotic high expression vector, and electrotransfected into the CHO-K1 (ATCC CCL-64) cells maintained in suspension culture. Stable expression strains were obtained by MSX screening, and fed-batch cultured with a serum-free protein-free CD-CHO medium (Invitrogen). The supernatant obtained by the culture was purified by Protein A (GE, Mabselect sure column) affinity chromatography column, and impurities were further removed by a cation exchange column. By SDS-PAGE and HPLC analysis, the purity of the product was greater than 95% (the results shown in FIG. 20, with H5L11 as an example), and the endotoxin level was <3EU / mg. It can be used for various pharmaceutical experiments and monkey pharmacokinetic analysis.

### Example 11 The three humanized variants (H5L6, H5L11, H5L12) of Hetuzumab all recognize and bind to the same epitope of the same antigen protein as the parental mouse monoclonal antibody.

### 1. Immunohistochemistry was used to demonstrated that the three humanized variants (H5L6, H5L11, H5L12) of Hetuzumab recognize and bind to the same antigen protein as the chimeric antibody Hetuximab and the parental mouse monoclonal antibody Hetumomab and have the same specificity.

Using the aforementioned conventional immunohistochemical techniques, with Hetumomab as the primary antibody and anti-mouse antibody as the secondary antibody; with the chimeric antibody Hetuximab as the primary antibody and the anti-human antibody as the secondary antibody; and with three humanized variants of Hetuzumab as the primary antibody and the anti-human antibody used as the secondary antibody, 10 Hetumomab target antigen-positive tissue sections from human liver cancer, lung cancer, and gastric cancer patients and 10 negative tissue sections of human liver cancer, lung cancer, and gastric cancer patients were detected.

The experimental results showed that the three humanized variants (H5L6, H5L11, H5L12) of Hetuzumab, the chimeric antibody Hetuximab and the parental mouse monoclonal antibody Hetumomab could positively stain the 10 Hetumomab target antigen-positive tissue sections from human liver cancer, lung cancer, and gastric cancer patients , but did not stain the 10 negative tissue sections from human liver cancer, lung cancer, and gastric cancer patients, which showed that the three humanized variants (H5L6, H5L11, H5L12) of Hetuzumab, the chimeric antibody Hetuximab and the parent mouse monoclonal antibody Hetumomab recognized and bound to the same antigen protein, and did not recognize other proteins in human tissues, and thus have the same specificity.

### 2. A competitive inhibition cell ELISA experiment was performed to demonstrate that the three humanized variants (H5L6, H5L11, H5L12) of Hetuzumab and the parental mouse monoclonal antibody Hetumomab recognize and bind to the same epitope of the same antigen protein, where H5L6, H5L11 also have comparable affinity with the mouse monoclonal antibody Hetumomab.

MHCC-97L cells were seeded in a 96-well culture plate (4×10³ cells/well), and experiments were performed when the cells grew to 90% to 100%. After discarding the culture medium in the wells, the plate was washed with PBS containing 0.05% Tween-20, 300 µl/well, 1 min× 5 times. The samples were then added in duplicate, 100 µl/well, and incubated at 37°C for 1.5 h. After discarding the liquid from the wells, the plate was washed with PBS containing 0.05% Tween-20, 300 µl/well, 1 min x 5 times. Corresponding secondary antibodies against human IgG Fc-HRP (not reactive with mouse IgG Fc) or against mouse IgG Fc-HRP (not reactive with human IgG Fc) 1:5000 diluted was then added, 100 µl/well, and incubated for 1 h at 37 °C. After discarding the liquid in the wells, the plate was washed with PBS containing 0.05% Tween-20, 300 µl / well, 1 min× 3 times, and then washed again with pure water for two times. After shaking off the pure water, TMB was then added, 100 µl/well, and then incubated at 37 °C for 30 min for visualization; and then 2M H₂SO₄ was added, 50 µl/well. OD450 was measured by microplate reader.

The results are shown in the Tables 28, 29 below and FIG. 21:

**Table 28 Anti-human IgG Fc-HRP (nonreactive with mouse IgG Fc) used as the secondary antibody:**

| Grouping of Samples | 0.25µg/ml Hetuzumab H5L16 | | 0.25µg/ml Hetuzumab H5L11 | | 0.25µg/ml Hetuzumab H5L12 | | 0.25µg/ml Hetuximab | |
|---|---|---|---|---|---|---|---|---|
| 0.0825µg/ml Hetumomab | 2.8605 | 2.8955 | 2.16950 | 2.15350 | 2.1985 | 2.1925 | 1.11950 | 1.18450 |
| 0.25 µg/ml Hetumomab | 2.7485 | 2.7055 | 2.00150 | 2.00150 | 1.9385 | 1.9625 | 0.89350 | 0.91750 |
| 0.75µg/ml Hetumomab | 2.4605 | 2.4425 | 1.47950 | 1.48750 | 1.4095 | 1.4335 | 0.55050 | 0.56650 |
| 1.75µg/ml Hetumomab | 1.9125 | 2.0175 | 0.89150 | 0.94950 | 0.8805 | 0.9055 | 0.30450 | 0.32050 |
| 3.75 µg/ml Hetumomab | 1.5115 | 1.4845 | 0.54650 | 0.56450 | 0.5505 | 0.5275 | 0.19250 | 0.20150 |
| 7.75 µg/ml Hetumomab | 1.0435 | 0.9985 | 0.35550 | 0.38750 | 0.3245 | 0.3325 | 0.14950 | 0.15550 |
| 15.75µg/ml Hetumomab | 0.6835 | 0.6475 | 0.24950 | 0.25950 | 0.2425 | 0.2485 | 0.11250 | 0.12250 |

**Table 29 Anti-mouse IgG Fc-HRP (nonreactive with human IgG Fc) used as the secondary antibody:**

| Grouping of Samples | Hetuzumab H5L16 | | Hetuzumab H5L11 | | Hetuzumab H5L12 | | Hetuximab | |
|---|---|---|---|---|---|---|---|---|
| 0.25µg/ml Hetumomab +0.0825µg/ml Hetuzumab/Hetuximab | 1.4055 | 1.4175 | 1.58350 | 1.56150 | 1.5845 | 1.5115 | 1.54250 | 1.86250 |
| 0.25µg/ml Hetumomab +0.25µg/ml Hetuzumab/Hetuximab | 1.1355 | 1.1785 | 1.38350 | 1.42250 | 1.3745 | 1.3405 | 1.62750 | 1.71150 |
| 0.25µg/ml Hetumomab +0.75µg/ml Hetuzumab/Hetuximab | 0.7525 | 0.7505 | 1.03150 | 1.02850 | 0.9995 | 0.9925 | 1.23850 | 1.30850 |
| 0.25µg/ml Hetumomab +1.75µg/ml Hetuzumab/Hetuximab | 0.4735 | 0.4625 | 0.75850 | 0.73350 | 0.6735 | 0.6995 | 1.03650 | 1.00750 |
| 0.25µg/ml Hetumomab +3.75µg/ml Hetuzumab/Hetuximab | 0.2745 | 0.2695 | 0.52050 | 0.53250 | 0.4985 | 0.5095 | 0.72550 | 0.76350 |
| 0.25µg/ml Hetumomab +7.75µg/ml Hetuzumab/Hetuximab | 0.1395 | 0.1305 | 0.32650 | 0.32750 | 0.3205 | 0.3035 | 0.54650 | 0.51750 |
| 0.25µg/ml Hetumomab +15.75µg/ml Hetuzumab/Hetuximab | 0.0785 | 0.0765 | 0.16650 | 0.19050 | 0.2025 | 0.1955 | 0.42250 | 0.41550 |

When the concentration of each humanized monoclonal antibody was 0.25 ug/ml and the mouse monoclonal antibody concentration gradients was used to competively inhibit the binding of each humanized monoclonal antibody to the antigen, the calculated IC50 results for the mouse monoclonal antibody are shown in Table 30 below.

**Table 30 IC50 of mouse monoclonal antibodies on humanized and chimeric monoclonal antibodies**

| IC50 of each group of mouse monoclonal antibody | Hetumomab Vs Hetuzumab H5L6 | Hetumomab Vs Hetuzumab H5L11 | Hetumomab Vs Hetuzumab H5L12 | Hetumomab Vs Hetuximab |
|---|---|---|---|---|
| IC50 (µg/ml) | 1.178 | 0.9757 | 0.1902 | 3.138 |

It can be seen from the above results that the parental mouse monoclonal antibody Hetumomab can significantly inhibit the binding of the three variants (H5L6, H5L11, H5L12) of Hetuzumab, the chimeric antibody Hetuximab to the target antigen on the surface of MHCC-97L cells, which indicates that the three variants (H5L6, H5L11, H5L12) of Hetuzumab, the chimeric antibody Hetuximab, and the parental mouse monoclonal antibody Hetumomab recognize and bind to the same epitope of the same antigen protein. The two variants (H5L6, H5L11) of Hetuzumab showed similar degrees of competitive inhibition, slightly higher than the chimeric antibody Hetuximab, while one variant (H5L12) of Hetuzumab showed significantly higher degree of competition inhibition. This suggested that the affinity of the chimeric antibody Hetuximab and the parental mouse monoclonal antibody Hetumomab were comparable. Although the affinity of the two humanized variants (H5L6, H5L11) of Hetuzumab were slightly reduced, they have a generally comparable affinity; while the affinity of the humanized variant H5L12 of Hetuzumab has decreased.

When the concentration of the mouse monoclonal antibody was 0.25 ug/ml and concentration gradient of each humanized monoclonal antibody was used to competively inhibit the binding of the mouse monoclonal antibody to the antigen, the calculated IC50 results for each humanized monoclonal antibody are shown in Table 31 below.

**Table 31 IC50 of the humanized monoclonal antibody and the chimeric monoclonal antibody for inhibiting the mouse monoclonal antibody**

| IC50 of each group | Hetuzumab H5L6 | Hetuzumab H5L11 | Hetuzumab H5L12 | Hetuximab |
|---|---|---|---|---|
| IC50 (µg/ml) | 0.7814 | 0.7180 | 1.412 | 0.4900 |

It can be seen from the above results the three variants (H5L6, H5L11, H5L12) of Hetuzumab and the chimeric antibody Hetuximab can significantly competively inhibit the binding of the parental mouse monoclonal antibody Hetumomab to the target antigen on the surface of MHCC-97L cells, which indicates that the three variants (H5L6, H5L11, H5L12) of Hetuzumab, the chimeric antibody Hetuximab, and the parental mouse monoclonal antibody Hetumomab recognize and bind to the same epitope of the same antigen protein; the two variants (H5L6, H5L11) of Hetuzumab showed similar competitive inhibition efficiency that was slightly lower than that of the chimeric antibody Hetuximab, while one variant (H5L12) of Hetuzumab showed a lower competitive inhibition efficiency. This suggested that the affinity of the chimeric antibody Hetuximab and the parental mouse monoclonal antibody Hetumomab were comparable. Although the affinity of the two variants (H5L6, H5L11) of the humanized antibody Hetuzumab were slightly reduced, they have generally comparable affinity. The affinity of humanized variant H5L12 of Hetuzumab has decreased.

It may be seen from these results that the three humanized variants (H5L6, H5L11, H5L12) of Hetuzumab, the chimeric antibody Hetuximab, and the parental mouse monoclonal antibody Hetumomab can all significantly compete against each other for binding to the target antigen on the surface of target cells MHCC-97L cells, which indicates that three humanized variants (H5L6, H5L11, H5L12) of Hetuzumab , the chimeric antibody Hetuximab, and the parental mouse monoclonal antibody Hetumomab recognize and bind to the same epitope of the same antigen protein. H5L6, H5L11, Hetuximab, and Hetumomab showed similar competitive inhibition with each other, suggesting that H5L6, H5L11 and the mouse monoclonal antibody Hetumomab have comparable affinity.

### Example 12 The humanized variants of Hetuzumab all recognize the same tumor stem cells as their parental monoclonal antibody and have the same pharmaceutical effect of inhibiting tumor stem cells in vitro and in vivo.

### 1. A flow immunofluorescence expriment was used to demonstrate that three humanized variants (H5L6, H5L11, H5L12) recognized the same group of tumor stem cells as the parental mouse monoclonal antibody Hetumomab

According to the aforementioned method, four cell lines, SNU-5, BGC-823, MHCC-97L, and BEL7402 V13, were used to harvest both parental cells and tumor-stem-cell-rich sphere cells. The 3 humanized variants (H5L6, H5L11, H5L12) of Hetuzumab, Hetuximab (using anti-human IgG Fc as the secondary antibody) and Hetumomab (using anti-mouse IgG Fc as the secondary antibody) were used respectively for flow immunofluorescence experiments, the proportion of positive cells in the parental cells and the tumor-stem-cell-rich sphere cells of the above 4 cell lines, and their fold enrichment in the sphere cells were detected. The results are as follows:

**Table 32 Positive rate of different antibodies in parental cells and sphere cells of 4 cell lines and their fold enrichment in sphere cells**

| | *Hetuximab* | | |
|---|---|---|---|
| | Parent | Sphere | Fold Enrichment |
| SNU-5 | 3.68% | 6.24% | 1.70 |
| BGC-823 | 3.28% | 5.39% | 1.64 |
| BEL7402 V13 | 6.47% | 11.83% | 1.83 |
| MHCC-97L | 6.80% | 13.79% | 2.03 |

| | Hetuximab | | |
|---|---|---|---|
| | Parent | Sphere | Fold Enrichment |
| SNU-5 | 4.35% | 7.66% | 1.76 |
| BGC-823 | 4.12% | 6.55% | 1.59 |
| BEL7402 V13 | 7.94% | 14.13% | 1.78 |
| MHCC-97L | 8.21% | 18.47% | 2.25 |

| | Hetuzumab H5L6 | | |
|---|---|---|---|
| | Parent | Sphere | Fold Enrichment |
| | | | |
| SNU-5 | 3.68% | 6.20% | 1.69 |
| BGC-823 | 3.84% | 6.26% | 1.63 |
| BEL7402 V13 | 7.12% | 12.03% | 1.69 |
| MHCC-97L | 7.35% | 16.17% | 2.20 |

| | Hetuzumab H5L11 | | |
|---|---|---|---|
| | Parent | Sphere | Fold Enrichment |
| | | | |
| SNU-5 | 3.59% | 5.92% | 1.65 |
| BGC-823 | 3.39% | 5.42% | 1.60 |
| BEL7402 V13 | 6.94% | 11.31% | 1.63 |
| MHCC-97L | 6.85% | 14.66% | 2.14 |

| | Hetuzumab H5L12 | | |
|---|---|---|---|
| | Parent | Sphere | Fold Enrichment |
| | | | |
| SNU-5 | 2.63% | 4.10% | 1.56 |
| BGC-823 | 2.90% | 4.44% | 1.53 |
| BEL7402 V13 | 5.38% | 8.39% | 1.56 |
| MHCC-97L | 5.25% | 10.61% | 2.02 |

It can be seen from the above results that the three humanized variants (H5L6, H5L11, H5L12) of Hetuzumab, the chimeric antibody Hetuximab and the parental mouse monoclonal antibody Hetumomab have very consistent positive rate and enrichment in four kinds of tumor cells and tumor-stem cell-rich sphere cells, especially Hetuzumab H5L6 and Hetuzumab H5L11. As such, the three variants (H5L6, H5L11, H5L12) of Hetuzumab, the chimeric antibody Hetuximab and the parental mouse monoclonal antibody Hetumomab recognize the same group of tumor stem cells.

### 2. A sphere-forming inhibition experiment was used to demonstrate that the humanized variants of Hetuzumab, the chimeric antibody Hetuximab and the parental mouse monoclonal antibody Hetuzumab have the same pharmaceutical effect of inhibiting tumor stem cells in vitro.

According to the same process described above, by using the tumor-stem-cell-rich sphere cells of the four cell lines, i.e., the SNU-5, BGC-823, MHCC-97L, BEL7402 V13 cell lines, and by using different variants of Hetuzumab, the Hetuximab and Hetumomab to perform conventional sphere-forming inhibition experiments, the pharmaceutical effects of inhibiting tumor stem cells of the antibodies were detected. The results for the four cell lines are as follows:

**Table 33. Sphere-forming inhibition rate of different antibodies for the 4 cell lines**

| **SNU-5** | | | | |
|---|---|---|---|---|
| | | **Concentration of antibody ug/ml** | **Average number of formed spheres** | **Inhibition Rate (%)** |
| | **Hetuximab** | | | |
| | | 160 | 120 | 51.0 |
| | | 80 | 149.5 | 38.0 |
| | | 40 | 169.5 | 30.8 |
| | | 20 | 183.5 | 25.1 |
| | | 10 | 207 | 15.6 |
| | | 0 | 245 | - |

| | **Hetumomab** | | | |
|---|---|---|---|---|
| | | 160 | 115 | 53.1 |
| | | 80 | 147 | 40.0 |
| | | 40 | 164 | 33.1 |
| | | 20 | 190 | 22.4 |
| | | 10 | 212 | 13.5 |
| | | 0 | 245 | - |

| | **Hetuzumab H5L6** | | | |
|---|---|---|---|---|
| | | 160 | 124 | 49.4 |
| | | 80 | 151.5 | 38.2 |
| | | 40 | 176 | 28.2 |
| | | 20 | 201 | 18.0 |
| | | 10 | 216 | 11.8 |
| | | 0 | 245 | - |

| | **Hetuzumab H5L11** | | | |
|---|---|---|---|---|
| | | 160 | 126 | 48.6 |
| | | 80 | 156 | 36.3 |
| | | 40 | 178 | 27.3 |
| | | 20 | 188 | 23.3 |
| | | 10 | 218 | 11.0 |
| | | 0 | 245 | - |
| | | | | |

| **BEL7402 V13** | | | | |
|---|---|---|---|---|
| | | **Concentration of antibody ug/ml** | **Average number of formed spheres** | **Inhibition Rate (%)** |
| | **Hetuximab** | | | |
| | | 160 | 103 | 55.2 |
| | | 80 | 127 | 44.8 |
| | | 40 | 148 | 35.7 |
| | | 20 | 175 | 23.9 |
| | | 10 | 195.5 | 15.0 |
| | | 0 | 230 | - |

| | **Hetumomab** | | | |
|---|---|---|---|---|
| | | 160 | 101 | 56.1 |
| | | 80 | 124.5 | 45.9 |
| | | 40 | 150 | 34.8 |
| | | 20 | 172 | 25.2 |
| | | 10 | 192.5 | 16.3 |
| | | 0 | 230 | - |

| | **Hetuzumab H5L6** | | | |
|---|---|---|---|---|
| | | 160 | 109 | 52.6 |
| | | 80 | 121 | 47.8 |
| | | 40 | 156 | 32.2 |
| | | 20 | 189 | 17.8 |
| | | 10 | 201 | 12.6 |
| | | 0 | 230 | - |

| | **Hetuzumab H5L11** | | | |
|---|---|---|---|---|
| | | 160 | 101 | 56.1 |
| | | 80 | 129.5 | 43.7 |
| | | 40 | 151 | 34.3 |
| | | 20 | 191 | 17.0 |
| | | 10 | 205 | 10.9 |
| | | 0 | 230 | - |
| | | | | |

| **MHCC-97L** | | | | |
|---|---|---|---|---|
| | | **Concentration of antibody ug/ml** | **Average number of formed spheres** | **Inhibition Rate (%)** |
| | **Hetuximab** | | | |
| | | 160 | 117 | 51.5 |
| | | 80 | 146 | 39.4 |
| | | 40 | 159 | 34.0 |
| | | 20 | 175.5 | 27.2 |
| | | 10 | 201 | 16.6 |
| | | 0 | 241 | - |

| | **Hetumomab** | | | |
|---|---|---|---|---|
| | | 160 | 115 | 52.3 |
| | | 80 | 141 | 40.7 |
| | | 40 | 155 | 35.7 |
| | | 20 | 178 | 26.1 |
| | | 10 | 204 | 15.4 |
| | | 0 | 241 | - |

| | **Hetuzumab H5L6** | | | |
|---|---|---|---|---|
| | | 160 | 113 | 53.1 |
| | | 80 | 138 | 42.7 |
| | | 40 | 158.5 | 34.2 |
| | | 20 | 186 | 22.8 |
| | | 10 | 214 | 11.2 |
| | | 0 | 241 | - |

| | **Hetuzumab H5L11** | | | |
|---|---|---|---|---|
| | | 160 | 110 | 54.4 |
| | | 80 | 145 | 39.8 |
| | | 40 | 160.5 | 33.4 |
| | | 20 | 190 | 21.2 |
| | | 10 | 212 | 12.0 |
| | | 0 | 241 | - |
| | | | | |

| **BGC-823** | | | | |
|---|---|---|---|---|
| | | **Concentration of antibody ug/ml** | **Average number of formed spheres** | **Inhibition Rate (%)** |
| | **Hetuximab** | | | |
| | | 160 | 131 | 48.2 |
| | | 80 | 150.5 | 40.5 |
| | | 40 | 167 | 34.0 |
| | | 20 | 187 | 26.1 |
| | | 10 | 208.5 | 17.6 |
| | | 0 | 253 | - |

| | **Hetumomab** | | | |
|---|---|---|---|---|
| | | 160 | 130.5 | 48.4 |
| | | 80 | 156 | 38.3 |
| | | 40 | 176 | 30.4 |
| | | 20 | 190 | 24.9 |
| | | 10 | 211 | 16.6 |
| | | 0 | 253 | - |

| | **Hetuzumab H5L6** | | | |
|---|---|---|---|---|
| | | 160 | 132 | 47.8 |
| | | 80 | 160 | 36.8 |
| | | 40 | 185 | 26.8 |
| | | 20 | 197 | 22.1 |
| | | 10 | 221 | 12.6 |
| | | 0 | 253 | - |

| | **Hetuzumab H5L11** | | | |
|---|---|---|---|---|
| | | 160 | 135 | 46.6 |
| | | 80 | 155 | 38.7 |
| | | 40 | 188 | 25.7 |
| | | 20 | 198 | 21.7 |
| | | 10 | 225 | 11.1 |
| | | 0 | 253 | - |

It can be seen from the above results that the two humanized variants (H5L6, H5L11) of Hetuzumab, the chimeric antibody Hetuximab, and the parental mouse monoclonal antibody Hetumomab of various concentrations have very consistent efficiency in inhibiting sphere-forming of tumor-stem-cell-rich sphere cells of the four tumor cell lines. Therefore, the two humanized variants (H5L6, H5L11) of Hetuzumab and the parental mouse monoclonal antibody Hetumomab have the same pharmacetical effect of inhibiting tumor stem cells in vitro.

### 3. The humanized variants of Hetuzumab significantly inhibits the in vivo growth of human liver cancer transplanted tumors, and can synergically enhance therapeutic efficacy of chemotherapy, have significant anti-tumor pharmacetical effect.

An experimental on the treatment of human liver cancer in nude mice by the humanized Hetumomab was carried out. The therapeutic efficacy of the monoclonal antibody alone, the chemotherapeutic drugs alone and the combination of the monoclonal antibody and the chemotherapeutic drugs for treating liver cancer was observed and compared. In addition, it was compared and analyzed whether Hetumomab can inhibit the growth of liver tumors in vivo and synergically enhance therapeutic effect of chemotherapy.

Specifically, the sphere cells of the human liver cancer cell line Bel7402-V13 were harvested and inoculated subcutaneouly into nude mice at 30000 cells/mouse. The mice were divided into following 7 groups, with 8 mice in each group: the PBS control group; the mouse IgG control group; the chemotherapy group (cisplatin 0.3 mg/kg); the high dose Hetuzumab H5L11 group (20 mg/kg); the low dose Hetuzumab H5L11 group (1.25 mg/kg); the high dose Hetuzumab H5L11 + chemotherapeutic drug group; and the low dose Hetuzumab H5L11 + chemotherapeutic drug group. The antibody treatments were started on the second day after inoculation of cancer cells. The experimental group and the control group were treated by intraperitoneal injection. The antibody treatments were performed for 36 days. The chemotherapeutic drug group was treated for 4 weeks, twice a week. The major and minor diameters of the subcutaneously transplanted tumor were measured twice a week. The tumor volume was calculated with the formula V = (π / 6) × (major diameter × minor diameter × minor diameter). The change in tumor volume in each group was observed.

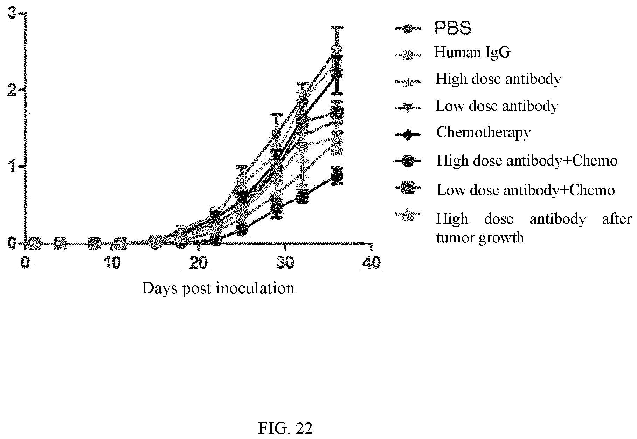

When the treatment was stopped 36 days after the inoculation, the tumor volume growth of the mice was observed and measured, and the inhibition rate was calculated. The growth curve of transplanted tumors in mice is shown in FIG. 22, and the tumor volume inhibition rate is shown in Table 34. The humanized monoclonal antibody Hetuzumab H5L11 can significantly inhibit the growth of human liver cancer transplanted tumors in nude mice, with its inhibition rate against the transplanted tumors increasing along with the increase of the dose of the antibody. At the time of drug withdrawal, the inhibition rates of high dose and low dose humanized monoclonal antibody Hetuzumab H5L11 on the transplanted tumors were 47.47% and 36.80%, respectively. The inhibition rate of the chemotherapeutic group was only 14.52%, which means that it was almost ineffective. However, the inhibition rates of the high and low dose of humanized monoclonal antibody Hetuzumab H5L11 combined with chemotherapeutic drug groups reached 65.08% and 32.66% respectively. The results suggest that the inhibition rate of the humanized monoclonal antibody Hetuzumab H5L11 combined with the chemotherapeutic drug on the transplanted tumors in mice was higher than that of the chemotherapy alone and the antibody alone, *p* <0.05. The humanized monoclonal antibody Hetuzumab H5L11 in combination with the chemotherapeutic drug group showed a better therapeutic effect in the treatment of the transplanted tumors.

The above results show that the humanized monoclonal antibody Hetuzumab H5L11 as applied alone may significantly inhibit the growth of human malignant tumor transplanted tumors and has a significant pharmaceutical effect of inhibiting human malignant tumor. The combinations of humanized monoclonal antibody Hetuzumab H5L11 and the chemotherapeutic drug all showed a high inhibition rate against transplanted tumors, thus can significantly inhibit tumor growth, have a better treatment effect than that of the antibody alone and the chemotherapeutic drug alone, which indicates that the combination of humanized monoclonal antibody Hetuzumab H5L11 and the chemotherapeutic drug may effectively inhibit the tumor growth and reduce the chemotherapeutic drug resistance.

**Table 34 Inhibitory effect of the humanized monoclonal antibody Hetuzumab H5L11 on the growth of human liver cancer cell line Bel7402-V13 transplanted tumors within animals**

| Group | Tumor Volume Inhibition Rate (%) |
|---|---|
| PBS | |
| Human IgG | 7.16 |
| Chemotherapeutic Drug Alone | 14.52 |
| High Dose Hetuzumab H5L11 | 47.47 |
| Low Dose Hetuzumab H5L11 | 36.80 |
| High Dose Hetuzumab H5L11+ Chemotherapeutic Drug | 65.08 |
| Low Dose Hetuzumab H5L11+Chemotherapeutic Drug | 32.66 |
| High Dose Hetuzumab H5L11 After Growth of Tumor | 45.65 |

The above animal experimental results of inhibiting tumors demonstrated that the humanized monoclonal antibody Hetuzumab H5L11 has a significant inhibitory effect (pharmaceutical effect) on the growth and drug resistance of human tumors in vivo, and has important application value for treating the growth, metastasis and drug resistance of human tumors.

Sequence Listing

>SEQ ID NO:1 Hetumomab light chain variable region
DIVMTQAAFSNPVTLGTSASISCRSSKSLLHSNGITYLYWFLQKPGQSPQLLIYQMSNL
ASGVPDRFSSSGSGTDFTLRISRVEAEDVGVYYCAQNLELYTFGGGTKLEIK

>SEQ ID NO:2 Hetumomab VL-CDR1
RSSKSLLHSNGITYLY

>SEQ ID NO:3 Hetumomab VL-CDR2
QMSNLAS

>SEQ ID NO:4 Hetumomab VL-CDR3
AQNLELYT

>SEQ ID NO:5 Hetumomab heavy chain variable region
QVTLKESGPGILQPSQTLSLTCSFSGFSLSTSGMGVSWIRQPSGKGLEWLAHIYWDDD
KRYNPSLKSRLTISKDSSNNQVFLKITSVDTADTATYYCARSFYYYANNSFAYWGQGT
LVTVSS

>SEQ ID NO:6 Hetumomab VH-CDR1
TSGMGVS

>SEQ ID NO:7 Hetumomab VH-CDR2
HIYWDDDKRYNPSLKS

>SEQ ID NO:8 Hetumomab VH-CDR3
SFYYYANNSFAY

>SEQ ID NO:9 Hetuximab chimeric antibody heavy chain amino acid sequence
QVTLKESGPGILQPSQTLSLTCSFSGFSLSTSGMGVSWIRQPSGKGLEWLAHIYWDDD
KRYNPSLKSRLTISKDSSNNQVFLKITSVDTADTATYYCARSFYYYANNSFAYWGQGT
LVTVSSASTKGPSVFPLAPSSKSTSGGTAALGCLVKDYFPEPVTVSWNSGALTSGVHT
FPAVLQSSGLYSLSSVVTVPSSSLGTQTYICNVNHKPSNTKVDKKVEPKSCDKTHTCPP
CPAPELLGGPSVFLFPPKPKDTLMISRTPEVTCVVVDVSHEDPEVKFNWYVDGVEVH
NAKTKPREEQYNSTYRVVSVLTVLHQDWLNGKEYKCKVSNKALPAPIEKTISKAKGQ
PREPQVYTLPPSREEMTKNQVSLTCLVKGFYPSDIAVEWESNGQPENNYKTTPPVLDS
DGSFFLYSKLTVDKSRWQQGNVFSCSVMHEALHNHYTQKSLSLSPGK

>SEQ ID NO:10 Hetuximab chimeric antibody light chain amino acid sequence
DIVMTQAAFSNPVTLGTSASISCRSSKSLLHSNGITYLYWFLQKPGQSPQLLIYQMSNL
ASGVPDRFSSSGSGTDFTLRISRVEAEDVGVYYCAQNLELYTFGGGTKLEIKRTVAAPS
VFIFPPSDEQLKSGTASVVCLLNNFYPREAKVQWKVDNALQSGNSQESVTEQDSKDS
TYSLSSTLTLSKADYEKHKVYACEVTHQGLSSPVTKSFNRGEC

>SEQ ID NO:11 Human IgG1 CH
ASTKGPSVFPLAPSSKSTSGGTAALGCLVKDYFPEPVTVSWNSGALTSGVHTFPAVLQ
SSGLYSLSSVVTVPSSSLGTQTYICNVNHKPSNTKVDKKVEPKSCDKTHTCPPCPAPEL
LGGPSVFLFPPKPKDTLMISRTPEVTCVVVDVSHEDPEVKFNWYVDGVEVHNAKTKP
REEQYNSTYRVVSVLTVLHQDWLNGKEYKCKVSNKALPAPIEKTISKAKGQPREPQV
YTLPPSREEMTKNQVSLTCLVKGFYPSDIAVEWESNGQPENNYKTTPPVLDSDGSFFL
YSKLTVDKSRWQQGNVFSCSVMHEALHNHYTQKSLSLSPGK

>SEQ ID NO:12 Human IgG CK



>SEQ ID NO:13 Mutated VH CDR2
HIYWDDDKRYAPSLKS

>SEQ ID NO:14 Mutated VH CDR3
SFYYYAANSFAY

>SEQ ID NO:15 Humanized heavy chain variable region H1
QVTLKESGPTLVKPTQTLTLTCTFSGFSLSTSGMGVSWIRQPPGKALEWLAHIYWDDD
KRYNPSLKSRLTISKDTSKNQVVLTMTNMDPVDTATYYCARSFYYYANNSFAYWGQG
TLVTVSS

>SEQ ID NO:16 Humanized heavy chain variable region H2
QVTLKESGPGILQPTQTLTLTCTFSGFSLSTSGMGVSWIRQPPGKALEWLAHIYWDDD
KRYNPSLKSRLTISKDTSKNQVVLTMTNMDPVDTATYYCARSFYYYANNSFAYWGQG
TLVTVSS

>SEQ ID NO:17 Humanized heavy chain variable region H3
QVTLKESGPTLVKPTQTLTLTCTFSGFSLSTSGMGVSWIRQPPGKALEWLAHIYWDDD
KRYNPSLKSRLTISKDTSKNQVFLKITSVDPVDTATYYCARSFYYYANNSFAYWGQGT
LVTVSS

>SEQ ID NO:18 Humanized heavy chain variable region H4
QVTLKESGPTLVKPTQTLTLTCTFSGFSLSTSGMGVSWIRQPPGKALEWLAHIYWDDD
KRYAPSLKSRLTISKDTSKNQVVLTMTNMDPVDTATYYCARSFYYYANNSFAYWGQG
TLVTVSS

>SEQ ID NO:19 Humanized heavy chain variable region H5
QVTLKESGPTLVKPTQTLTLTCTFSGFSLSTSGMGVSWIRQPPGKALEWLAHIYWDDD
KRYNPSLKSRLTISKDTSKNQVVLTMTNMDPVDTATYYCARSFYYYAANSFAYWGQG
TLVTVSS

>SEQ ID NO:20 Humanized light chain variable region L1
DIVMTQTPLSLPVTLGQPASISCRSSKSLLHSNGITYLYWYLQKPGQSPQLLIYQMSNL
ASGVPDRFSGSGSGTDFTLKISRVEAEDVGVYYCAQNLELYTFGQGTKVEIK

>SEQ ID NO:21 Humanized light chain variable region L2
DIVMTQAAFSLPVTLGQPASISCRSSKSLLHSNGITYLYWYLQKPGQSPQLLIYQMSNL
ASGVPDRFSGSGSGTDFTLKISRVEAEDVGVYYCAQNLELYTFGQGTKVEIK

>SEQ ID NO:22 Humanized light chain variable region L3
DIVMTQTPLSNPVTLGTSASISCRSSKSLLHSNGITYLYWYLQKPGQSPQLLIYQMSNL
ASGVPDRFSGSGSGTDFTLKISRVEAEDVGVYYCAQNLELYTFGQGTKVEIK

>SEQ ID NO:23 Humanized light chain variable region L4
DIVMTQTPLSLPVTLGQPASISCRSSKSLLHSNGITYLYWFLQKPGQSPQLLIYQMSNL
ASGVPDRFSGSGSGTDFTLRISRVEAEDVGVYYCAQNLELYTFGQGTKVEIK

>SEQ ID NO:24 Humanized light chain variable region L5
DIVMTQTPLSNPVTLGTSASISCRSSKSLLHSNGITYLYWFLQKPGQSPQLLIYQMSNL
ASGVPDRFSGSGSGTDFTLKISRVEAEDVGVYYCAQNLELYTFGQGTKVEIK

>SEQ ID NO:25 Humanized light chain variable region L6
DIVMTQTPFSNPVTLGTSASISCRSSKSLLHSNGITYLYWFLQKPGQSPQLLIYQMSNL
ASGVPDRFSGSGSGTDFTLKISRVEAEDVGVYYCAQNLELYTFGQGTKVEIK

>SEQ ID NO:26 Humanized light chain variable region L7
DIVMTQTPFSNPVTLGTSASISCRSSKSLLHSNGITYLYWYLQKPGQSPQLLIYQMSNL
ASGVPDRFSGSGSGTDFTLKISRVEAEDVGVYYCAQNLELYTFGQGTKVEIK

>SEQ ID NO:27 Humanized light chain variable region L8
DIVMTQSPFSNPVTLGTSASISCRSSKSLLHSNGITYLYWFLQKPGQSPQLLIYQMSNL
ASGVPDRFSGSGSGTDFTLKISRVEAEDVGVYYCAQNLELYTFGQGTKVEIK

>SEQ ID NO:28 Humanized light chain variable region L9
DIVMTQSAFSNPVTLGTSASISCRSSKSLLHSNGITYLYWFLQKPGQSPQLLIYQMSNL
ASGVPDRFSGSGSGTDFTLKISRVEAEDVGVYYCAQNLELYTFGQGTKVEIK

>SEQ ID NO:29 Humanized light chain variable region L10
DIVMTQTPFSLPVTLGTSASISCRSSKSLLHSNGITYLYWFLQKPGQSPQLLIYQMSNL
ASGVPDRFSGSGSGTDFTLKISRVEAEDVGVYYCAQNLELYTFGQGTKVEIK

>SEQ ID NO:30 Humanized light chain variable region L11

DIVMTQTPFSNPVTLGQPASISCRSSKSLLHSNGITYLYWFLQKPGQSPQLLIYQMSNL
ASGVPDRFSGSGSGTDFTLKISRVEAEDVGVYYCAQNLELYTFGQGTKVEIK

>SEQ ID NO:31 Humanized light chain variable region L12
DIVMTQTPFSLPVTLGQPASISCRSSKSLLHSNGITYLYWFLQKPGQSPQLLIYQMSNL
ASGVPDRFSGSGSGTDFTLKISRVEAEDVGVYYCAQNLELYTFGQGTKVEIK

>SEQ ID NO:32 Humanized heavy chain H1
QVTLKESGPTLVKPTQTLTLTCTFSGFSLSTSGMGVSWIRQPPGKALEWLAHIYWDDD
KRYNPSLKSRLTISKDTSKNQVVLTMTNMDPVDTATYYCARSFYYYANNSFAYWGQG
TLVTVSSASTKGPSVFPLAPSSKSTSGGTAALGCLVKDYFPEPVTVSWNSGALTSGVH
TFPAVLQSSGLYSLSSVVTVPSSSLGTQTYICNVNHKPSNTKVDKKVEPKSCDKTHTCP
PCPAPELLGGPSVFLFPPKPKDTLMISRTPEVTCVVVDVSHEDPEVKFNWYVDGVEVH
NAKTKPREEQYNSTYRVVSVLTVLHQDWLNGKEYKCKVSNKALPAPIEKTISKAKGQ
PREPQVYTLPPSREEMTKNQVSLTCLVKGFYPSDIAVEWESNGQPENNYKTTPPVLDS
DGSFFLYSKLTVDKSRWQQGNVFSCSVMHEALHNHYTQKSLSLSPGK

>SEQ ID NO:33 Humanized heavy chain H2
QVTLKESGPGILQPTQTLTLTCTFSGFSLSTSGMGVSWIRQPPGKALEWLAHIYWDDD
KRYNPSLKSRLTISKDTSKNQVVLTMTNMDPVDTATYYCARSFYYYANNSFAYWGQG
TLVTVSSASTKGPSVFPLAPSSKSTSGGTAALGCLVKDYFPEPVTVSWNSGALTSGVH
TFPAVLQSSGLYSLSSVVTVPSSSLGTQTYICNVNHKPSNTKVDKKVEPKSCDKTHTCP
PCPAPELLGGPSVFLFPPKPKDTLMISRTPEVTCVVVDVSHEDPEVKFNWYVDGVEVH
NAKTKPREEQYNSTYRVVSVLTVLHQDWLNGKEYKCKVSNKALPAPIEKTISKAKGQ
PREPQVYTLPPSREEMTKNQVSLTCLVKGFYPSDIAVEWESNGQPENNYKTTPPVLDS
DGSFFLYSKLTVDKSRWQQGNVFSCSVMHEALHNHYTQKSLSLSPGK

>SEQ ID NO:34 Humanized heavy chain H3
QVTLKESGPTLVKPTQTLTLTCTFSGFSLSTSGMGVSWIRQPPGKALEWLAHIYWDDD
KRYNPSLKSRLTISKDTSKNQVFLKITSVDPVDTATYYCARSFYYYANNSFAYWGQGT
LVTVSSASTKGPSVFPLAPSSKSTSGGTAALGCLVKDYFPEPVTVSWNSGALTSGVHT
FPAVLQSSGLYSLSSVVTVPSSSLGTQTYICNVNHKPSNTKVDKKVEPKSCDKTHTCPP
CPAPELLGGPSVFLFPPKPKDTLMISRTPEVTCVVVDVSHEDPEVKFNWYVDGVEVH
NAKTKPREEQYNSTYRVVSVLTVLHQDWLNGKEYKCKVSNKALPAPIEKTISKAKGQ
PREPQVYTLPPSREEMTKNQVSLTCLVKGFYPSDIAVEWESNGQPENNYKTTPPVLDS
DGSFFLYSKLTVDKSRWQQGNVFSCSVMHEALHNHYTQKSLSLSPGK

>SEQ ID NO:35 Humanized heavy chain H4
QVTLKESGPTLVKPTQTLTLTCTFSGFSLSTSGMGVSWIRQPPGKALEWLAHIYWDDD
KRYAPSLKSRLTISKDTSKNQVVLTMTNMDPVDTATYYCARSFYYYANNSFAYWGQG
TLVTVSSASTKGPSVFPLAPSSKSTSGGTAALGCLVKDYFPEPVTVSWNSGALTSGVH
TFPAVLQSSGLYSLSSVVTVPSSSLGTQTYICNVNHKPSNTKVDKKVEPKSCDKTHTCP
PCPAPELLGGPSVFLFPPKPKDTLMISRTPEVTCVVVDVSHEDPEVKFNWYVDGVEVH
NAKTKPREEQYNSTYRVVSVLTVLHQDWLNGKEYKCKVSNKALPAPIEKTISKAKGQ
PREPQVYTLPPSREEMTKNQVSLTCLVKGFYPSDIAVEWESNGQPENNYKTTPPVLDS
DGSFFLYSKLTVDKSRWQQGNVFSCSVMHEALHNHYTQKSLSLSPGK

>SEQ ID NO:36 Humanized heavy chain H5
QVTLKESGPTLVKPTQTLTLTCTFSGFSLSTSGMGVSWIRQPPGKALEWLAHIYWDDD
KRYNPSLKSRLTISKDTSKNQVVLTMTNMDPVDTATYYCARSFYYYAANSFAYWGQG
TLVTVSSASTKGPSVFPLAPSSKSTSGGTAALGCLVKDYFPEPVTVSWNSGALTSGVH
TFPAVLQSSGLYSLSSVVTVPSSSLGTQTYICNVNHKPSNTKVDKKVEPKSCDKTHTCP

PCPAPELLGGPSVFLFPPKPKDTLMISRTPEVTCVVVDVSHEDPEVKFNWYVDGVEVH
NAKTKPREEQYNSTYRVVSVLTVLHQDWLNGKEYKCKVSNKALPAPIEKTISKAKGQ
PREPQVYTLPPSREEMTKNQVSLTCLVKGFYPSDIAVEWESNGQPENNYKTTPPVLDS
DGSFFLYSKLTVDKSRWQQGNVFSCSVMHEALHNHYTQKSLSLSPGK

>SEQ ID NO:37 Humanized light chain L1
DIVMTQTPLSLPVTLGQPASISCRSSKSLLHSNGITYLYWYLQKPGQSPQLLIYQMSNL
ASGVPDRFSGSGSGTDFTLKISRVEAEDVGVYYCAQNLELYTFGQGTKVEIKRTVAAP
SVFIFPPSDEQLKSGTASVVCLLNNFYPREAKVQWKVDNALQSGNSQESVTEQDSKD
STYSLSSTLTLSKADYEKHKVYACEVTHQGLSSPVTKSFNRGEC

>SEQ ID NO:38 Humanized light chain L2
DIVMTQAAFSLPVTLGQPASISCRSSKSLLHSNGITYLYWYLQKPGQSPQLLIYQMSNL
ASGVPDRFSGSGSGTDFTLKISRVEAEDVGVYYCAQNLELYTFGQGTKVEIKRTVAAP
SVFIFPPSDEQLKSGTASVVCLLNNFYPREAKVQWKVDNALQSGNSQESVTEQDSKD
STYSLSSTLTLSKADYEKHKVYACEVTHQGLSSPVTKSFNRGEC

>SEQ ID NO:39 Humanized light chain L3
DIVMTQTPLSNPVTLGTSASISCRSSKSLLHSNGITYLYWYLQKPGQSPQLLIYQMSNL
ASGVPDRFSGSGSGTDFTLKISRVEAEDVGVYYCAQNLELYTFGQGTKVEIKRTVAAP
SVFIFPPSDEQLKSGTASVVCLLNNFYPREAKVQWKVDNALQSGNSQESVTEQDSKD
STYSLSSTLTLSKADYEKHKVYACEVTHQGLSSPVTKSFNRGEC

>SEQ ID NO:40 Humanized light chain L4
DIVMTQTPLSLPVTLGQPASISCRSSKSLLHSNGITYLYWFLQKPGQSPQLLIYQMSNL
ASGVPDRFSGSGSGTDFTLRISRVEAEDVGVYYCAQNLELYTFGQGTKVEIKRTVAAP
SVFIFPPSDEQLKSGTASVVCLLNNFYPREAKVQWKVDNALQSGNSQESVTEQDSKD
STYSLSSTLTLSKADYEKHKVYACEVTHQGLSSPVTKSFNRGEC

>SEQ ID NO:41 Humanized light chain L5
DIVMTQTPLSNPVTLGTSASISCRSSKSLLHSNGITYLYWFLQKPGQSPQLLIYQMSNL
ASGVPDRFSGSGSGTDFTLKISRVEAEDVGVYYCAQNLELYTFGQGTKVEIKRTVAAP
SVFIFPPSDEQLKSGTASVVCLLNNFYPREAKVQWKVDNALQSGNSQESVTEQDSKD
STYSLSSTLTLSKADYEKHKVYACEVTHQGLSSPVTKSFNRGEC

>SEQ ID NO:42 Humanized light chain L6
DIVMTQTPFSNPVTLGTSASISCRSSKSLLHSNGITYLYWFLQKPGQSPQLLIYQMSNL
ASGVPDRFSGSGSGTDFTLKISRVEAEDVGVYYCAQNLELYTFGQGTKVEIKRTVAAP
SVFIFPPSDEQLKSGTASVVCLLNNFYPREAKVQWKVDNALQSGNSQESVTEQDSKD
STYSLSSTLTLSKADYEKHKVYACEVTHQGLSSPVTKSFNRGEC

>SEQ ID NO:43 Humanized light chain L7
DIVMTQTPFSNPVTLGTSASISCRSSKSLLHSNGITYLYWYLQKPGQSPQLLIYQMSNL
ASGVPDRFSGSGSGTDFTLKISRVEAEDVGVYYCAQNLELYTFGQGTKVEIKRTVAAP
SVFIFPPSDEQLKSGTASVVCLLNNFYPREAKVQWKVDNALQSGNSQESVTEQDSKD
STYSLSSTLTLSKADYEKHKVYACEVTHQGLSSPVTKSFNRGEC

>SEQ ID NO:44 Humanized light chain L8
DIVMTQSPFSNPVTLGTSASISCRSSKSLLHSNGITYLYWFLQKPGQSPQLLIYQMSNL
ASGVPDRFSGSGSGTDFTLKISRVEAEDVGVYYCAQNLELYTFGQGTKVEIKRTVAAP
SVFIFPPSDEQLKSGTASVVCLLNNFYPREAKVQWKVDNALQSGNSQESVTEQDSKD

STYSLSSTLTLSKADYEKHKVYACEVTHQGLSSPVTKSFNRGEC

>SEQ ID NO:45 Humanized light chain L9
DIVMTQSAFSNPVTLGTSASISCRSSKSLLHSNGITYLYWFLQKPGQSPQLLIYQMSNL
ASGVPDRFSGSGSGTDFTLKISRVEAEDVGVYYCAQNLELYTFGQGTKVEIKRTVAAP
SVFIFPPSDEQLKSGTASVVCLLNNFYPREAKVQWKVDNALQSGNSQESVTEQDSKD
STYSLSSTLTLSKADYEKHKVYACEVTHQGLSSPVTKSFNRGEC

>SEQ ID NO:46 Humanized light chain region L10
DIVMTQTPFSLPVTLGTSASISCRSSKSLLHSNGITYLYWFLQKPGQSPQLLIYQMSNL
ASGVPDRFSGSGSGTDFTLKISRVEAEDVGVYYCAQNLELYTFGQGTKVEIKRTVAAP
SVFIFPPSDEQLKSGTASVVCLLNNFYPREAKVQWKVDNALQSGNSQESVTEQDSKD
STYSLSSTLTLSKADYEKHKVYACEVTHQGLSSPVTKSFNRGEC

>SEQ ID NO:47 Humanized light chain L11
DIVMTQTPFSNPVTLGQPASISCRSSKSLLHSNGITYLYWFLQKPGQSPQLLIYQMSNL
ASGVPDRFSGSGSGTDFTLKISRVEAEDVGVYYCAQNLELYTFGQGTKVEIKRTVAAP
SVFIFPPSDEQLKSGTASVVCLLNNFYPREAKVQWKVDNALQSGNSQESVTEQDSKD
STYSLSSTLTLSKADYEKHKVYACEVTHQGLSSPVTKSFNRGEC

>SEQ ID NO:48 Humanized light chain L12
DIVMTQTPFSLPVTLGQPASISCRSSKSLLHSNGITYLYWFLQKPGQSPQLLIYQMSNL
ASGVPDRFSGSGSGTDFTLKISRVEAEDVGVYYCAQNLELYTFGQGTKVEIKRTVAAP
SVFIFPPSDEQLKSGTASVVCLLNNFYPREAKVQWKVDNALQSGNSQESVTEQDSKD
STYSLSSTLTLSKADYEKHKVYACEVTHQGLSSPVTKSFNRGEC

## Claims

1. An isolated monoclonal antibody or antigen-binding fragment thereof against tumor stem cell, wherein the monoclonal antibody comprises a light chain variable region and a heavy chain variable region,
the light chain variable region comprises:
a VL CDR1 comprising an amino acid sequence set forth in SEQ ID NO: 2, or an amino acid sequence having 1 or 2 amino acid residue substitutions, deletions or additions relative to SEQ ID NO: 2,
a VL CDR2 comprising an amino acid sequence set forth in SEQ ID NO: 3, or an amino acid sequence having 1 or 2 amino acid residue substitutions, deletions or additions relative to SEQ ID NO: 3, and
a VL CDR3 comprising an amino acid sequence set forth in SEQ ID NO: 4, or an amino acid sequence having 1 or 2 amino acid residue substitutions, deletions or additions relative to SEQ ID NO: 4;
the heavy chain variable region comprises:
a VH CDR1 comprising an amino acid sequence set forth in SEQ ID NO:6, or an amino acid sequence having 1 or 2 amino acid residue substitutions, deletions or additions relative to SEQ ID NO:6,
a VH CDR2 comprising an amino acid sequence set forth in SEQ ID NO:7, or an amino acid sequence having 1 or 2 amino acid residue substitutions, deletions or additions relative to SEQ ID NO:7, such as a sequence set forth in SEQ ID NO:13, and
a VH CDR3 comprising an amino acid sequence set forth in SEQ ID NO:8, or an amino acid sequence having 1 or 2 amino acid residue substitutions, deletions or additions relative to SEQ ID NO:8, such as a sequence set forth in SEQ ID NO:14.

2. The isolated monoclonal antibody or antigen-binding fragment thereof according to claim 1, wherein the monoclonal antibody comprises a light chain variable region and a heavy chain variable region, the light chain variable region comprises:
a VL CDR1 comprising an amino acid sequence set forth in SEQ ID NO: 2,
a VL CDR2 comprising an amino acid sequence set forth in SEQ ID NO: 3, and
a VL CDR3 comprising an amino acid sequence set forth in SEQ ID NO:4;
the heavy chain variable region comprises:
a VH CDR1 comprising an amino acid sequence set forth in SEQ ID NO:6,
a VH CDR2 comprising an amino acid sequence set forth in SEQ ID NO: 7, and
a VH CDR3 comprising an amino acid sequence set forth in SEQ ID NO:14.

3. The isolated monoclonal antibody or antigen-binding fragment thereof according to claim 1, the light chain variable region comprises an amino acid sequence set forth in SEQ ID NO:1, or an amino acid sequence having at least 85%, at least 90%, at least 95% or higher sequence identity to SEQ ID NO:1.

4. The isolated monoclonal antibody or antigen-binding fragment thereof according to claim 1, the heavy chain variable region comprises an amino acid sequence set forth in SEQ ID NO:5, or an amino acid sequence having at least 85%, at least 90%, at least 95% or higher sequence identity to SEQ ID NO:5.

5. The isolated monoclonal antibody or antigen-binding fragment thereof according to claim 1, the heavy chain variable region comprises an amino acid sequence set forth in one of SEQ ID NOs: 15-19.

6. The isolated monoclonal antibody or antigen-binding fragment thereof according to claim 1, the light chain variable region comprises an amino acid sequence set forth in one of SEQ ID NOs: 20-31.

7. The isolated monoclonal antibody or antigen-binding fragment thereof according to claim 1, the heavy chain variable region comprises an amino acid sequence set forth in SEQ ID NO: 19, the light chain variable region comprises an amino acid sequence set forth in SEQ ID NO: 30.

8. The isolated monoclonal antibody or antigen-binding fragment thereof according to claim 1, the monoclonal antibody comprises a human heavy chain constant region, for example, a human heavy chain constant region comprising an amino acid sequence set forth in SEQ ID NO: 11.

9. The isolated monoclonal antibody or antigen-binding fragment thereof according to claim 1, the monoclonal antibody comprises a human light chain constant region, for example, a human light chain constant region comprising an amino acid sequence set forth in SEQ ID NO: 12.

10. The isolated monoclonal antibody or antigen-binding fragment thereof according to claim 1, the monoclonal antibody comprises a heavy chain having an amino acid sequence set forth in SEQ ID NO: 9 and a light chain having an amino acid sequence set forth in SEQ ID NO:10.

11. The isolated monoclonal antibody or antigen-binding fragment thereof according to claim 1, the monoclonal antibody comprises a heavy chain having an amino acid sequence set forth in one of SEQ ID NOs:32-36 and a light chain having an amino acid sequence set forth in one of SEQ ID NOs:37-48.

12. The isolated monoclonal antibody or antigen-binding fragment thereof according to claim 1, the monoclonal antibody comprises a heavy chain set forth in SEQ ID NO:36 and a light chain set forth in SEQ ID NO:47.

13. The isolated monoclonal antibody or antigen-binding fragment thereof according to claim 1, the monoclonal antibody comprises a heavy chain set forth in SEQ ID NO:36 and a light chain set forth in SEQ ID NO:42.

14. A monoclonal antibody or antigen-binding fragment thereof, the monoclonal antibody is produced with a mouse hybridoma deposited in China General Microbiological Culture Collection Center on March 16, 2016 under the accession number of CGMCC NO. 12251.

15. A hybridoma cell deposited in China General Microbiological Culture Collection Center on March 16, 2016 under the accession number of CGMCC NO. 12251.

16. A pharmaceutical composition comprising a monoclonal antibody or an antigen-binding fragment thereof according to any one of claims 1-14, and a pharmaceutically acceptable carrier.

17. The pharmaceutical composition according to claim 16, wherein the monoclonal antibody or antigen-binding fragment thereof is conjugated to a therapeutic moiety selected from the group consisting of a cytotoxin, a radioisotope, or a biologically active protein.

18. A method for treating a malignant tumor, preventing and/or treating metastasis or relapse of a malignant tumor in a patient, the method comprising administering to the patient an effective amount of the monoclonal antibody or antigen-binding fragment thereof according to any one of claims 1-14 or the pharmaceutical composition according to claim 16 or 17.

19. The method according to claim 18, wherein the malignant tumor is selected from the group consisting of breast cancer, colorectal cancer, pancreatic cancer, prostatic cancer, liver cancer, lung cancer, and gastric cancer.

20. The method according to claim 18 or 19, further comprising administering to the patient other anti-tumor treatment means, such as administering a chemotherapeutic agent, an antibody targeting other tumor-specific antigens, or radiation therapy.

21. Use of the monoclonal antibody or antigen-binding fragment thereof according to any one of claims 1-14 or a pharmaceutical composition according to claim 16 or 17 in preparing a medicament for treating a malignant tumor, preventing and/or treating metastasis or relapse of a malignant tumor.

22. The use according to claim 21, wherein the malignant tumor is selected from the group consisting of breast cancer, colorectal cancer, pancreatic cancer, prostatic cancer, liver cancer, lung cancer, and gastric cancer.

23. A method for detecting the presence of tumor stem cells in a biological sample, comprising:
a) contacting the biological sample with the monoclonal antibody or antigen-binding fragment thereof according to any one of claims 1-14;
b) detecting the binding of the monoclonal antibody or antigen-binding fragment thereof to a target antigen in said biological sample,
wherein the detected binding indicates the presence of tumor stem cells in said biological sample.

24. A method for isolating tumor stem cells, the method comprising:
(a) providing a cell population suspected of containing tumor stem cells;
(b) identifying a subpopulation of said cells that bind to the monoclonal antibody or antigen-binding fragment thereof according to any one of claims 1-14; and
(c) isolating the subpopulation.

25. The method according to claim 23 or 24, the tumor stem cells are selected from the group consisting of breast cancer stem cells, colorectal cancer stem cells, pancreatic cancer stem cells, prostatic cancer stem cells, liver cancer stem cells, lung cancer stem cells, and gastric cancer stem cells.

26. A method for detecting the presence of a malignant tumor in a patient, comprising:
a) contacting a biological sample from the patient with the monoclonal antibody or antigen-binding fragment thereof according to any one of claims 1-14;
b) detecting the binding of the monoclonal antibody or antigen-binding fragment thereof to a target antigen in said biological sample, wherein the detected binding represents the presence of the malignant tumor in said patient.

27. A method for prognosis of relapse or progression of a malignant tumor in a patient, the method comprising:
(a) isolating a biological sample comprising circulating cells from the patient;
b) contacting the biological sample comprising circulating cells with the monoclonal antibody or antigen-binding fragment thereof according to any one of claims 1-14; and
(c) identifying the presence of the circulating cells that bind to the monoclonal antibody or antigen-binding fragment thereof,
thereby prognosing the relapse or progression of the malignant tumor in the patient.

28. The method according to claim 27, the progression of the malignant tumor comprises metastasis of the malignant tumor in the patient.

29. The method according to any one of claims 26-28, wherein the biological sample comprises a blood sample, a lymph sample, or components thereof.

30. The method according to any one of claims 26-28, wherein the malignant tumor is selected from the group consisting of breast cancer, colorectal cancer, pancreatic cancer, prostatic cancer, liver cancer, lung cancer, and gastric cancer.

31. An isolated nucleic acid molecule encoding the monoclonal antibody or an antigen-binding fragment thereof according to any one of claims 1-14.

32. The isolated nucleic acid molecule according to claim 31, the nucleic acid molecule is operably linked to an expression regulatory sequence.

33. An expression vector comprising the nucleic acid molecule according to any one of claims 31-32.

34. A host cell transformed with the nucleic acid molecule according to any one of claims 31-32 or the expression vector according to claim 31.

35. A method of producing a monoclonal antibody or antigen-binding fragment thereof against human tumor stem cells, the method comprising:
(i) culturing the host cell according to claim 34 in a condition suitable for expression of the nucleic acid molecule or expression vector, and
(ii) isolating and purifying the antibody or antigen-binding fragment thereof expressed by the nucleic acid molecule or expression vector.
